# EUROPEAN PATENT SPECIFICATION

(11) **EP 4 322 877 B1**
(45) Date of publication and mention of the grant of the patent: **25.03.2026**
(21) Application number: 22720846.9
(22) Date of filing: 11.04.2022
(51) Int. Cl.: A61B 18/24, A61B 17/34, A61B 18/22, A61B 18/28, A61K 9/00, A61K 41/00, A61N 5/06, A61N 5/067

(54) **SYSTEM FOR ABLATION THERAPY**
SYSTEM FÜR ABLATIONSTHERAPIE
SYSTÈME POUR THÉRAPIE PAR ABLATION

(30) Priority: 13.04.2021 US 202163174431 P
(43) Date of publication of application: 21.02.2024
(73) Proprietor: Nanospectra Biosciences, Inc., Houston, TX 77054 (US)
(72) Inventor: SCHWARTZ, Jon, Alexander, Houston, Texas 77054 (US); GOODRICH, Glenn, Patrick, Houston, Texas 77054 (US); MURPHY, Andrew, Mark, Bend, Oregon 97703 (US)
(74) Representative: Durán-Corretjer, S.L.P.
(86) International application number: PCT/US2022/024291
(87) International publication number: WO 2022/221208

(56) References cited:
- US-A1- 2020 155 234

## Description

### BACKGROUND

### Description of the Related Art

Thermal and radiative ablation can be used to burn and/or ablate tissues for therapeutic methods. These techniques can be used to ablate cancer tissues and tumors.

### SUMMARY

The invention is defined in the appended set of claims. Methods disclosed hereinafter do not form part of the invention. Several embodiments disclosed herein pertain to systems, kits, and devices for therapeutic treatments and/or ablation therapy of tissues. Several embodiments disclosed herein pertain to laser illuminating assemblies. A laser illuminator assembly, or laser catheter, is a device that is configured to receive a laser waveguide (such as a fiber optic). In several embodiments, the laser illuminator assembly is a cooled laser illuminator assembly configured to receive coolant to cool the laser waveguide when activated. In several embodiments, the laser illuminator assembly can include a main body and a probe. In several embodiments, the laser illuminator assembly can include a sheath connector. It has been found that, in using a sheath connector, certain advantages can be achieved.

In several embodiments, the main body can include a first fluid chamber. In several embodiments, the main body can include a first hub. In several embodiments, the first hub can be in fluidic communication with the first fluid chamber. In several embodiments, the first hub can include a first fluid port. In several embodiments, the first hub can include a fitting configured to engage a first coolant line.

In several embodiments, the main body can include a second fluid chamber. In several embodiments, the second fluid chamber can include a waveguide aperture. In several embodiments, the main body can include a second hub. In several embodiments, the second hub can be in fluidic communication with the second fluid chamber. In several embodiments, the second hub can include a second fluid port. In several embodiments, the second hub can include a fitting configured to engage a second coolant line. In several embodiments, the main body can include a waveguide hub. In several embodiments, the waveguide hub provides access to the second fluid chamber of the main body. In several embodiments, the waveguide hub can be configured to receive a waveguide and to direct it through the second fluid chamber and the waveguide aperture of the second fluid chamber.

In several embodiments, the probe extends distally from the main body. In several embodiments, the probe can include an elongate probe member. In several embodiments, the elongate probe member can include a flexible external wall. In several embodiments, the flexible external wall defines a tubular cavity within the elongate probe. In several embodiments, the external wall defines a first lumen that can be in fluidic communication with first fluid chamber. In several embodiments, the flexible external wall extends distally from the main body. In several embodiments, the elongate probe member can include a proximal portion and a distal portion. In several embodiments, the proximal portion can be located nearer (e.g., can be adjacent to, can be attached to, etc.) the main body than the distal portion. In several embodiments, the distal portion comprising a sealed tip (e.g., the elongate probe member terminates in a sealed tip).

In several embodiments, the probe can include an internal tubular member. In several embodiments, the internal tubular member extends distally from the main body and within the tubular cavity of the elongate probe member. In several embodiments, the internal tubular member defines a second lumen of the probe. In several embodiments, the internal tubular member can include an exit aperture. In several embodiments, the exit aperture can be disposed within the distal portion of the elongate probe member. In several embodiments, the internal tubular member can be configured to receive the waveguide when the waveguide is directed through the waveguide aperture.

In several embodiments, the sheath connector can include a fitting. In several embodiments, the sheath connector can be configured to engage a corresponding connector (e.g., fitting) of a catheter sheath. In several embodiments, the catheter sheath can be configured to at least partially cover the probe when connected to the sheath connector via the connector of the catheter sheath. In several embodiments, when the sheath connector is connected to the connector sheath via the connector of the catheter sheath, the catheter sheath stabilizes the probe. In several embodiments, when the sheath connector is connected to the connector sheath via the connector of the catheter sheath, the catheter sheath lowers incidences of kinking of the probe. In several embodiments, when the sheath connector is connected to the connector sheath via the connector of the catheter sheath, the laser illuminator assembly can be held by the probe in a position where the probe is horizontal without kinking the probe.

In several embodiments, the sheath connector can be disposed circumferentially around at least a portion of the elongate probe member. In several embodiments, the sheath connector can be connected to and/or unitary with the main body of the laser illuminator assembly. In several embodiments, the sheath connector can be connected to and/or unitary with the elongate probe member.

In several embodiments, a length of the probe can be graduated. In several embodiments, the graduations can be provided on the proximal portion of the probe. In several embodiments, the graduations are optionally not provided on the distal portion of the probe. In several embodiments, the graduations of the probe begin at a position at or near the sheath connector. In several embodiments, the graduations of the probe can be about 4 mm apart. In several embodiments, the probe can be a material that can be transmissive to visible wavelengths of light, infrared light, ultraviolet light, or combinations of the foregoing. In several embodiments, the sealed tip can be a domed tip. In several embodiments, the domed tip can be transmissive to visible wavelengths of light, infrared light, ultraviolet light, or combinations of the foregoing.

In several embodiments, when received by the laser illuminator assembly, the waveguide can be positioned within the second lumen and can be configured to transmit laser radiation through the domed end of the introducer probe.

In several embodiments, the first lumen of the probe can be in fluidic communication with the second lumen of the probe. In several embodiments, the first chamber can be in fluidic communication with the second fluid chamber via the probe.

In several embodiments, the probe can include a pooling section located between the sealed tip and the exit aperture of the internal tubular member, the pooling section configure to allow the circulation and/or transfer of fluid between the first lumen and second lumen of the probe.

Several embodiments pertain to a laser illuminator assembly, comprising a body, a sheath connector, and a probe. In several embodiments, the probe can include an elongate probe member extending distally from the body, the elongate probe comprising a first lumen and a sealed end. In several embodiments, the probe can include a distally extending internal tubular member. In several embodiments, the internal tubular member can be disposed and/or located within the first lumen of the introducer probe. In several embodiments, the internal tubular member can include a second lumen and terminating at an exit aperture within the elongate probe. In several embodiments, the first lumen can be in fluidic communication with the second lumen. In several embodiments, the sheath connector can be configured to engage a corresponding connector of a catheter sheath. In several embodiments, the catheter sheath can be configured to at least partially cover the probe when connected to the sheath connector via the connector of the catheter sheath. In several embodiments, when the sheath connector can be connected to the connector sheath via the connector of the catheter sheath, the catheter sheath stabilizes the probe. In several embodiments, when the sheath connector can be connected to the connector sheath via the connector of the catheter sheath, the catheter sheath can lower incidences of kinking of the probe. In several embodiments, a length of the probe can be graduated. In several embodiments, the graduations can be provided on a proximal portion of the probe. In several embodiments, the graduations are optionally not provided on a distal portion of the probe. In several embodiments, the sealed end of the probe can be a domed surface configured to allow laser light transmission.

In several embodiments, the laser illuminator assembly can include a first hub in fluidic communication with the first lumen. In several embodiments, the first hub can include a first fluid port and a fitting configured to engage a first coolant line. In several embodiments, the laser illuminator assembly can include a second hub in fluidic communication with the second lumen. In several embodiments, the second hub can include a second fluid port and a fitting configured to engage a second coolant line.

Any of the embodiments described above, or described elsewhere herein, can include one or more of the following features.

In several embodiments, the first fluid port can be a coolant outlet configured to distribute coolant into the first coolant line and the second fluid port can be a coolant inlet configure to receive coolant from the second coolant line.

In several embodiments, the laser illuminator assembly can be configured to allow the passage of a coolant from the coolant inlet through the second lumen into the first lumen and out of the coolant outlet.

In several embodiments, the fluid inlet and the fluid outlet can be configured to interact with different connectors to prevent improper routing of coolant through the laser illuminator device.

In several embodiments, the fitting of the first hub can be a male or female connector, the fitting of the second hub can be a male or female connector, and the sex of the connector of the first hub and the second hub can be different sexes.

In several embodiments. the sheath connector can be threaded and configured to engage corresponding threads of the connector of the catheter sheath.

In several embodiments, the sheath connector can include a luer fitting configured to engage corresponding luer fitting of the connector of the catheter sheath. In several embodiments, the sheath connector can include a male luer fitting configured to engage corresponding female luer fitting of the connector of the catheter sheath.

Several embodiments pertain to a system comprising the laser illuminator assembly as described above or elsewhere herein. In several embodiments, the system further can include the waveguide, the catheter sheath, or both. In several embodiments, the catheter sheath can be graduated along at least a portion of its length. In several embodiments, the graduations can be provided on the catheter sheath at a position proximal to the connector of the catheter sheath.

In several embodiments, the probe can be longer than the catheter sheath. In several optional embodiments, when the catheter sheath can be connected to the laser illuminator assembly via the sheath connector and the connector of the catheter sheath, the probe can protrude from the catheter sheath. In several embodiments, the exit aperture of the internal tubular member can be optionally not covered by the catheter sheath when the catheter sheath is connected to the laser illuminator assembly via the sheath connector and the connector of the catheter sheath.

In several embodiments, the waveguide can include a connector fitting configured to engage a corresponding fitting of the waveguide hub. In several embodiments, the waveguide can be an optical fiber. In several embodiments, the optical fiber can include a diffusive portion configured to distribute radiation from the optical fiber and out of the laser illuminator assembly.

In several embodiments, the system further can include the first coolant line. In several embodiments, the system further can include the second coolant line. In several embodiments, the system further can include a coolant reservoir configured to engage the second coolant line and to be in fluidic communication with the laser illuminator assembly when engaged to the second coolant line, wherein the coolant reservoir configured to supply coolant to the laser illuminator assembly. In several embodiments, the system further can include a pump configured to convey coolant from the coolant reservoir to the laser illuminator assembly via the second line to cool the waveguide when in operation. In several embodiments, the system further can include a coolant recovery bag configured to engage the first coolant line and to be in fluidic communication with the laser illuminator assembly when engaged to the first coolant line, wherein the coolant recovery bag can be configured to receive coolant from the laser illuminator assembly.

In several embodiments, the laser illuminating system can include a laser source configured to be in optical communication with the optical fiber. In several embodiments, the laser source can be configured to transmit radiation through the optical fiber to the laser illuminator assembly. In several embodiments, when activated, the laser source transmits electromagnetic radiation through optical fiber and through the sealed end.

Several embodiments pertain to a kit comprising a laser illuminator assembly as disclosed above or elsewhere herein. Several embodiments pertain to a kit comprising a system as disclosed above or elsewhere herein.

The invention may be used in a method of treating a tumor. The method can include injecting nanoparticles into a patient systemically wherein the nanoparticles can be adapted to transduce infrared light into heat energy. The method can include allowing the nanoparticles to preferentially accumulate in a tumor. The method can include inserting a trocar assembly comprising a trocar and a catheter sheath into the patient at a first insertion point. In several embodiments, the trocar assembly the trocar can be sheathed in the catheter sheath. The method can include positioning the trocar assembly in the patient by passing the trocar assembly through the tumor such that the trocar assembly passes through a proximal face of the tumor and terminates at a distal side of the tumor and creates a first path within the tumor. The method can include removing the trocar from the trocar assembly leaving the catheter sheath in the patient within the first path. The method can include inserting the probe of the laser illuminator assembly of into the catheter sheath. The method can include guiding the probe to a first position within the first path in the tumor, wherein the first position can be located at or near the distal side of the tumor. The method can include connecting the catheter sheath to the laser illuminator assembly using the sheath connector. The method can include activating a laser source connected to a waveguide within the probe when the probe is at the first position within the first path to generate infrared radiation at the first position for a first period of time thereby heating the nanoparticles to an ablative temperature. The method can include disconnecting the catheter sheath from the laser illuminator assembly. The method can include withdrawing the probe to a second position within the first path in the tumor, the second position being proximally located relative to the first position. The method can include connecting the catheter sheath to the laser illuminator assembly using the sheath connector. The method can include activating the laser source when the probe is at the second position to generate infrared radiation for a second period of time thereby heating the nanoparticles to an ablative temperature.

The method can include removing the catheter sheath and the probe from the first path and creating a second path through the tumor using the trocar assembly. The method can include removing the trocar from the trocar assembly leaving the catheter sheath in the patient within the second path. The method can include inserting the probe of the laser illuminator assembly into the catheter sheath. The method can include guiding the probe to a first position within the second path in the tumor, wherein the first position can be located at or near the distal side of the tumor. The method can include connecting the catheter sheath to the laser illuminator assembly using the sheath connector. The method can include activating the laser source when the probe can be at the first position within the second path to generate infrared radiation for a third period of time thereby heating the nanoparticles to an ablative temperature.

In several embodiments, the first position and the second position of the first path can be about 8 mm apart. In several embodiments, a template grid can be used to position the trocar assembly at the first insertion point and at the second insertion point.

The method can include inserting the trocar assembly into the patient at additional insertion points that can be laterally disposed on the proximal side of the tumor from the first insertion point and second insertion points.

In several embodiments, the template grid can be used to position the trocar assembly at the additional insertion points. In several embodiments, the laser can be activated to emit radiation that can be of insufficient to induce photothermal coagulation of tissue.

The method can include activating the laser illuminator to a subablative level. In several embodiments, the laser illuminator can be activated to emit radiation that can be subablative in the absence of the nanoparticles. In several embodiments, the laser illuminator can be activated to emit radiation between about 3.5 W/cm and about 4.5 W/cm of the diffuser tip.

A method using the invention for treating a tumor can include obtaining the laser illuminator assembly as disclosed elsewhere herein. The method can include positioning a catheter sheath in a tissue comprising the tumor by passing the catheter sheath through a proximal face of the tissue to a distal side of the tissue to a first position. The method can include inserting the probe of the laser illuminator assembly into the catheter sheath. The method can include connecting the catheter sheath to the laser illuminator assembly using the sheath connector. The method can include activating a laser source of the laser illuminator assembly while the probe is at the first position to transmit sub-ablative infrared radiation to the tissue for a first period of time.

The method can include withdrawing the probe while connected to the catheter sheath to a second position in the tissue, the second position being proximally located relative to the first position. The method can include activating the laser source when the introducer probe is at the second position to transmit sub-ablative infrared radiation to the tissue for a second period of time.

In several embodiments, the catheter sheath can be graduated and the graduations of the catheter sheath can be used to measure the distance between the first position and the second position.

The method can include disconnecting the catheter sheath from the laser illuminator assembly after activating the laser source at the first position. The method can include withdrawing the probe to a second position within the first path in the tumor using graduations on the probe to measure a distance between the first position and the second position. The method can include reconnecting the catheter sheath to the laser illuminator assembly using the sheath connector. The method can include activating the laser source when the introducer probe is at the second position to transmit sub-ablative infrared radiation to the tissue for a second period of time. In several embodiments, the second position can be proximally located relative to the first position.

The tumor can be a prostate tumor.

Several embodiments pertain to a method of manufacturing a laser illuminator assembly. In several embodiments, the method can include obtaining a main body of the laser illuminator assembly. In several embodiments, the method can include affixing a probe to the main body. In several embodiments, the method can include affixing a sheath connector to the laser illuminator assembly. In several embodiments, the sheath connector can be affixed to the main body. In several embodiments, the sheath connector can be affixed to the probe. In several embodiments, the sheath connector can be positioned between the main body and the probe.

In several embodiments, the laser illuminating system can include a laser illuminator assembly. In several embodiments, as disclosed elsewhere herein, the laser illuminator assembly may be provided as a standalone component, outside of the laser illuminating system. In several embodiments, the laser illuminator assembly can include a sheath connector. In several embodiments, the laser illuminating system can include a trocar assembly. In several embodiments, the laser illuminating system can include a catheter sheath. In several embodiments, the laser illuminating system can include an optical fiber with a diffuser tip and an optical fiber connector. In several embodiments, the optical fiber connector can be configured to engage the laser illuminator assembly. In several embodiments, when the optical fiber connector is engaged with the laser illuminator assembly, the diffuser tip of the optical fiber can be positioned within the laser illuminator assembly. In several embodiments, the laser illuminating system can include a laser source configured to be in optical communication with the optical fiber. In several embodiments, the laser source can be configured to transmit radiation through the optical fiber to the laser illuminator assembly. In several embodiments, when activated, the laser source transmits electromagnetic radiation through optical fiber and through the sealed end. In several embodiments, the laser illuminating system can include a coolant reservoir. In several embodiments, the coolant reservoir can be in fluidic communication with the laser illuminator assembly. In several embodiments, the laser illuminator assembly can include a coolant inlet tube configured to convey coolant from the coolant reservoir to the laser illuminator assembly. In several embodiments, the laser illuminating system can include a pump configured to convey coolant from the coolant reservoir to the laser illuminator assembly via the coolant inlet tube to cool the optical fiber. In several embodiments, the laser illuminating system can include a coolant recovery bag in fluidic communication with the laser illuminator assembly and configured to receive coolant from the laser illuminator assembly. In several embodiments, the laser illuminator assembly can include a coolant outlet tube configured to convey coolant from the laser illuminator assembly to the coolant recovery bag. In several embodiments, the laser illuminating system can include an actuator configured to activate and deactivate the laser source. In several embodiments, the actuator can be a foot pedal. In several embodiments, the actuator also controls the pump. In several embodiments, the laser and the pump can be activated by the actuator substantially simultaneously. In several embodiments, the laser and the pump can be deactivated by the actuator substantially simultaneously. In several embodiments, the coolant inlet tube can be tygon material. In several embodiments, the coolant inlet tube can be about 4 meters in length. In several embodiments, the coolant inlet tube can be configured to allow coolant flow at a low rate. In several embodiments, the coolant inlet tube can be configured to allow coolant flow at a rate of about 8 ml/min. In several embodiments, the laser source provides a radiation having a wavelength that can be near infrared wavelength. In several embodiments, the laser source provides a radiation that has a wavelength ranging from about 805 nm to about 810 nm. In several embodiments, the optical fiber can include a diffusive portion configured to distribute radiation from the optical fiber and out of the laser illuminator assembly. In several embodiments, a length of the diffusive portion of the optical fiber ranges from about 1.0 cm to about 1.8 cm. In several embodiments, the diffusive portion of the optical fiber may be of a length equal to or less than about: 50 mm, 30 mm, 18 mm, 10mm, values between the aforementioned values, or ranges including and/or spanning those values.

### BRIEF DESCRIPTION OF THE DRAWINGS

The features of laser illuminator assemblies, laser illuminating systems, and components thereof disclosed herein are described below with reference to the drawings of certain embodiments. The illustrated embodiments are intended to demonstrate, but not to limit, the present disclosure. The proportions and relative dimensions and sizes of each component as shown in these drawings forms part of the supporting disclosure of this specification, but should not be limiting on the scope of this specification, except to the extent that such proportions, dimensions, or sizes are included in any individual claims.
Figure 1 illustrates an embodiment of a laser illuminating system.
Figure 2A illustrates an embodiment of a laser catheter assembly comprising a sheath connector.

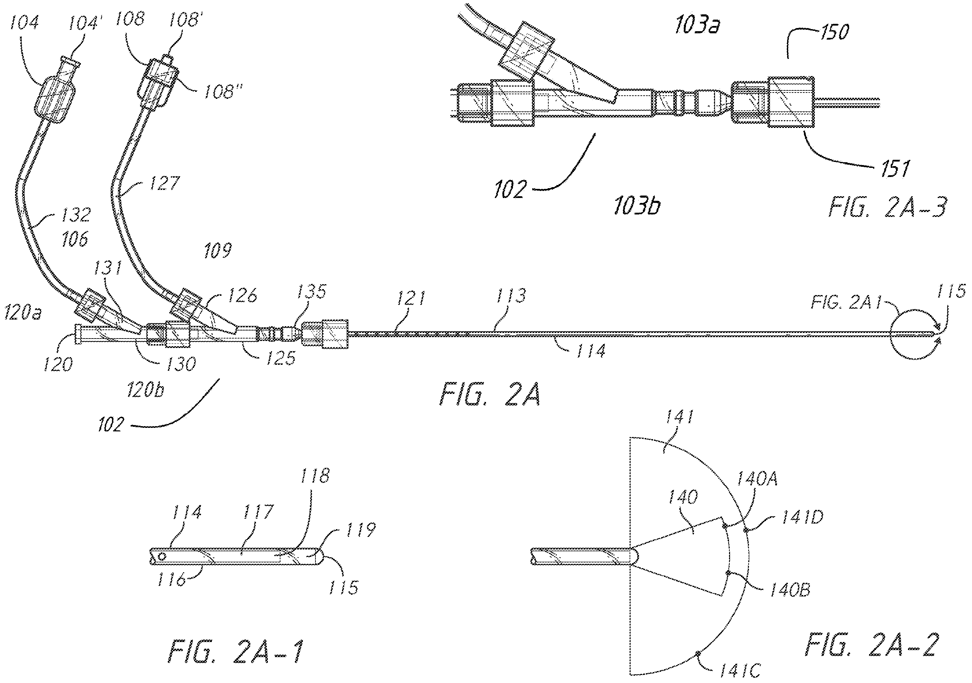

Figures 2A-1, 2A-2, and 2A-3 illustrate expanded views of the laser catheter assembly shown in Figure 2A.

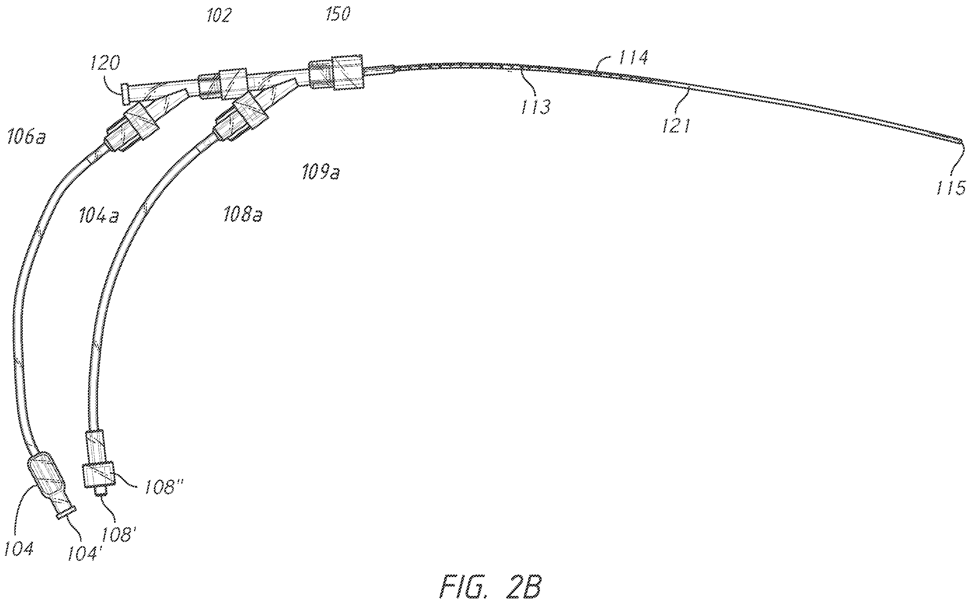

Figure 2B is a view of another embodiment of a laser catheter assembly where the sheath connector is unitary with the main body of the laser catheter assembly.

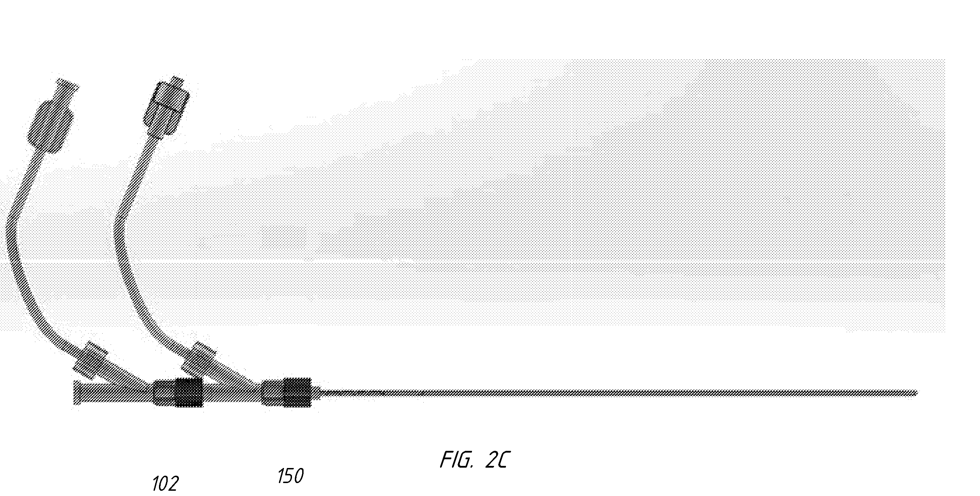

Figures 2C-2E are additional laser catheter assemblies comprising sheath connectors.

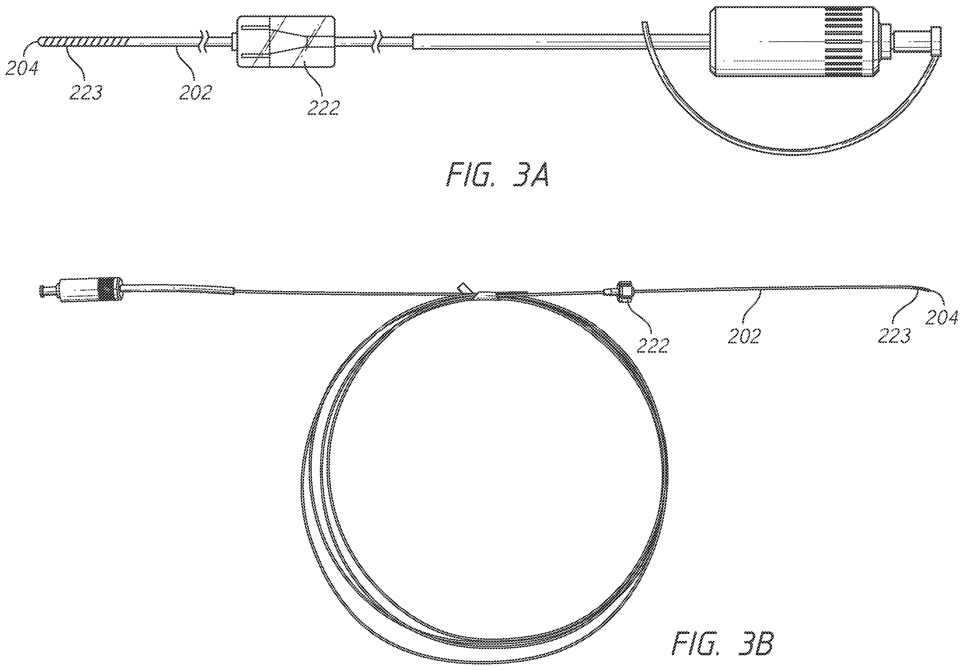

Figure 3A illustrates an embodiment of an optical fiber that can be inserted into the laser catheter assembly of Figure 2A.
Figure 3B is a view of an optical fiber as shown in Figure 3A.

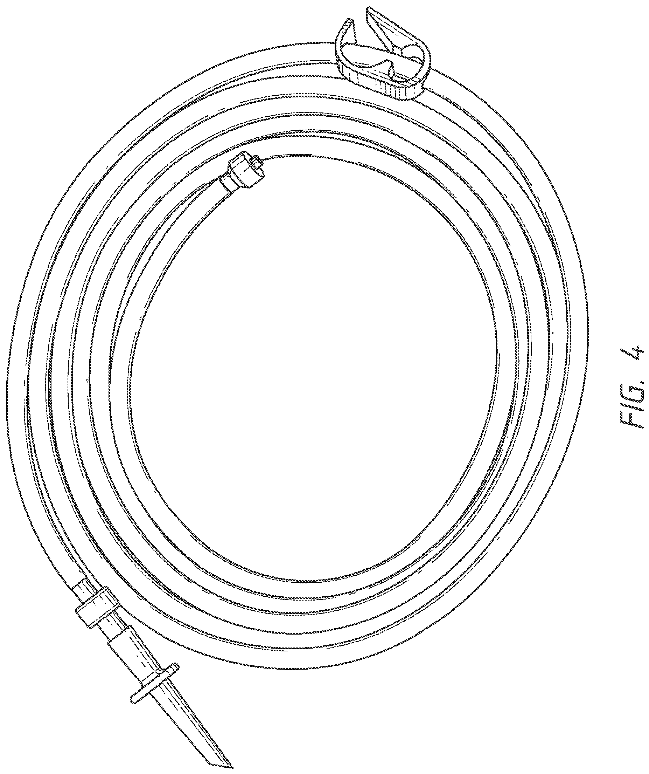

Figure 4 is a view of an embodiment of a coolant inlet conduit.

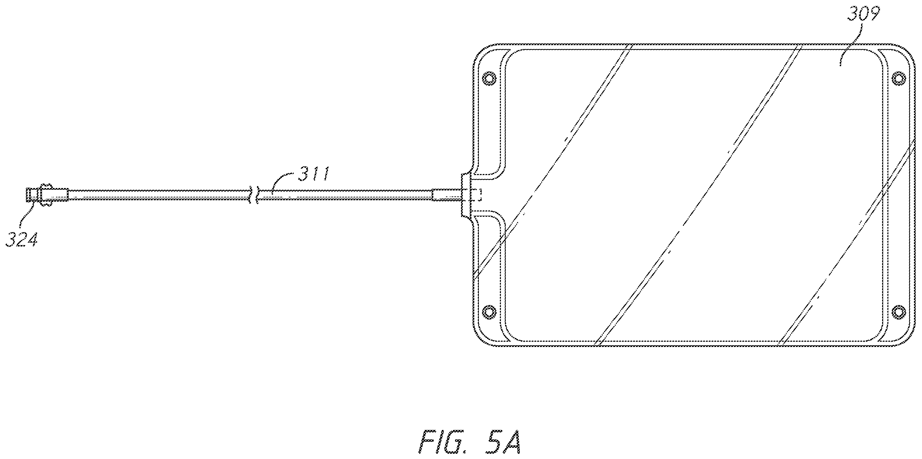

Figure 5A illustrates an embodiment of a coolant recovery bag.

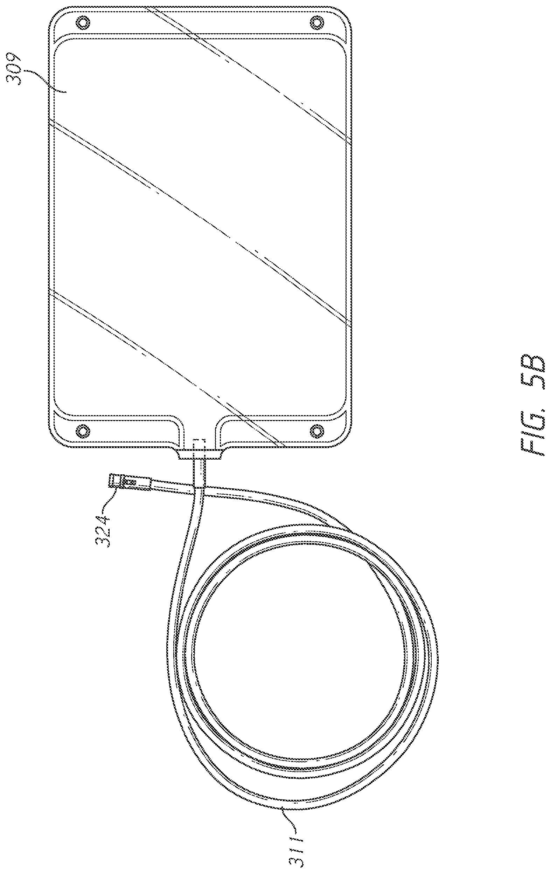

Figure 5B is a view of a coolant recovery bag as shown in Figure 5A.

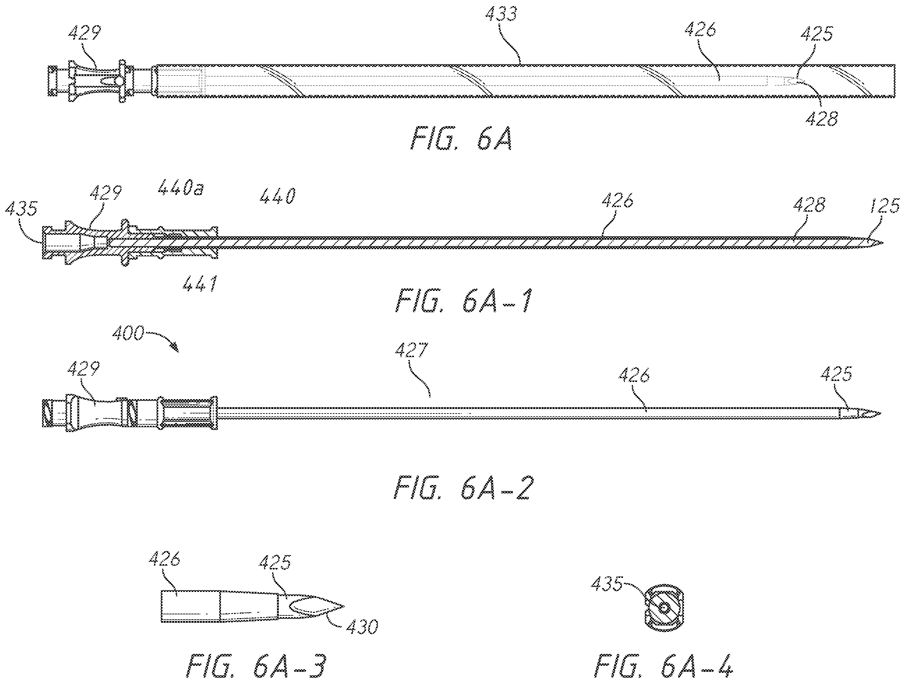

Figures 6A to 6B-4 illustrate views of a trocar sleeve assembly.

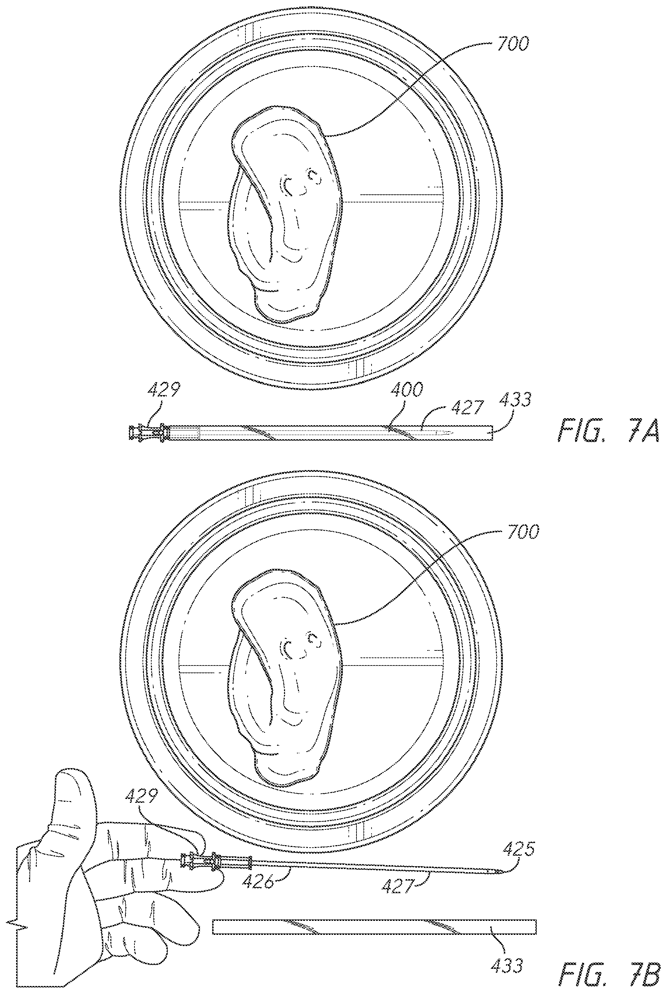

Figure 7A to 7K illustrate the insertion and illumination of a test tissue using an embodiment of a laser catheter assembly described herein.

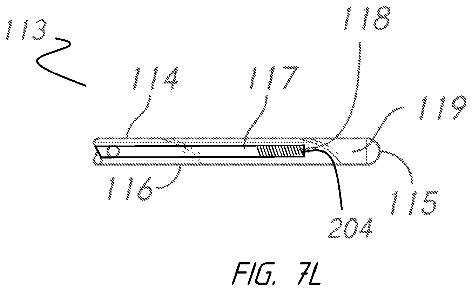

Figure 7L illustrates an embodiment of a probe tip housing a waveguide.

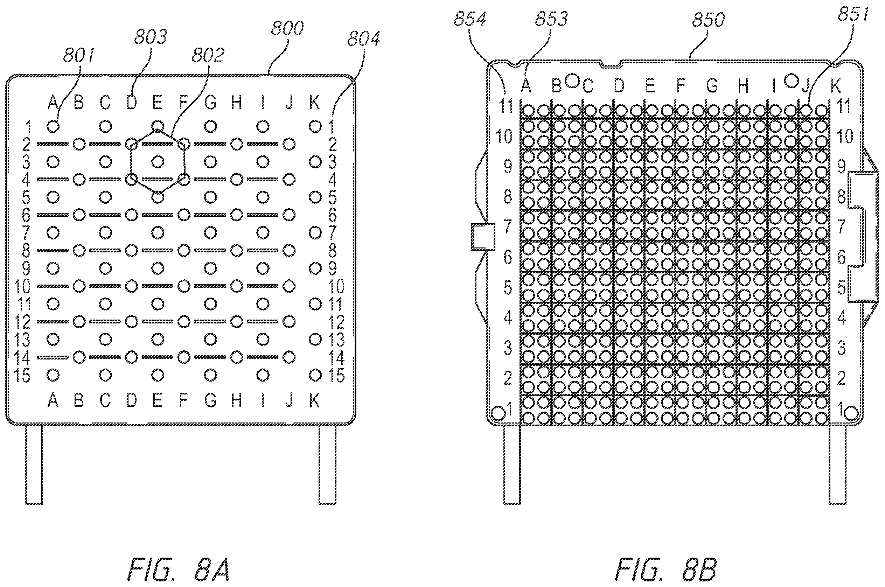

Figure 8A depicts an embodiment of a hexagonal grid template for laser probe placement.
Figure 8B depicts an embodiment of a square grid template.

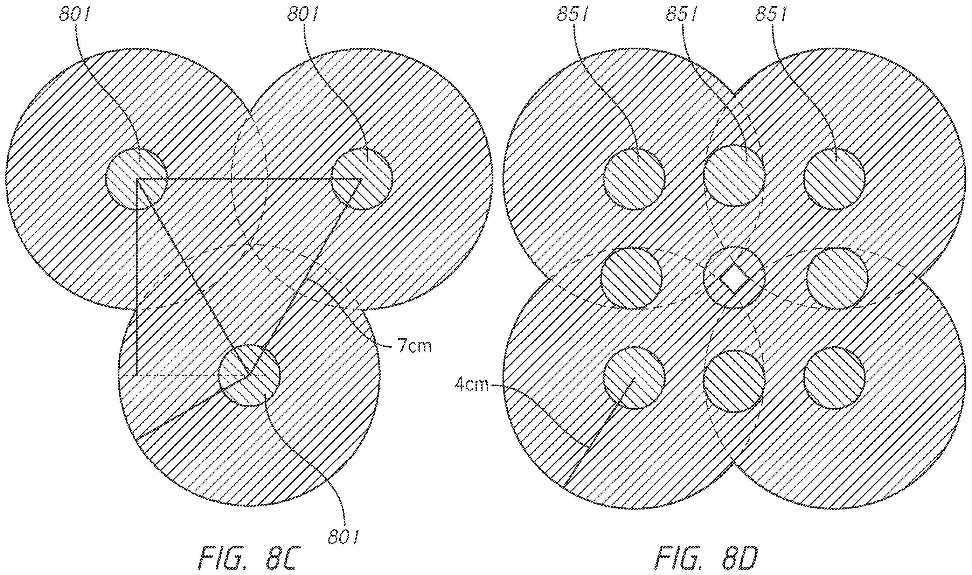

Figure 8C depicts an embodiment of laser exposure regions using a hexagonal grid template with 7 mm spacings between grid apertures.
Figure 8D depicts an embodiment of laser exposure regions using a square grid template with 3 mm spacings between grid apertures.

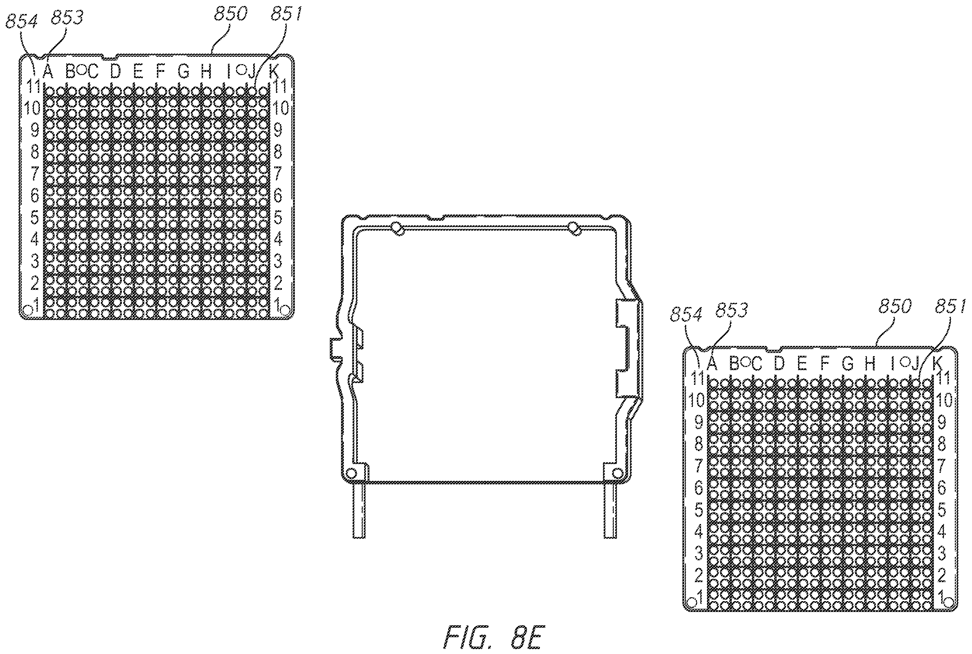

Figure 8E shows a view of a disassembled three-part square grid.

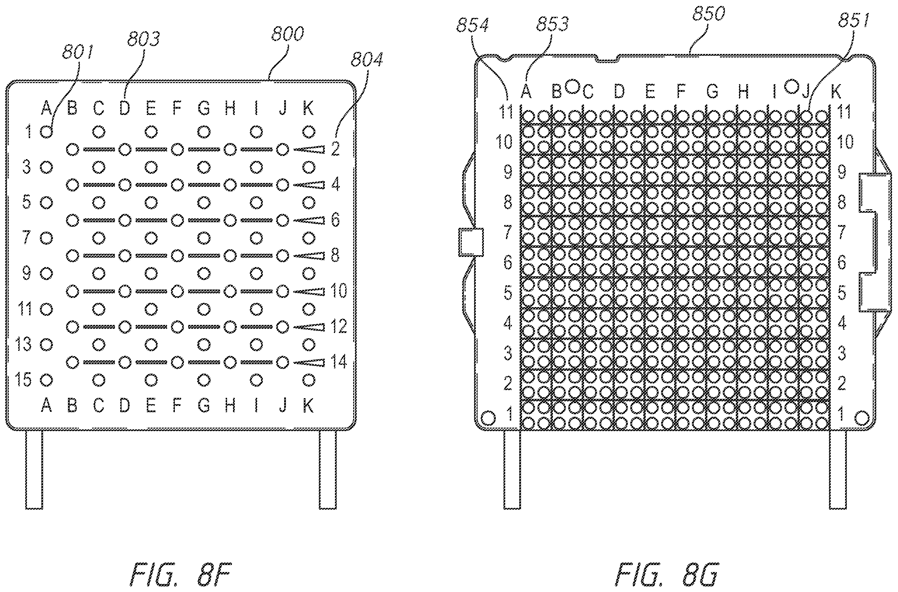

Figure 8F is a view of an embodiment of a monolithic hexagonal grid.
Figure 8G is a view of an embodiment of a three-part square grid.

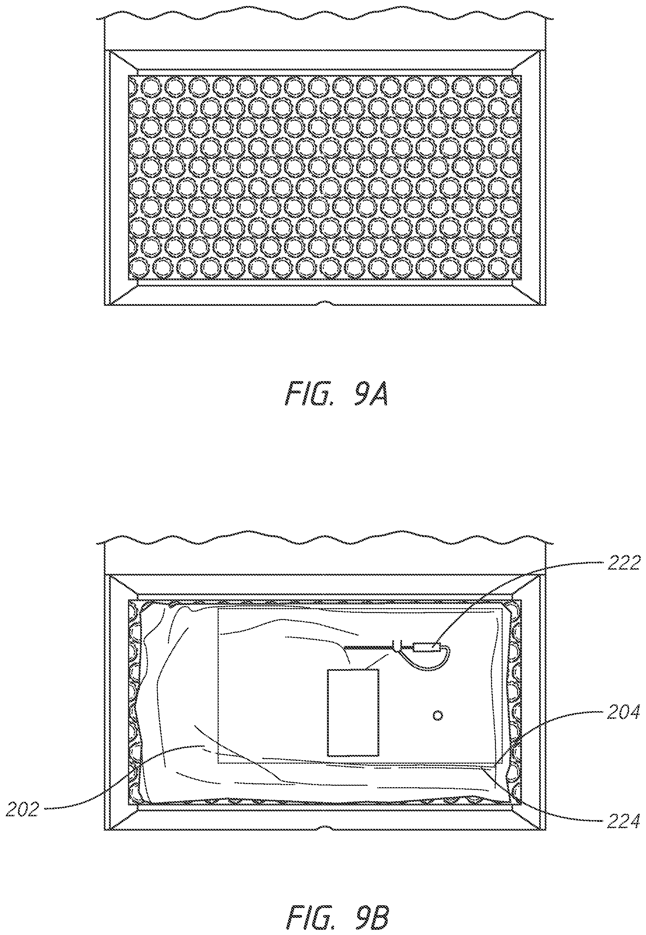

Figures 9A to 9G illustrate components of an embodiment of a laser illuminating system kit.

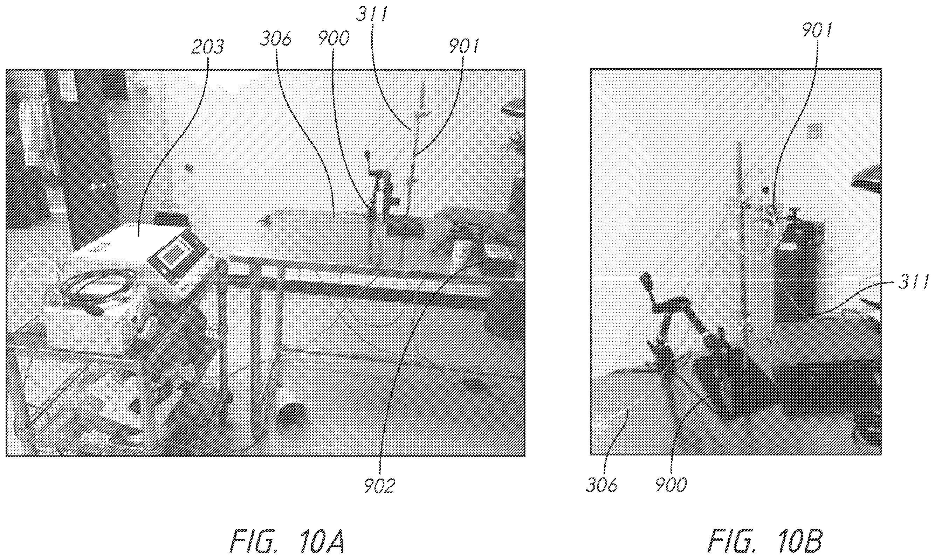

Figures 10A to 10B are photographs of an experimental set-up for the testing of a laser catheter having a conical tip and a laser catheter assembly having a transmissive tip.

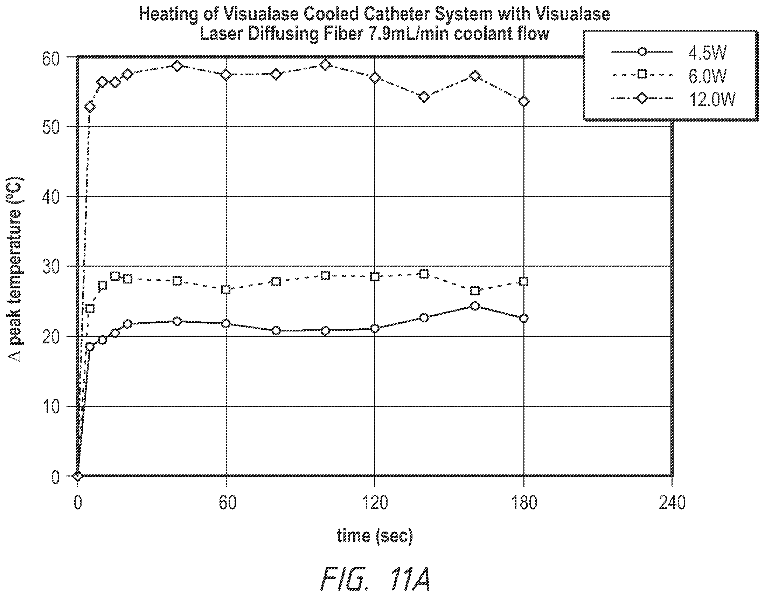

Figures 11A to 11B are graphs showing the heating of a laser catheter having a conical tip (11A) and of a laser catheter assembly having a rounded transmissive tip (11B).

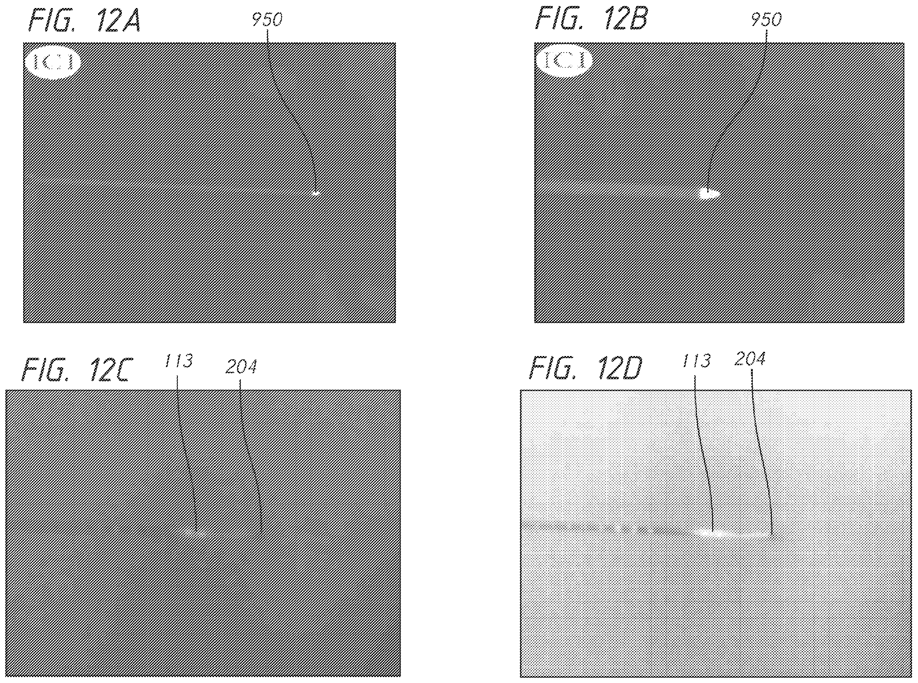

Figures 12A to 12B depict thermal images of the distal end of a laser catheter having a conical tip after 6.0 W irradiation.
Figures 12C to 12D depict thermal images of the distal end of a laser catheter having a domed tip after 6.0 W irradiation.

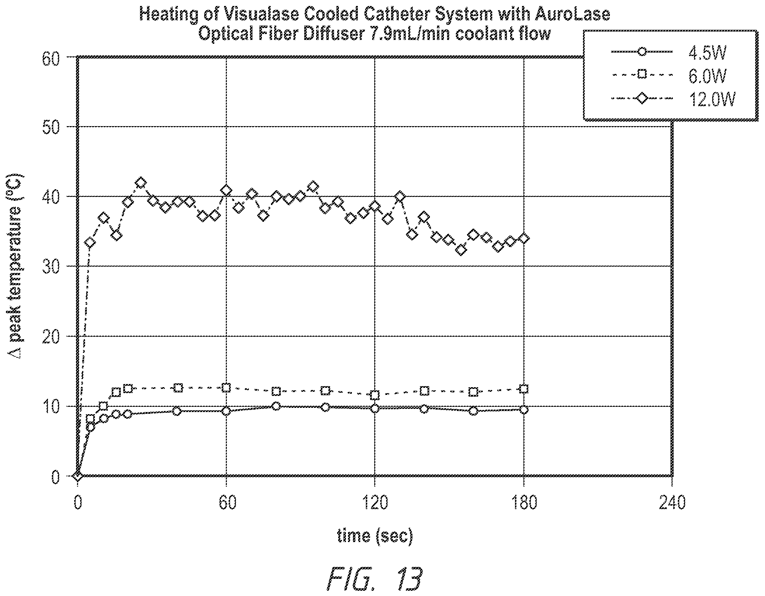

Figure 13 depicts the heating of a conical tip laser catheter when an optical fiber having an 18 mm diffuser is used.

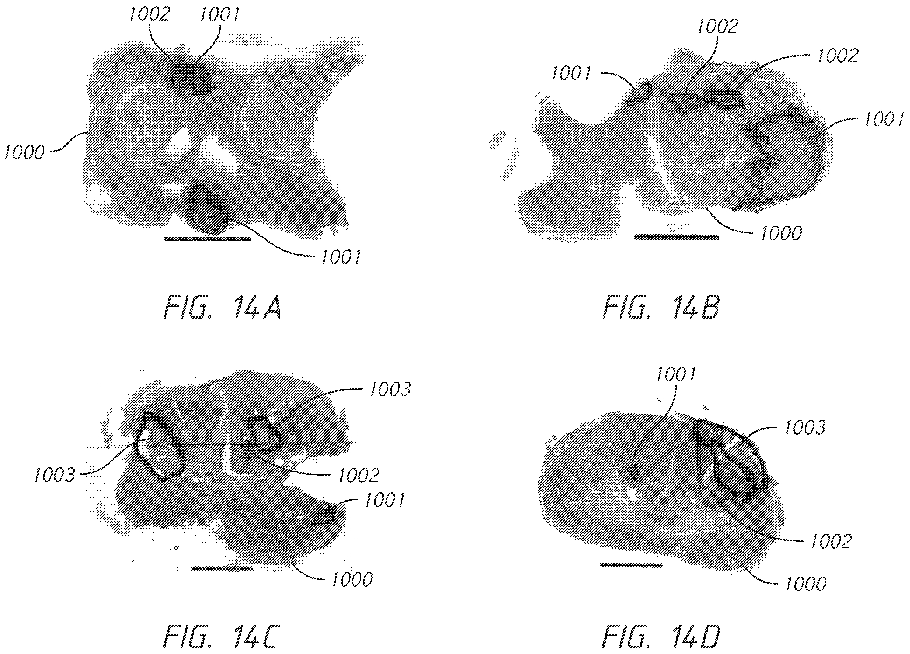

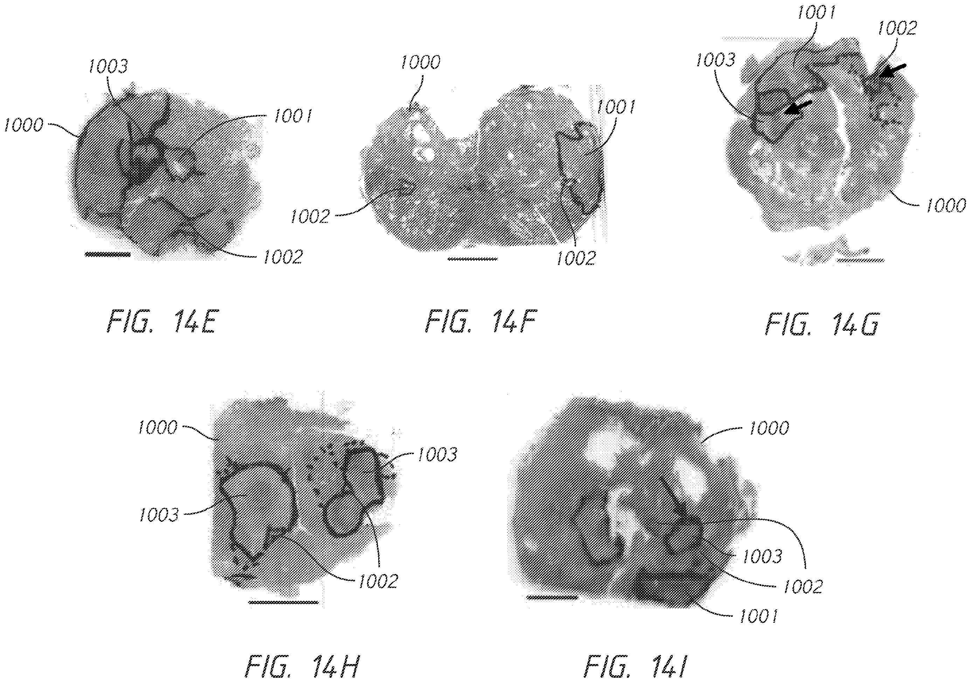

Figures 14A to 14I are photographs of 9 whole-mount sections of patients' prostates (scale bars = 1 cm).

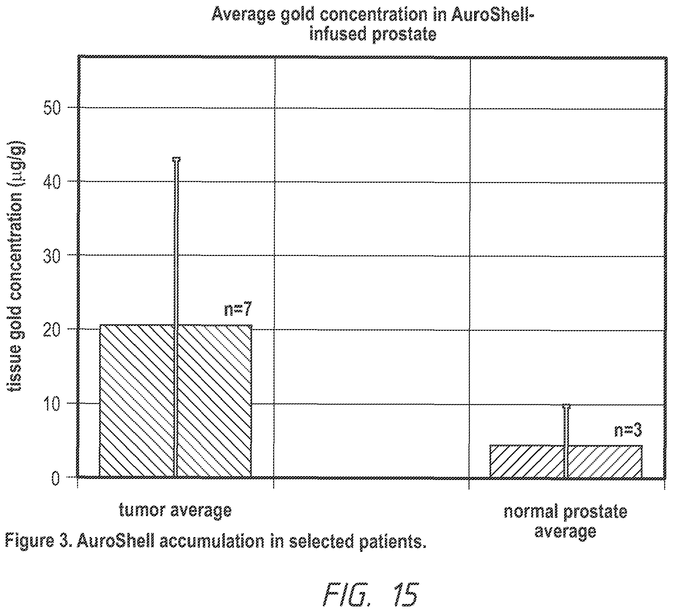

Figure 15 is a graph showing the relative accumulation of nanoparticles in samples tumor tissue versus healthy tissue from two patients.

### DETAILED DESCRIPTION

Several embodiments disclosed herein pertain to systems used for irradiating tissue and tumors with electromagnetic radiation, components and devices of that system, and kits for providing systems used for irradiating tissue and tumors with electromagnetic radiation. In several embodiments, the system can be configured to provide infrared radiation (e.g., to tissue of a patient). In several embodiments, the infrared radiation can be non-ablative (e.g., radiation that can be of insufficient intensity to ablate tissue by itself and/or sub-ablative radiation). In several embodiments, the non-ablative infrared radiation can be absorbed by nanoparticles that can be dispersed within or around a target tissue to be treated. In several embodiments, the nanoparticles absorb the radiation converting it into heat energy. In several embodiments, though the infrared radiation itself may be sub-ablative, the heat energy generated by the nanoparticles can be sufficient to cause thermal coagulation, hyperthermia, and/or tissue ablation. A variety of methods, devices, systems, and kits for treating tumors and ablating tissue are described below to illustrate various examples that may be employed to achieve one or more desired improvements. These examples are only illustrative and are not intended in any way to restrict the general inventions presented and the various aspects and features of these inventions. Furthermore, the phraseology and terminology used herein can be for the purpose of description and should not be regarded as limiting. No features, structure, or step disclosed herein can be essential or indispensable. Any of the systems or methods disclosed herein can exclude one or more steps or features described herein.

Photothermal coagulation as a therapeutic method can make use of the ability of laser energy to be transmitted through optical fiber in order to deliver particular amounts of energy interstitially into tissue. Laser Interstitial Thermal Therapy (LITT) can be the technique of delivering sufficient optical power to directly generate thermal coagulation in tissue. Essentially, optical energy can be delivered into tissue at a rate greater than the body's ability to remove the resulting heat. This heat can result in hyperthermia and tissue ablation.

However, the effective treatment depth of LITT and other photon-based therapies can be hindered by light scatter and absorption in tissues (including that caused by thermal coagulation). This scatter and absorption can result in light intensity that diminishes exponentially with increased distance from the light source into tissue. A further constraint is that light intensity, or irradiance (measured in W/cm²), also declines and/or decreases in the radial direction when delivered by a one-dimensional source (e.g., optical fiber diffusers used to deliver laser light interstitially into tissue). The net result can be that optical irradiance can be higher immediately adjacent to the optical source delivering it and much lower in areas just outside the immediate vicinity of the optical source (e.g., a diffuser). Photon-based processes of phototherapy are also non-specific; the shape of the region of coagulation can be a function of the shape of the energy source, and the volume of the region of coagulation can be dependent upon the total energy deposited. For example, current laser catheters used for photon-based processes use optical fiber diffusers and 12-15 W of power to generate ellipsoidal coagulation zones around the diffuser. Thus, selectivity to treat target tissue can be low.

Further, because current systems require high power irradiance, high laser temperatures occur that can destroy and/or deform laser delivery catheters. This also causes tissue char, which blocks the depth to which the radiation can penetrate into the tissue. Active cooling can be used to prevent carbonization of the tissue immediately around the laser delivery catheter and/or to prevent instrument damage. Nonetheless, even when cooled, many catheters have tips that absorb heat energy and cause it to melt the laser catheters. It has also been noted that catheter probes may be difficult to accurately place in tissues. Thus, doctors may be left to approximate the location of the laser catheter in the tissue, leaving some areas untreated and some areas overtreated. Further, the laser catheters may be quite fragile and subject to kinking and breakage, which can lead to aborted treatments and can also exacerbate heating and char issues. Several embodiments disclosed herein provide solutions to one or more of these problems or others.

Disclosed herein are methods, devices, systems, and kits configured to deliver radiation to generate photothermal coagulation, in particular systems and kits configured to deliver non-ablative radiation to generate particle-directed photothermal coagulation (e.g., using nanoparticles or other agents). In several embodiments, the disclosed techniques and devices allow improved selectivity in targeting of tissues and in effectively treating tissues. Several embodiments disclosed herein pertain to systems and devices used to generate radiation at a site where photothermal coagulation, hyperthermia, and/or tissue ablation can be desired.

In several embodiments, the devices pertain to photothermal coagulation used for treating tumors. In several embodiments, a laser probe of a laser catheter assembly can be positioned (e.g., inserted, placed, etc.) into or near the site of interest (e.g., in or near a tumor). In several embodiments, the laser catheter assembly can be activated to deliver electromagnetic radiation (e.g., ultraviolet light, visible light, near-infrared light, far-infrared light, microwave, etc.) to the tissue. In several embodiments, the radiation can be infrared radiation. In several embodiments, the laser catheter assembly delivers radiation that can be of sufficient intensity to cause photothermal coagulation (e.g., radiation that can be ablative radiation), hyperthermia, and/or tissue ablation.

In several embodiments, the devices pertain to particle-directed (e.g., with nanoparticles) photothermal coagulation used for treating tumors. The methods can involve injecting nanoparticles into a patient with a tumor (or another tissue that can be to be targeted). The nanoparticles can be allowed to accumulate in the tumor over a defined period of time. Once the nanoparticles have accumulated in a site of interest, the laser probe of a laser catheter assembly can be positioned (e.g., inserted, placed, etc.) into or near the site of interest (e.g., in or near a tumor). In several embodiments, the laser catheter assembly can be activated to deliver electromagnetic radiation (e.g., ultraviolet light, visible light, near-infrared light, far-infrared light, microwave, etc.) to the nanoparticles. In several embodiments, the radiation can be infrared radiation. In several embodiments, the laser catheter assembly delivers radiation that can be of insufficient intensity to cause photothermal coagulation (e.g., radiation that can be non-ablative radiation and/or sub-ablative), hyperthermia, and/or tissue ablation by itself (e.g., in the absence of the nanoparticles). In several embodiments, the radiation intensity can be transduced into heat energy by the nanoparticles. In several embodiments, the heat transduced by the nanoparticles can be sufficient to cause ablative hyperthermia, tissue coagulation, and/or tissue ablation.

The nanoparticles used in the disclosed methods can be designed to absorb infrared radiation and convert it to heat energy. In several embodiments, gold nanoshells (e.g., AuroShells, etc.) can be used as nanoparticles that transduce the sub-ablative infrared radiation to ablative temperatures. While, gold nanoshells (e.g., AuroShells, etc.) are used as a representative nanoparticle for illustration, it should be noted that any nanoparticle (or microparticle) that absorbs infrared photons and transduces those photons to heat energy can be envisioned. Without being bound to any particular theory, it can be believed that absorption of a photon by a nanoshell effectively annihilates the photon and results in the transduction of its energy into heat, which can be emitted into the surrounding tissue. When a photon can be scattered by a particle (e.g., not absorbed), the scattered photon can be available for absorption elsewhere (e.g., by tissue, another nanoshell, etc.). Transducing nanoparticles include, among others: nanoshells (including gold-shell silica core nanoshells such as Auroshells, gold-gold sulfide nanoshells and other variants), solid nanospheres (gold, silver, etc.), metal nanorods (gold, silver, etc.), nanostars, hollow nanoparticles, nanocages, elliptical "nanorice," carbon particles, fullerenes, carbon fullerenes, metallic nanoparticles, metal colloids, carbon particles, carbon nanotubes, buckyballs, and any combination thereof.

The methods disclosed herein can be used to target tissues for treatment. In several embodiments, the target tissue can be tumor tissue. In several embodiments, the tumor targets include tumors caused by cancer. In several embodiments, the tumors can be those caused by colorectal cancer, brain cancer, lung cancer, breast cancer, head and neck cancer, pancreatic cancer, ovarian cancer, melanoma cancer, prostate cancer, and other forms of cancer. In several embodiments, the techniques disclosed herein can be suitable for treating tumors in a tissue and/or for preserving the function of healthy tissue while destroying the underlying tumor tissue. For example, in several embodiments, the techniques and devices disclosed herein can be used to specifically target tumor tissues or dysfunctional tissues. The loss of function of a particular healthy tissue can have devastating effects for a patient's quality of life. For example, surgical manipulation to remove cancer tumor tissue from the throat can cause loss of speech. Surgical manipulation to remove cancer tumor tissue from the prostate can cause loss of sexual function. In current methods of ablation, tissues can be heated based on the placement of a heating instrument in a tumor. The heating element can be activated, thereby killing tissue surrounding it, including healthy tissue. In several embodiments, the treatments disclosed herein can partially, substantially, and/or fully preserve the structure and/or function of the non-diseased underlying healthy tissue to promote or substantially preserve normal function of those underlying tissues.

The methods can involve injecting (e.g., infusing) nanoparticles into the patient systemically. Systemic introduction can involve the introduction of nanoparticles into the circulatory system and/or at a site within the body but away from or remote from the target site. In several embodiments, the nanoparticles can be introduced to a patient via parenteral administration routes (e.g., injection via the intravenous, intramuscular, sub-cutaneous, intralesional, intraperitoneal, etc.). These sites can be located in areas of the patient's body that are not proximally located to the site of treatment (e.g., at sites in the body other than the tumor site). For instance, nanoparticles accumulate preferentially within tumors largely as a result of their size and passive extravasation from the leaky, chaotic and immature vasculature of tumors; a phenomenon referred to as the "enhanced permeability and retention" (EPR) effect. In several embodiments, this passive accumulation allows targeted deposition of the radiation transducers and affords photothermal coagulation therapy that can be tumor-specific.

In several embodiments, after being introduced to the body, the nanoparticles can be allowed to passively accumulate at a tumor. In several embodiments, this passive accumulation can be achieved by the passage of a defined period of time. In several embodiments, the nanoparticles can be allowed to accumulate in the tumor for a period of time ranging from between equal to or at least about 12 hours and/or less than or equal to about 36 hours after infusion and/or injection. In several embodiments, the nanoparticles can be allowed to accumulate in the tumor site for a period of at least: about 12 hours, 24 hours, about 36, or ranges including and/or spanning the aforementioned values.

In several embodiments, the nanoparticles accumulate in the perivascular space of tumor neovasculature and serve as foci for laser energy. In several embodiments, after accumulation at a site of interest, laser and/or electromagnetic energy can be used to heat the nanoparticles. In several embodiments, this laser energy (e.g., electromagnetic energy) heats the nanoparticles to a sufficient temperature to cause thermal coagulation, hyperthermia, and/or ablation of target tissue.

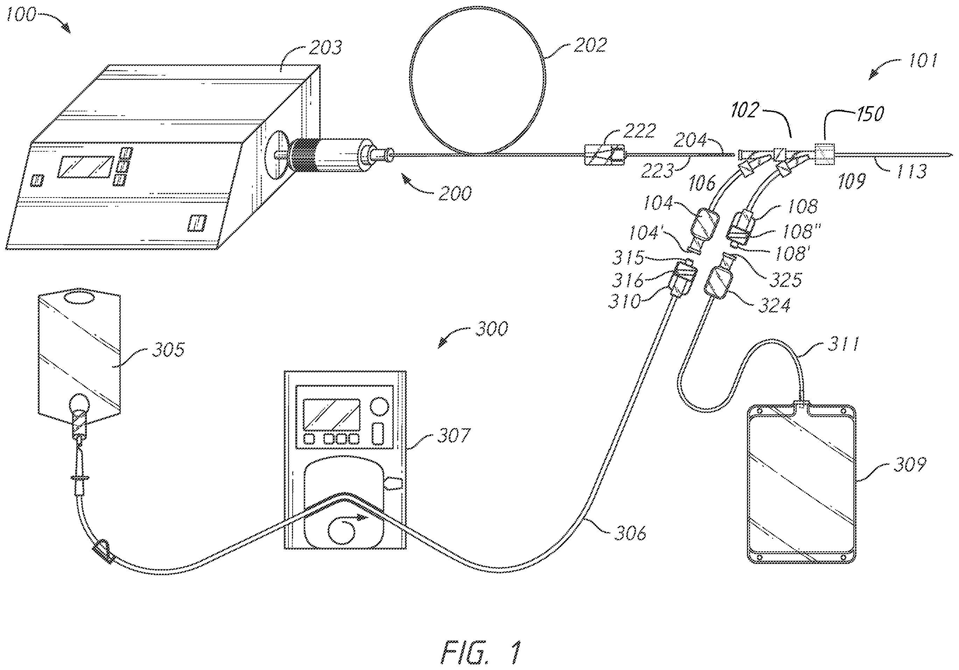

In several embodiments, laser energy can be generated at a target site for treatment using a laser illuminating system 100, as shown in Figure 1. In several embodiments, as shown in Figure 1, the laser illuminating system 100 can include a laser catheter assembly 101 (e.g., a laser illuminator). Though not shown in Figure 1, the system can further include a catheter sheath 426 (as shown in Figure 6A-6A-4). In several embodiments, though not shown in Figure 1, the system can include a trocar 425 (as shown in Figure 6A-6A-4). Though not shown in Figure 1, the system can further include a trocar assembly 400 (as shown in Figure 6A-6A-4), which can include a trocar 425 and a catheter sheath 426.

In several embodiments, as shown in Figure 1, the laser illuminating system can further include one or more of an illuminating system 200 and a cooling system 300. In several embodiments, the illuminating system 200 can include an optical fiber 202 and/or a laser source 203. In several embodiments, the optical fiber 202 can be configured to connect to, to be in optical communication with, and/or to receive laser energy from the laser source 203. In several embodiments, as disclosed elsewhere herein, the laser source, the optical fiber, and/or the laser catheter can be in optical communication. In several embodiments, the cooling system can include one or more of a coolant reservoir 305, a coolant pump 307, and/or a coolant recovery bag 309. In several embodiments, as disclosed elsewhere herein, the coolant reservoir, the laser catheter assembly, and/or the coolant recovery bag can be configured to be in fluidic communication with each other.

In several embodiments, the laser illuminating system includes the laser catheter assembly 101 but lacks one or more of the other features, such as the illuminating system 200 and/or the coolant delivery system 300 and/or individual components of any of the foregoing.

In several embodiments, as shown in Figure 1, as shown in Figure 1, the laser illuminator 101 can include a main body 102, hubs 106, 109, 120a configured to engage various features of a laser illuminating system (e.g., cooling fluid conduits, optical fiber connectors, etc.), a probe 113 (e.g., an introducer probe configured to be inserted into the body of and/or into a tissue of a patient), and a sheath connector 150.

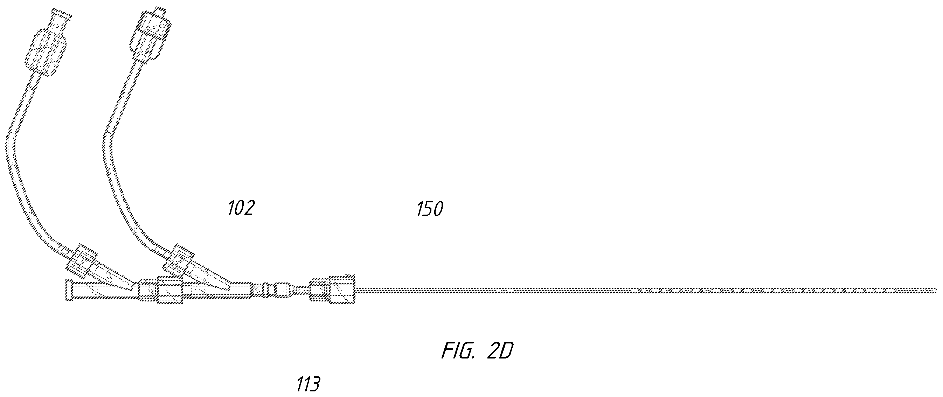

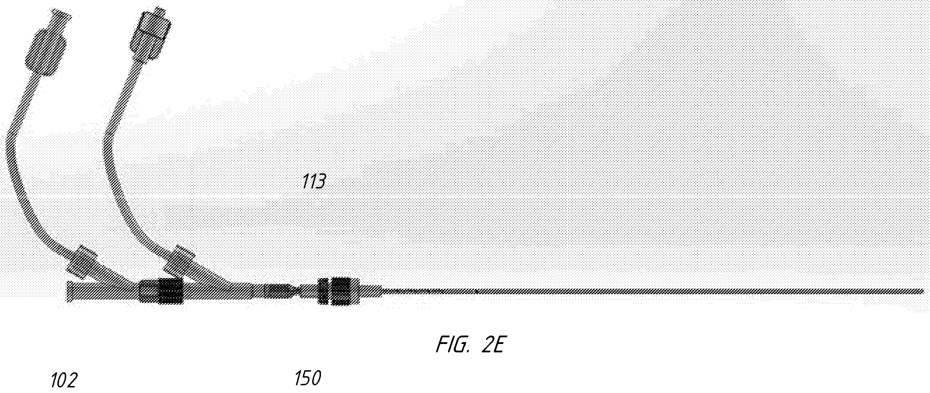

As shown in Figure 1 and as disclosed elsewhere herein, in several embodiments, the laser catheter assembly 101 can include one or more hubs 106, 109, 120a. As shown in Figure 2, these hubs 106, 109, 120a, may be located on and/or part of the main body 102 of the laser illuminator assembly 101. In several embodiments, as disclosed elsewhere herein, the hubs can include connection structures configured to engage various connectors.

In several embodiments, as shown in Figure 1, the main body 102 of the laser catheter assembly 101 can be configured to receive coolant via a hub 106. In several embodiments, the hub 106 can include a coolant inlet connector (not shown) or the hub 106 can be configured to engage a coolant inlet connector assembly having an inlet connector 104 (as shown in Figure 2A). As shown in Figure 2B, the coolant inlet connector 104 may be in fluidic communication with the main body via a catheter conduit 132 with a catheter conduit connector 104a and a hub mating connector 106a of the hub 106. In several embodiments, the coolant inlet connector assembly can include the coolant inlet connector 104, the catheter conduit 132, and a catheter conduit connector 104a. In several embodiments, the catheter conduit connector 104a and the hub mating connector 106a may include various connection structures, as disclosed elsewhere herein.

In some embodiments, the hub 106 can include connection structures and/or a connector configured to directly engage a corresponding inlet conduit connector 310 of a coolant inlet conduit 306 (not shown). For example, in several embodiments, the laser illuminator assembly may lack an intervening catheter inlet conduit 132 and separate coolant inlet connector 104.

In several embodiments, the hub 106 can include a port that provides an access point into the main body 102 of the laser illuminator 101. For instance, in several embodiments, the laser illuminating system 100 can include a coolant reservoir 305 (e.g., a bag, bottle, etc.). In several embodiments, when connected to the laser illuminator assembly 101, the coolant reservoir 305 can be in fluidic communication with the coolant inlet connector 104 via a coolant inlet conduit 306. In several embodiments, when in operation, coolant from the coolant reservoir 305 can be transferred into the laser catheter assembly 101 via the coolant inlet connector 104 and hub 106 through operation of a coolant pump 307. In several embodiments, the coolant inlet conduit 306 has an inlet conduit connector 310 that can be configured to interact with the coolant inlet connector 104.

In several embodiments, as shown in Figure 1, the main body 102 of the laser catheter assembly 101 can be configured to expel coolant via an outlet hub 109. In several embodiments, the hub 109 (e.g., an outlet hub) can include a coolant outlet (not shown) or can be configured to engage a coolant outlet assembly having a coolant outlet connector 108 (as shown in Figure 2B). As shown in Figure 2B, the coolant outlet connector 108 may be in fluidic communication with the main body 102 via a catheter conduit 127 with a catheter conduit connector 108a and a hub mating connector 109a of the hub 109. In several embodiments, the coolant outlet connector assembly can include the coolant outlet connector 108, the catheter conduit 127, and the catheter conduit connector 108a. In several embodiments, the catheter conduit connector 108a and the hub mating connector 109a may include various connection structures, as disclosed elsewhere herein.

In some embodiments, the hub 109 can include connection structures and/or a connector configured to directly engage a corresponding outlet conduit connector 324 of a coolant outlet conduit 311 (not shown). For example, in several embodiments, the laser illuminator assembly may lack an intervening catheter outlet conduit 127 and separate coolant outlet connector 108.

In several embodiments, the laser illuminating system 100 can include a coolant recovery bag 309 (e.g., a bag, bottle, etc.). In several embodiments, the coolant recovery bag 309 can be in fluidic communication with the coolant outlet connector 108 via a coolant outlet conduit 311. In several embodiments, when in operation, coolant from the laser catheter assembly 101 can be transferred into the coolant recovery bag 309 via the coolant outlet connector 108. In several embodiments, the coolant outlet conduit 311 has an outlet conduit connector 312 that can be configured to interact with the coolant outlet connector 108.

In several embodiments, as shown in Figure 2A, the inlet and outlet connectors 104, 108 of the laser catheter assembly 101 may be of a different sex. In several embodiments, this configuration can prevent incorrect connection and/or mismatching of the inlet and outlet connectors of the laser catheter assembly 101 to the coolant supply and recovery attachments (e.g., preventing backward connection). For example, as shown in Figure 1, the coolant inlet connector 104 can be a female connector with a female receiving portion 104' and the coolant supply inlet 310 can have a male connection with a male protrusion 315 and optionally a shroud 316 (e.g., a hood, a threaded shroud, etc.) while the coolant outlet connector 108 can be a male connector with a male protrusion 108' and optionally a shroud 108" (e.g., a hood, a threaded shroud, etc.) and the coolant outlet conduit connector 324 can have a female connection with a female receiving portion 325. In several embodiments, the coolant inlet connector 104 can be a male connector while the coolant outlet connector 108 can be a female connector (not shown). In several embodiments, instead of or in addition to having different sexes, the inlet connector 104 and outlet connector 108 can be color-coded to match a color on the inlet conduit connector 310 and the outlet conduit connector 324 (not shown). For example, in several embodiments, the inlet connector 104 and the inlet conduit connector 310 could be one color (e.g., red, orange, yellow, green, cyan, blue, indigo, violet, purple, magenta, pink, brown, white, gray, black), and the outlet connector 108 and the outlet conduit connector 324 could be of another different color (e.g., red, orange, yellow, green, cyan, blue, indigo, violet, purple, magenta, pink, brown, white, gray, black). In several embodiments, the male and female connectors can be luer connectors (which in several embodiments can include an ISO 594-compliant luer taper). In several embodiments, the inlet and outlet connectors can be optionally not of a different sex.

In several embodiments, the laser illuminator assembly can include a sheath connector 150 configured to engage a corresponding connector (e.g., its mate). In several embodiments, the mate for the sheath connector 150 can be a connector of a catheter sheath 440. In several embodiments, the sheath connector 150 can include connection structures and the connector of the catheter sheath 440 can include corresponding connection structures. In several embodiments, when the sheath connector 150 is engaged to a connector of the catheter sheath 440, the catheter sheath 426 can be configured to at least partially cover the probe 113. As shown in Figures 2A-2E, the sheath fitting 150 (e.g., sheath connector) may disposed, at least in part, circumferentially around at least a portion of the elongate probe member. In other embodiments, not shown, the sheath fitting is optionally not circumferentially disposed around the probe. For example, the sheath connector may be a hinge that engages a tooth or lip of the catheter sheath (not shown). In several embodiments, the sheath fitting can be connected to and/or unitary with the main body of the laser illuminator assembly. For instance, the sheath connector may be welded to, molded to, or molded with the main body of the laser illuminator. In other embodiments, the sheath connector can be connected to and/or unitary with the elongate probe member. For example, the sheath connector may be welded to, molded to, or molded with the probe of the laser illuminator.

In several embodiments, as disclosed elsewhere herein, the sheath connector 150 and/or any other connector disclosed herein (such as the fluid inlet and outlet connectors, waveguide/optical fiber connectors, trocar connectors, the connector of the catheter sheath, corresponding mating connectors for any of the foregoing, etc.) can include one or more connection structures. These connection structures can include one or more ridges, threads, clasps, arms, latches, protrusions, recesses, or combinations of any of the foregoing. The connection structures may include a taper (e.g., configured to engage a corresponding taper of its mating connector).

In several embodiments, the connection structures of a first connector (e.g., a sheath connector 150) may be configured to help guide, attach, and/or retain a corresponding connector (e.g., a connector of a catheter sheath 440) into engagement to couple with the corresponding connector. In several embodiments, the first connector (e.g., a sheath connector 150) engages a corresponding connector (e.g., a connector of a catheter sheath 440) through a friction (e.g., providing a friction-fit). In several embodiments, the connection structure can include a screw thread. In some embodiments, at least a portion of the screw thread can be oversized. In several embodiments, the screw thread can include a disconnection-resistant feature (e.g., a locking feature, a barb, threads that widening the more the connectors can be twisted into one another, etc.). In several embodiments, to disconnect a connector after the connector can be engaged with its mate, the disconnection resistant feature requires an initial force to overcome the hold of disconnection resistant feature and a different, smaller force thereafter.

In several embodiments, a mated pair of connectors (e.g., connector mates, such as, a sheath connector 150 and a connector of a catheter sheath 440) may be male and female luer connectors (e.g., can include connection structures that can be male and female components of luer connectors, respectively). In several embodiments, the connectors can include an ISO 594-compliant luer taper. For example, the connector of a catheter sheath 440 can be a female connector with a female receiving portion and the sheath connector 150 can have a male connection with a male protrusion (which may provide a portion of or transition directly from the probe 113). In several embodiments, as shown in Figure 2A, the sheath connector can optionally include a shroud 151 (e.g., a hood, a threaded shroud, etc.) and the connector of a catheter sheath 440 can have a female connection with a female receiving portion 441.

As shown in Figures 2A and 2B, in several embodiments, the laser catheter assembly 101 can include an introducer probe 113. In several embodiments, as shown in Figure 2A, the laser catheter assembly 101 can include one or more of an outlet arm unit 125, an outlet arm 126, and inlet arm unit 130, and an inlet arm 131. In several embodiments, the introducer probe 113 can include an elongated tube (e.g., an elongate probe member) that extends from a proximal introducer probe connector seal 135 (e.g., a seal, sleeve, etc.) to a sealed end 115 of the introducer probe 113.

In several embodiments, the introducer probe 113 can include an outer tubular section 114 (e.g., an external wall that defines a tubular cavity within the introducer probe). In several embodiments, as shown in Figure 2A and 2B, the outer tubular section 114 can be a continuous material (e.g., a single piece molded out of a single material, a unitary structure, etc.). In several embodiments, the outer tubular section 114 can be transparent or substantially transparent to certain wavelengths of electromagnetic radiation, for example, one or more of ultraviolet light, visible light. near-infrared light, far-infrared light, and/or microwave radiation.

In several embodiments, the outer tubular section 114 can include a sealed end 115. In several embodiments, the sealed end 115 can be clear, optically and/or infrared transparent, and/or substantially optically and/or infrared transparent. In several embodiments, the sealed end 115 can be transparent or substantially transparent to certain wavelengths of electromagnetic radiation, for example, one or more of ultraviolet light, visible light, near-infrared light, far-infrared light, and/or microwave radiation. In several embodiments, the sealed end 115 can be transmissive. In several embodiments, the sealed end 115 allows electromagnetic radiation from a laser source 203 to be transmitted through it (e.g., via a diffuser tip 204 as disclosed elsewhere herein). In several embodiments, as shown in Figure 2A-2, the sealed end 115 can be shaped to distribute light from the optical fiber 202 in line with the introducer probe 113, in a conical distribution 140 expanding outwardly and distally from the sealed end 115, or in a semi-spherical distribution 141 expanding outwardly and distally from the sealed end 115. In several embodiments, the distribution of light (conically or semi-spherically) can be diffused such that, within the distribution, the amount of radiation at a given distance can be substantially or approximately of the same intensity (e.g., at points 140A and 140B and/or at points 141C and 141D. In several embodiments, the end 115 can be configured to allow radiation and/or light to pass through it without substantially absorbing it.

In several embodiments, the optical fiber tip 204 can be configured to inhibit the passage of radiation through the tip and/or the optical fiber tip 204 substantially blocks radiation. In several embodiments, the diffuser tip 223 can be configured to emit a majority and/or substantially all and/or all of the radiation received via the radiation source laterally (e.g., sideways from the diffuser tip) and not along the path of the optical fiber 202 (e.g., through the optical fiber tip 204). In several embodiments, a diffusive tip that emits radiation laterally (e.g., to the sides) advantageously allows the treatment of tissues adjacent to the diffuser tip 223. In several embodiments, this configuration prevents and/or lowers the amount of heating and/or of irradiation of tissue directly in front of the fiber tip 204.

In several embodiments, as noted elsewhere herein, a diffuser tip 223 (e.g., of a waveguide) can be configured to distribute electromagnetic radiation laterally from the introducer probe (e.g., when the waveguide is engaged with the laser illuminator assembly via the optical fiber connector 222). In several embodiments, the intensity of electromagnetic radiation from the diffuser can be approximately or substantially equal at equal distances from the diffuser tip 223. In several embodiments, light emitted from the introducer probe 113 can be distributed and/or not focused. In several embodiments, light emitted from the introducer probe 113 can be distributed substantially evenly. In several embodiments, because light can be distributed from the diffuser along the surface of the diffuser, the radiation emitted does not cause shadows (e.g., places where the radiation can be blocked by a component of the laser catheter, etc.) and/or focal points. In several embodiments, the laser catheter assembly and/or the diffuser tip lacks a focusing lens and does not include a mirror.

In several embodiments, the sealed end can be domed (e.g., hemispherical, having the shape of a hemisphere, semi-spherical, dome-shaped, etc.). In several embodiments, as described elsewhere herein, the sealed-end can be transmissive. In several embodiments, transmissive means allowing all, substantially all, or part of the electromagnetic radiation from the diffuser tip 223 to pass. In several embodiments, the outer tubular section 114 can be continuous with the sealed end 115 (e.g., the sealed end and the outer tubular section can be a single piece molded out of a single material, a unitary structure, etc.). In several embodiments, the transmissive sealed end 115 has the benefit of reducing the build-up of heat in the tip of the probe. This feature helps avoid melting of the probe and also allows the laser to be distributed into tissue along the direction of the probe. In several embodiments, the sealed end can be optionally not cone-shaped and/or not a ground material (e.g., a scattering material, an opaque or substantially opaque material, etc.). In several embodiments, it has been found that round and/or cone configurations do not allow radiation to pass through the introducer probe. In several embodiments, it has been found ground and/or conical configurations can concentrate radiation causing hot spots and heating that can lead to warping and/or charring of a probe and/or burning of tissue around the probe.

In several embodiments, the domed tip can be prepared using radio frequency. In several embodiments, the domed tip can be formed from a nylon extrusion, which can be placed over a mandrel, and then inserted into a stainless steel die. In several embodiments, the die can be heated with RF energy, while the extrusion can be simultaneously pushed into the cavity using pneumatically-actuated grippers, which causes the tip of the extrusion to be reflowed/reshaped over the end of the mandrel. In several embodiments, the RF machine can be set with temperature, pressure, and time settings that are specific to extrusion so it can produce very repeatable tips. In several embodiments, the tip can be optionally not hemispherically shaped, but can be another shape that allows the passage of radiation without substantial build-up of heat or concentration of radiation (e.g., a rounded cubical end shape, a cubical end shape, a rounded cylinder end shape, a cylinder end shape, etc.).

In several embodiments, as shown in Figure 2A1, the outer tubular section 114 can include a lumen 116 (e.g., a first lumen). In several embodiments, an internal tube 117 (e.g., an internal tubular member) resides within the first lumen 116. In several embodiments, the internal tube 117 can include a second lumen 118. In several embodiments, as shown in Figure 2A and Figure 2B, the laser catheter assembly 101 can be configured to receive coolant via the coolant inlet connector 104, though a catheter inlet conduit 132, through the inlet arm unit 131, and into a body of the inlet arm unit 130 that provides a fluid chamber 103b. The coolant then may travel via the internal tube 117, to the open pool 119, through the first lumen 116, to another fluid chamber 103a, through the outlet arm unit 125, through the outlet arm 126, into the catheter outlet conduit 127, and out via the coolant outlet connector 108. In other words, in several embodiments, one or more of the coolant inlet connector 104, the catheter inlet conduit 132, the inlet arm unit 131, the inlet arm unit 130, the internal tube 117, the open pool 119, the first lumen 116, the outlet arm unit 125, the outlet arm 126, the catheter outlet conduit 127, and/or the coolant outlet connector 108 can be in fluidic communication. In other embodiments, not shown, the laser illuminator assembly lacks one or more of one or more of a catheter inlet conduit, an inlet arm unit, an outlet arm, and a catheter outlet conduit. For example, the hubs 106, 109 may be directly placed on the main body 102 of the laser illuminator assembly 101 (not shown).

In several embodiments, the introducer probe 113 extends from a proximal end to a distal end, the sealed domed end 115 of the outer tubular section 114 being located at the distal end. In several embodiments, the internal tube 117 terminates a distance away from the sealed domed end 115 leaving an open pool 119 at the distal end of the introducer probe 113. In several embodiments, when the optical fiber can be positioned in the introducer probe 113, the terminal end of the diffuser tip 223 terminates at or approximately at the distal end of the internal tube 117. In several embodiments, a portion of the diffuser tip 223 protrudes from the internal tube 117 in the open pool 119. In several embodiments, this protrusion allows a more concentrated amount of electromagnetic radiation to penetrate the sealed end 115 of the introducer probe 113. In several embodiments, the open pool provides an area around which coolant can flow without substantial restriction by the fiber tip 204 (e.g., blocking and/or restricting coolant flow within the lumens).

In several embodiments, the laser catheter assembly 101 can include a waveguide hub 120a. In several embodiments, the waveguide hub 120a can include an aperture 120 (e.g., an end aperture). In several embodiments, the waveguide hub 120a can include a connection feature 120b configured to engage a waveguide connector 222. In several embodiments, the laser catheter assembly 101 can be configured to receive the optical fiber 202 into the introducer probe 113 via an end aperture 120. In several embodiments, the internal tube 117 can be configured to receive the optical fiber 202 within the second lumen 118. In several embodiments, the optical fiber connector 222 couples with features on the end aperture 120 to provide a fluid-tight seal (e.g., the waveguide hub can include a connector with one or more connection structures). In several embodiments, the coupling fixes the diffuser tip 223 in a position within the introducer probe 113. In several embodiments, the laser catheter assembly 101 receives only one optical fiber 202 and the introducer probe 113 and internal tube 117 can be sized and/or configured to receive only a single optical fiber tip 204 and/or diffuser tip 223.

In several embodiments, as noted elsewhere herein, the second lumen 118 can be in fluidic communication with the coolant inlet connector 104. In several embodiments, as noted elsewhere herein, the first lumen 116 can be in fluidic communication with the coolant outlet connector 108. In several embodiments, when the optical fiber 202 is positioned within the second lumen 118, the laser catheter assembly can be configured to allow the passage of a coolant from the coolant inlet connector 104 through the second lumen 118 into the first lumen 116 and out of the coolant outlet connector 312.

In several embodiments, the lumen of the laser catheter assembly 101 introducer probe 113 can be formed from a material such as nylon, PA12 nylon, or a similar material. In several embodiments, PA12 nylon, as opposed to a material such as polycarbonate, advantageously smooths the introducer probe's 113 passage through introducers and tissue.

In several embodiments, as shown in Figure 2A, the introducer probe 113 can include printed "hash" marks 121. In several embodiments, the introducer probe 113 can be graduated (e.g., with numbering, lettering, etc.). In several embodiments, hash marks and/or graduations can clearly mark the depth of penetration of the probe into tissue (and/or the distance the probe has been withdrawn from tissue). As disclosed elsewhere herein, in several embodiments, the hash marks (e.g., graduations) can be located on a proximal portion of the probe. In several embodiments, the hash marks can be optionally not located on a distal portion of the probe. For example, in several embodiments, the may extend equal to or less than about: 1/5 of the way down the probe, 1/4 of the way down the probe, 1/3 of the way down the probe, 1/2 of the way down the probe, or ranges including and/or spanning those values. In several embodiments, the hash marks can be distanced equal to or less than: about 4 mm apart, about 8 mm apart, about 12 mm apart, values between the aforementioned values, or ranges spanning and/or including those values. In several embodiments, alternating hash marks and dots at intervals permit the pullback of the laser catheter assembly 101 through tissue for multiple treatments with various optical diffusers (e.g, 1 cm diffusers, 1.8 cm diffusers, etc.), as discussed elsewhere herein. In several embodiments, these graduations and/or hash marks allow the probe depth to be visualized directly by a user. In several embodiments, these graduations and/or hash marks allow for fine control of the location of the probe allowing effective and/or controlled ablation of tissue. In some embodiments, the entire or substantially entire length of the probe can be graduated. In several embodiments, the introducer probe can be of a length equal to or less than about: 50 cm, 30 cm, 20 cm, 10 cm, values between the aforementioned values, or ranges including and/or spanning those values.

In several embodiments, as disclosed elsewhere herein, the optical fiber 202 can include a diffuser tip. In several embodiments, the diffuser tip 223 can be a portion of the optical fiber 202 having radiation scattering features (e.g., topography, bumps, roughenings, dimples, etc.) that encourage and/or allow the radiation (e.g., laser light, photons, radiation) to exit the optical tip in different directions (e.g., scatter, diffuse, etc.). In several embodiments, these features can be provided as a gradient along the diffuser and can be less dense closer to the laser source 203 and denser farther from the laser source 203. In several embodiments, the density gradient encourages a substantially even distribution of radiation along the entire diffuser tip. In several embodiments, the diffusive portion 223 of the optical fiber 202 may be of different lengths. In several embodiments, the diffusive portion of the optical fiber may be of a length equal to or less than about: 50 mm, 30 mm, 18 mm, 10mm, values between the aforementioned values, or ranges including and/or spanning those values. In several embodiments, the longer diffuser permits the treatment of larger tumor volumes in the same amount of time by using a higher laser power and distributing it along a longer diffuser. In several embodiments, using the smaller diffusive tip allows less laser power to be used and allows treatment of smaller sized tumors with greater specificity. In several embodiments, a single treatment can use various sizes of diffusers to tailor treatment to specific areas of the body and towards specific tumor dimensions. In several embodiments, as shown in Figure 3B, the optical fiber can be supple and/or flexible and can be configured to move within the introducer probe without kinking or cracking. In several embodiments, the optical fiber has a length sufficient to allow the laser source to be placed out of the way during an operation. In several embodiments, the length of the optical fiber can be equal to or less than: 1 m, 2 m, 3 m, 5 m, or ranges including and/or spanning the aforementioned values.

To illustrate one or more improvements achieved using design features of the disclosed laser catheter assembly, the following exemplary illustration is provided. In several embodiments, hash marks located along the introducer probe enable a user to determine the location of the laser catheter diffuser tip and the extent of treatment to a tumor being targeted. In several embodiments, the initial positioning of the introducer probe (e.g., depth to be inserted into the body along a z-axis and lateral positioning along the x and y axes) can be determined using Computer Tomography. Once the initial position is reached, the hash marks can be used to pinpoint treatment locations. The probe can be withdrawn from one position to the next at set spacings determined by the graduations on the probe. The printing on the laser catheter assembly provides a "depth gauge" for the user to know how far the laser catheter assembly has penetrated into tissue or how far the probe has been withdrawn from a starting position. In several embodiments, for example, 8 mm spacing between hash marks and the 8 mm spacing between "dots" halfway between the hash marks provide a 4 mm spaced "ruler" to permit accurate "pullback". In several embodiments, as described elsewhere herein, a grid can be used to aid in proper positioning of the diffuser tip of the laser catheter assembly within tissue laterally. In several embodiments, as disclosed elsewhere herein, an 8 mm pullback against a 10 mm grid permit can be used. These markings can provide a straightforward way for the user to know how deep the laser catheter can be situated in the target tumor or tissue and to provide a straightforward means of incrementally pulling back on the catheter in order to produce a contiguous zone of ablation along the catheter track.

In several embodiments, as shown in Figure 3A and 3B, the optical fiber can include an optical fiber connector 122. In several embodiments, the optical fiber connector (or waveguide connector) can include one or more connection structures configured to engage corresponding structures of the waveguide hub 120a of the laser illuminator assembly. For example, the waveguide connector 122 can include a luer connector, which in several embodiments can include an ISO 594-compliant luer taper. In several embodiments, the optical fiber connector can be bonded at a fixed distance from the distal end of the optical fiber tip 204. In several embodiments, as disclosed elsewhere herein, the optical fiber connector 122 can be configured to engage with the end aperture 120 of the laser catheter assembly 101. In several embodiments, the fixed positioning of the optical fiber connector 122 can ensure proper setback of the diffuser tip within the laser catheter assembly 101. In several embodiments, fixing the optical fiber 202 within the laser catheter assembly 101 can help prevent melting of the tip and can ensure proper coolant flow around the diffuser. In several embodiments, fixing the optical fiber can help prevent the diffuser tip from sliding out of the laser catheter assembly 101. In several embodiments, fixing the optical fiber 202 within the laser catheter assembly 101 can help ensure that the laser diffuses properly out of the diffuser tip (e.g., by preventing a portion of the diffuser from exiting the inner internal tube 117 or preventing the diffuser from bottoming out against the probe 113 tip where it could, for example, occlude the flow of coolant and/or cause uneven distribution of light emitted). In several embodiments, fixing the fiber can also advantageously provide sufficient diffusion of light from the tip of the introducer probe 113 allowing treatment of the tissue in front of the tip (in addition to tissue laterally adjacent to the diffuser tip).

In several embodiments, the optical fiber has a total length in meters of less than or equal to about: 2, 3.5, 5, or ranges including and/or spanning the aforementioned values. This length advantageously lessens clutter in the operating room given that, in several embodiments, the entire laser illuminating system as disclosed herein can be of a size that allows it to be housed entirely in the operating room (e.g., in a sterile environment).

In several embodiments, the coolant supply system of the laser illuminating system 100 can include the coolant reservoir 305, the coolant inlet conduit 306, and the coolant pump 307. In several embodiments, the coolant supply system supplies coolant (e.g., saline, water, etc.) to the laser catheter assembly 101. In several embodiments, the coolant helps prevent early onset of photocoagulation adjacent to the laser catheter assembly 101 introducer probe 113. Early onset photocoagulation could detrimentally limit light penetration of laser radiation into tissue and/or nullify the tumor specificity of the optically excited nanoparticles.

Figure 4 shows an embodiment of coolant inlet conduit 306. In several embodiments, the length of the coolant inlet conduit 306 can be less than or equal to: about 3 m, about 4m, about 5 m, about 10 m, values between the aforementioned values, or ranges spanning and/or including those values. In several embodiments, the length of the coolant inlet conduit 306 reduces clutter in the operating setting. In several embodiments, the coolant inlet conduit 306 can be composed of flexible Tygon tubing (polyvinyl chloride tubing or the like, e.g., a flexible tubing) enabling it to be clamped into the cooling pump at any location along its length. In several embodiments, the tubing can be about 1/8" in diameter. In several embodiments, the diameter of the tubing can be less than about: 1/12", 1/8", 1/4", or ranges including and/or spanning the aforementioned values. In several embodiments, this diameter permits a smaller pump head spacing, greater control of coolant flow rate, and/or greater consistency of flow rate between tubing sets. In several embodiments, this feature removes the need for an external flow restrictor and meter in the return line. This feature permits greater precision in controlling the low flow (e.g., equal to or less than about 8 mL/min) coolant flow used during certain methods described herein. In several embodiments, the flow rate in mL/min that can be achieved can be less than or equal to 2, 4, 8, 10, or ranges including and/or spanning the aforementioned values. Figures 5A and 5B show an embodiment of a coolant recovery bag. As shown in Figure 5B, the coolant recovery bag can include a flexible length of tubing configured to attach to the laser catheter assembly.

In several embodiments, the coolant supply set design disclosed herein advantageously eliminates or reduces the problem of not being able to position a coolant pump relative to a reservoir that supplies the coolant. In several embodiments, the coolant supply system can be fabricated with a continuous length of tubing, thereby reducing the part count and complexity. In several embodiments, the use of polyvinyl chloride tubing of the internal diameters disclosed herein advantageously allows controlling the low flow (e.g., 8 mL/min) coolant flow.

In several embodiments, the coolant pump 307 has a flow rate adjuster that can be calibrated to default to a given speed in order to produce a given flow rate. In several embodiments, the pump can be a peristaltic pump (e.g., Langer BT100-1L). In several embodiments, the pump delivers flow rates in mL / min of less than or equal to about: 0.002, 0.01, 0.1, 1, 20, 50, 100, 500, or ranges including and/or spanning the aforementioned values. In several embodiments, the coolant pump 307 has quiet operation features and operates at decibel levels equal to or less than about: 10, 20, 30, 40, 50, 60, 70, 80, or ranges including and/or spanning the aforementioned values. In several embodiments, the quiet features of this pump can be beneficial because the disclosed system can be placed in an operating room with the patient.

In several embodiments, the pump 307 can be connected an actuating device (e.g., a footswitch, pedal, panel, button). In several embodiments, the actuating device can be turned on or off by a user (e.g., a physician or technician operating the pump). In several embodiments, the laser source can be connected to an actuating device (e.g., a footswitch, pedal, panel, button) that can be the same or different from the actuating device that controls the pump. In several embodiments, where the actuating device of the pump and the laser source can be the same, the actuating device can have one or more toggle switches (e.g., buttons or panels) that allow the user to control the flow of coolant through the laser catheter and/or to control the emission of laser by the laser source separately or simultaneously. In several embodiments, where the laser source and the pump are to be activated simultaneously, the actuating device can have a single switch for turning both the laser source and the pump on or off simultaneously. In several embodiments, where the laser source and the pump are to be activated separately, the actuator can have different switches to activate one device at a time.

In several embodiments, the actuator connection can allow coolant flow when the laser is active and stops coolant flow when inactive. In several embodiments, in addition to reducing the volume of coolant consumed (e.g., the number of coolant bag changes, etc.), this feature can advantageously prevent pre-cooling of the tissue between laser treatments. In several embodiments, the pump can be programmable to have a default flow rate when the laser is in operation. In several embodiments, control of the default flow rate of the pump can be helpful for the disclosed methods which can use of a nominally sub-ablative laser dose without using real-time temperature monitoring. In several embodiments, the default flow rate in mL / min can be less than or equal to about: 0.002, 0.01, 0.1, 1, 5, or ranges including and/or spanning the aforementioned values. The predetermined flow rate allows the user to remove heat at a pre-determined rate. In several embodiments, the automatic stopping of coolant flow when the laser is off helps avoid chilling tissue. Flowing coolant through the laser catheter assembly between laser treatments (e.g., when the laser is off) chills the surrounding tissue (e.g., from at or around 30-37°C) to the temperature of the coolant (e.g., at or around 21-23°C). In several embodiments, this has the effect of producing under-treatment because of the additional temperature gradient that must be overcome in order to produce tissue ablation. In several embodiments, combinations of the disclosed features allow the cooling to be tightly regulated. In several embodiments, this temperature control can advantageously delay tissue coagulation adjacent to the laser catheter until the end of the timed treatment. In several embodiments, since the increase in optical scatter that arises from coagulation effectively attenuates optical penetration into tissue, the delay of photocoagulation can aid in treating tumor tissue at longer distances from the laser catheter since nanoparticles are activated only by optical photons.

In several embodiments, the coolant recovery bag 309 collects the coolant after a single pass through the laser catheter assembly 101. In several embodiments, the coolant recovery bag 309 can be connected to the laser catheter assembly 101 via a coolant outlet conduit 311 and an outlet conduit connector 324 (see Figures 5A-B). In several embodiments, the coolant recovery bag 309 volume can be greater than or equal to about 1.5 liters, 2.0 liters, or 3 liters. In several embodiments, this volume allows for the collection throughout the duration of treatment. In several embodiments the treatment duration can be equal to or at least about 62 standard 3-minute treatments at 8 mL/min using the footswitch activated pump. In several embodiments, the coolant recovery bag 309 has an integrated 1.5 meter tubing set, which permits it to be placed at the bedside.

In several embodiments, a trocar assembly 400 can be provided. In several embodiments, the laser illuminating system can include the trocar assembly 400 along with the laser illuminator assembly 101. In several embodiments, the probe 113 of the laser catheter assembly 101 can be introduced into a tumor or an organ comprising tumor tissue using a trocar assembly 400 comprising a trocar 425 with a sleeve catheter 426 (e.g., a catheter sheath) sheathed around the trocar 425. As shown, in Figure 2B, in several embodiments, the introducer probe 113 can be supple and/or flexible (e.g., a 25 cm length of probe can be looped into a circle without kinking). In several embodiments, the tip can be comprised of a material that allows flexing which advantageously allows the tip to around obstructions at tissues. In several embodiments, as disclosed elsewhere herein, the flexible material of the probe advantageously can be resistant to adhesion to tissue and/or can be non-stick and/or can be substantially non-stick. In several embodiments, the introducer probe 113 has a length (e.g., from the proximal to distal end) of less than or equal to about: 300 mm, 240 mm, 200 mm, or ranges including and/or spanning the aforementioned values. In several embodiments, the introducer probe has a width of less than or equal to about: 2 mm, 1 mm, 0.5 mm or ranges including and/or spanning the aforementioned values.

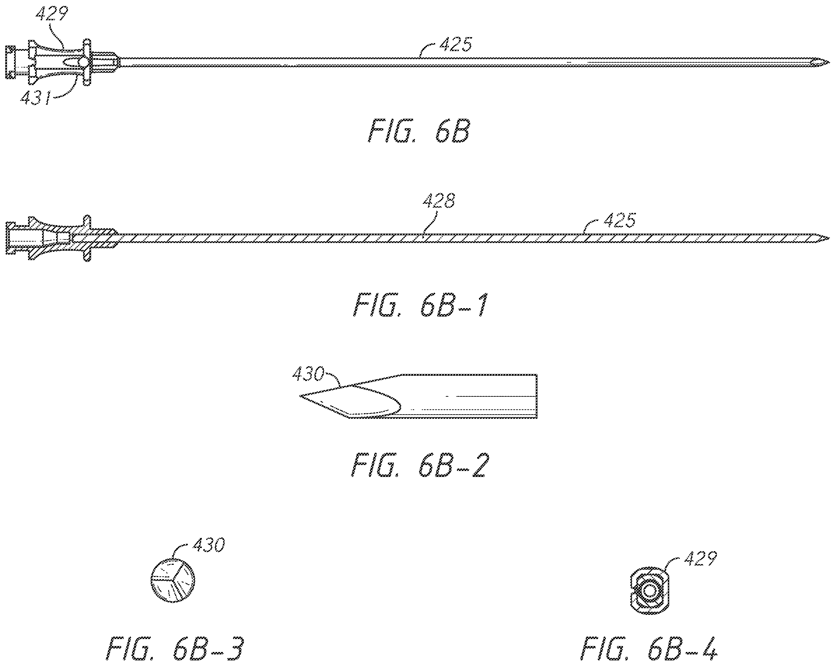

In several embodiments, to facilitate introduction of the introducer probe 113, the introducer probe 113 can be inserted into tissue using a trocar sleeve assembly 400 as shown in Figure 6A-6A-2. In several embodiments, as shown in Figure 6A, the trocar sleeve assembly 400 can include the trocar 425 and the sleeve catheter 426 (e.g., a probe sheath). In several embodiments, the trocar 425 can include a trocar lumen 428. Figure 6A-1 shows a bisected view of the trocar assembly 400 with the lumen 428 exposed. Figure 6A-4 shows an end on view of the trocar sleeve assembly 400. Figures 6B and 6B-1 show the trocar 425 without the sleeve, where Figure 6B-1 shows a bisected view. In several embodiments, the trocar 425 also can include a trocar handle 429, as shown. In several embodiments, the trocar handle 429 can be ergonomic. In several embodiments, the trocar handle 429 has one or more finger holds 431 that facilitate manipulation of the trocar 425 during placement. In several embodiments, a trocar handle 429 allows placement of the trocar through tissues of the patient and/or positioning of the trocar assembly 400 inside the patient at the site of treatment.

In several embodiments, the trocar 425 can include a three-sided cannula and/or needle 430. Figure 6A-3 shows an expanded view of the catheter tip 430 with the sheath 426. Figure 6B-2 shows an expanded view of the catheter tip 430 without the sheath 426. Figure 6B-3 shows a front view of the trocar tip 430. In several embodiments, the three-sided trocar tip 430 avoids tissue pull as the trocar is pushed through tissue of a patient. In several embodiments, beveled needles can pull to a side of the bevel as you pass through tissue. In other embodiments, a beveled needle can be used as a trocar (not pictured). Figure 6B-4 shows an end view of the trocar 429.

In several embodiments, after the trocar sleeve assembly 400 is inserted into a site of treatment, the trocar 425 can be removed from the patient's body. In several embodiments, in removing the trocar 425, the catheter sheath assembly 427 can be left inside the patient. In several embodiments, the catheter sheath 426 can then be used to position the laser catheter assembly 101 introducer probe 113 into the site of treatment. In several embodiments, as shown in Figure 6A-1, the probe sleeve 426 can include a connector 440. In several embodiments, the sleeve connector 440 (e.g., the connector of the catheter sheath) can include one or more connection structures, as disclosed elsewhere herein. In the instance shown in Figure 6A-1, the connection structure can include a ridge 440a that may thread the shroud 151 of a corresponding sheath connector 150. For example, as disclosed elsewhere herein, in several embodiments, as shown in Figure 2A, the sheath connector 150 can optionally include a shroud 151 (e.g., a hood, a threaded shroud, etc.) and the connector of a catheter sheath 440 can have a female connection with a female receiving portion 441. In several embodiments, the connection structure can include threads, clasps, arms, latches, protrusions, recesses, a taper, or combinations of any of the foregoing.

One or more advantages of the disclosed devices and components become apparent when performing a method utilizing a system as disclosed herein. One example of a method is illustrated in Figures 7A-7K. The method can include inserting a trocar assembly 400 comprising a trocar 426 and a catheter sheath assembly 427 sheathed around the trocar 426 into a patient at an insertion point (e.g., a first insertion point). Such an insertion is illustrated in the drawings of Figures 7A-7K using a test specimen 700 representing a target tissue (e.g., a tumor, etc.). In this case, the test specimen is a raw chicken breast. The test specimen is used as a model for a tumor or for an organ comprising a tumor.

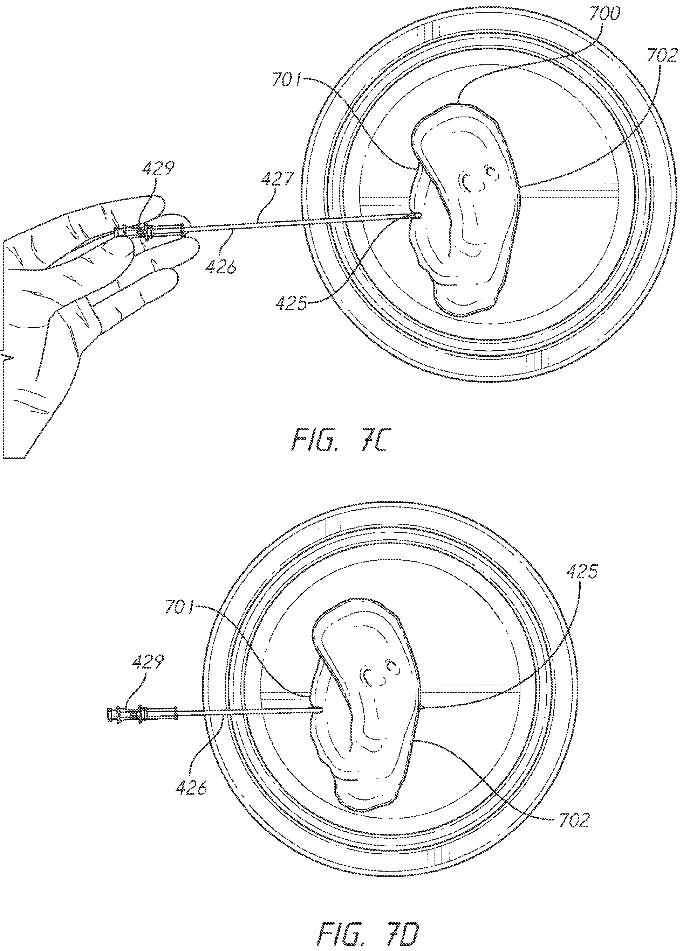

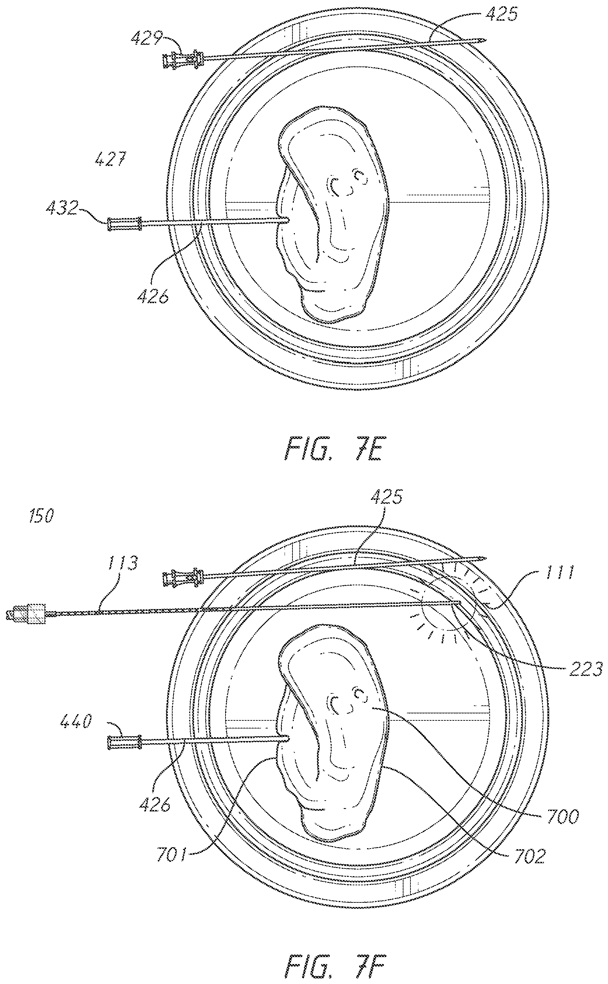

Figure 7A shows the test specimen and a trocar sleeve assembly 400 within a protective cover 433. In several embodiments, the trocar sleeve assembly can include a trocar Figure 7B shows the trocar sleeve assembly 400 removed from the protective cover 433. As shown in Figure 7C, in several embodiments, the trocar sleeve assembly 400 can be inserted into a proximal side 701 (e.g., a proximal portion, where proximal is in reference to the position of the user and/or the insertion point) of the target tissue (e.g., the test specimen 700). In several embodiments, as shown in Figure 7D, the trocar sleeve assembly 400 can be inserted through the body of the target tissue to a distal side 702 or substantially distal side of the target tissue 700. In several embodiments, the trocar sleeve assembly can be placed at a position intermediate of the proximal and distal side of the target tissue. In Figure 7D, the trocar 425 can be visible at the distal side 702 of the target tissue. As shown in Figure 7E, in several embodiments, once the trocar catheter assembly has reached the distal side of the target tissue 702, the trocar 425 can be removed leaving the catheter sheath 426 in place. Proper positioning of the trocar sleeve assembly 400 (or trocar, trocar tip, and/or catheter sheath 426) can be accomplished using MRI, ultrasound, or other real time imaging methods (e.g., Computer Tomography).

In several embodiments, as shown in Figure 7F, the laser catheter assembly 101 can be prepared for insertion into the catheter sheath. As shown, the introducer probe 113 of the laser catheter 101 has a radius of irradiation 111 present around the diffuser tip when the laser can be activated. In several embodiments, prior to insertion of the introducer probe 113 into the catheter sheath 426, the optical fiber 202 can be positioned inside the laser catheter assembly 101. In several embodiments, the optical fiber 202 can be fixed in the laser catheter assembly 101 via engagement of the optical fiber connector 222 with the end aperture 220. As shown in Figure 7L, the optical fiber 202 can be positioned within the probe 113. In some embodiments, the optical fiber and its diffusing tip 204 can transmit laser radiation through the domed end 115 of the introducer probe 113.

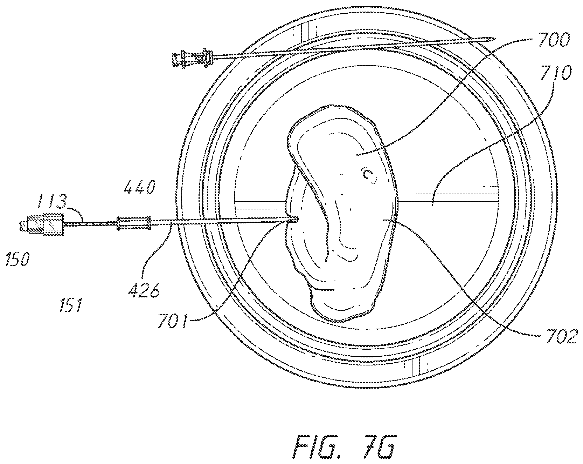

In several embodiments, as shown in Figure 7G, the introducer probe 113 of the laser catheter assembly 101 can be inserted into the catheter sheath 426. This guides the active tip of the catheter to the distal side 702 of the target tissue 700. In several embodiments, as shown in Figure 7E, the catheter sheath assembly 427 can include an insertion port 432 and sheath 426. In several embodiments, the insertion port 432 and sheath 426 facilitates insertion of instruments, such as the introducer probe 113. In several embodiments, the probe 113 of the laser illuminator can be inserted into the sheath 426 of the sheath assembly 427 via the insertion port 432. As shown in Figure 7G, the location of the probe 113 within the tissue can be determined using the catheter sheath's connector 440 and the graduations on the proximal portion of the probe 113. In several embodiments, the location of the catheter sheath's connector indicates to the user when the probe has reached the appropriate depth in the target tissue 700. In several embodiments, as an alternative of in addition to using the graduations of the probe 113, the initial positioning of the probe 113 within a target tissue may be determined by resistance felt by the user and due to tissue that has not been penetrated by the trocar.

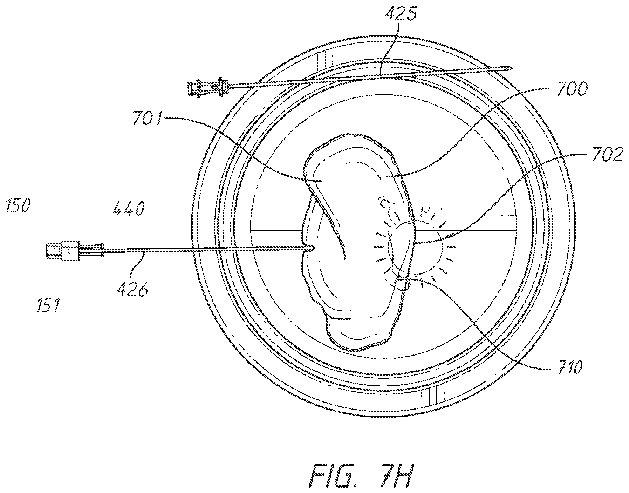

Once the appropriate and/or desired depth is reached, the catheter sheath assembly 727 can be slid back along the probe 113, as shown in Figure 7H. In several embodiments, after the introducer probe 113 reaches the proper and/or desired position in the target tissue (as shown in Figure 7G), the sheath assembly 427 can be withdrawn towards the proximal side of the target tissue 700 to expose the diffuser tip 223 within introducer probe 113 to begin irradiation of the target tissue (as shown in Figure 7H). As shown in Figure 7H, with the sheath assembly 427 being withdrawn, the sheath connector 150 of the laser illuminator assembly 101 can be used to engage the catheter sheath's connector 440 to fix the catheter sheath assembly 427 to the laser illuminator assembly 101. In several embodiments, connection structures of the connector of the catheter sheath 440 and connection structures of, for example, the shroud 151 of the sheath connector 150 can be cinched together to provide a connection between the catheter sheath 426 and the probe 113 of the laser illuminator assembly.

As shown in Figure 7G, where the catheter sheath 426 is not transparent (e.g., is not optically transparent or transparent to the radiation emitted from the tip), radiation may be partially blocked by the diffuser tip. As shown in Figure 7H, with the catheter sheath 426 withdrawn (e.g., pulled proximally) from the tissue to reveal the tip of the probe (within the tissue), radiation can be emitted from the tip into the tissue providing an irradiated region 710. In several embodiments, as shown in Figure 7H, the connector 440 of the catheter sheath 426 and the sheath connector 150 of the laser illuminator assembly 101 can be engaged (e.g., connected). In several embodiments, when the connectors 150, 440 can be engaged to one another, the probe is no longer masked by the catheter sheath. For instance, in several embodiments, the catheter sheath assembly can be a fraction of the length of the probe. In several embodiments, the catheter sheath assembly can have a length that can be equal to or less than 9/10, 7/8, 4/5, 3/4, 2/3, or 1/2 of the length of the probe (e.g., from the tip of the probe to the sheath connector).

In several embodiments, the catheter sheath 426 can be used to mask the probe 113 and/or to prevent irradiation of tissue from the probe. For example, in several embodiments, the laser may left on with coolant flow throughout the duration of a irradiation procedure. In several embodiments, the laser may be blocked during repositioning of the probe and/or after completion of an irradiation period. For example, the connectors 150, 440 may be disconnected and the graduations of the probe may be used to determine when the probe is masked and no longer substantially irradiating tissue. Alternatively, in other embodiments as disclosed elsewhere herein, the laser may be turned-off during repositioning and/or after completion of an irradiation period.

In several embodiments, as shown in Figure 7H, while the connector of the catheter sheath 440 and the sheath connector 150 proximal to each other and/or are engaged to each other, the tip of the probe can be exposed within the target tissue and the first region 710 may be irradiated. In several embodiments, the first region can be irradiated for a predetermined and/or defined period of time.

During a period of irradiation, with prior laser illuminator assemblies, a user was required to hold the laser illuminator assembly in place by hand. If the user let go of the laser illuminator assembly 101 during an irradiation time (such that the weight of the main body 102 of the laser illuminator assembly rested on the probe 113), there was a substantial risk that the delicate, flexible probe 113 of the laser illuminator assembly 101 would bend, kink, or break. If the probe 113 is kinked or otherwise damage, it may leak coolant (e.g., through a rupture in the probe) causing ineffective cooling of the optical fiber. Damage to the probe may lead to ineffective radiation distribution and/or ineffective treatment of an irradiated zone also (e.g., due to tissue char around the probe blocking the diffusion of radiation). Several embodiments disclosed herein address this issue or others. In several embodiments, because the probe can be configured to couple to a stabilizing device (e.g., the catheter sheath assembly 427), it can be protected from damage during a procedure. In several embodiments, using the disclosed configurations, the laser illuminator assembly 101 need not be held in place (e.g., can be let go of) during irradiation because the stabilizing device prevents and/or lowers the incidences of probe damage (from bending or breaking). Because irradiation may be tedious and/or may require long period of time, and because the laser illuminator assembly can be quite delicate in some embodiments, the use of a stabilizing feature can be especially advantageous in ensuring that the delicate probe does not kink, bend, or break when appropriately placed or in reducing a risk of the probe kinking, bending, or breaking when appropriately placed.

In several embodiments, the stabilizing device can be configured to surround a portion of the probe and to connect to the laser assembly via the sheath connector. In several embodiments, the stabilizing device provides support and/or added strength to the probe at the portion of the probe between the patient's body and the main body 102 of the laser illuminating system. In several embodiments, as disclosed elsewhere herein, the stabilizing device can be the catheter sheath assembly 427 and/or the catheter sheath. In several embodiments, the laser illuminator assembly can be configured to engage the catheter sheath 426 (e.g., via the connector 426 and the sheath connector 150). In several embodiments, when the sheath connector is engaged to the connector sheath via the connector of the catheter sheath, the probe can be stabilized. In several embodiments, when the sheath connector is engaged to the connector sheath via the connector of the catheter sheath, incidences of kinking of or damage to the probe can be avoided, minimized, and/or can be less frequent.

In several embodiments, using several configurations disclosed herein, instead of being forced to hold the probe by the main body 102 of the laser illuminator system 101 during a procedure, the catheter sheath assembly 427 can be used as a stabilizing device (e.g., when coupled to the sheath connector). In several embodiments, the user can be able to let go of the laser illuminator assembly 101 and/or to let the assembled probe 113 and catheter sheath assembly 427 support the full weight of the laser illuminator assembly 101.

In several embodiments, the sheath assembly adds rigidity and/or strength to the laser assembly 101 when engaged via the connectors 150, 440. In several embodiments, when the sheath connector 150 is engaged to the connector sheath 426 via the connector 450 of the catheter sheath assembly 427, the laser illuminator assembly 101 can be held by the probe in a position where the probe can be horizontal without kinking the probe.

While engaging the catheter sheath connector 440 and the sheath connector 150 together may have certain advantages, the catheter sheath connector 440 and the sheath connector 150 need not be engaged in any and all instances. In several embodiments, the proximity of the catheter sheath connector 440 and the sheath connector 150 may provide stabilization of the probe.

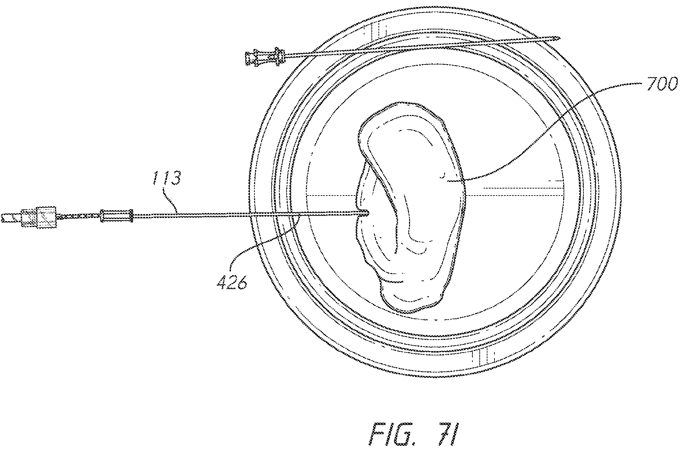

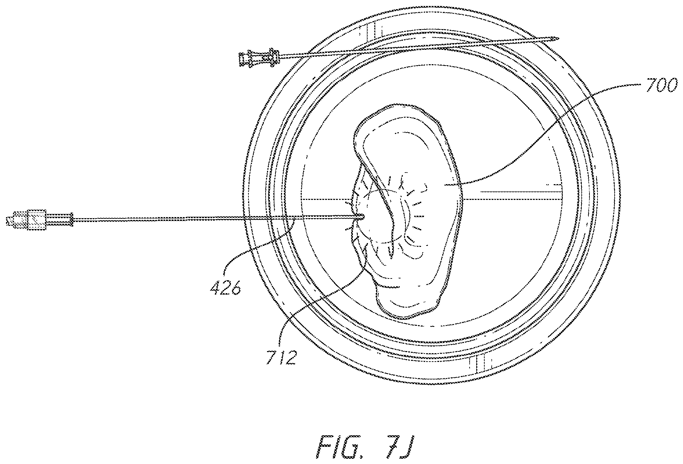

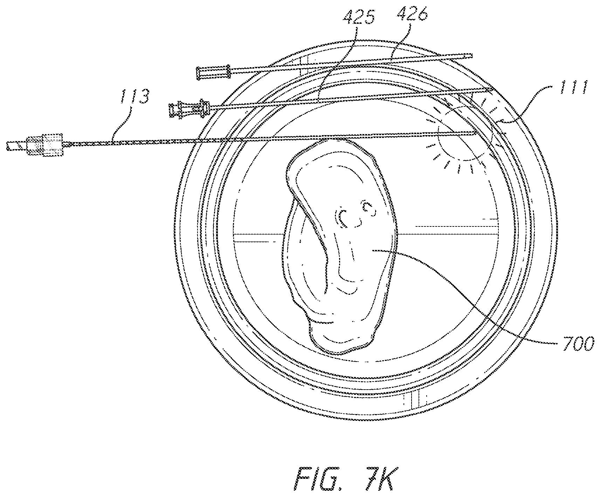

As shown in Figures 7H-7J, in several embodiments, after the irradiation of the first region is complete, the introducer probe 113 can be withdrawn slightly towards the proximal side 701 and away from the distal side 702 of the target tissue 700 exposing a second region (a second irradiated region 712) of the target tissue to treatment. In several embodiments, the withdraw/irradiate process can be repeated multiple times depending on the size of the target tissue. As shown in Figure 7I, after the probe is withdrawn, the catheter sheath assembly 427 can be withdrawn. In several embodiments, the connectors 150, 440 may be coupled and treatment performed at the second region 712, as shown in Figure 7J. Though not shown, in several embodiments, a third, fourth, fifth, and additional regions can be irradiated. In several embodiments, the hash marks 121 of the laser catheter assembly 101 allow the user to determine the proper position for the first, second, third, fourth, etc. regions for irradiation as the introducer probe 113 is withdrawn. As shown in Figure 7K, after the treatment is over, the probe 113 and sheath assembly 427 may be withdrawn completely.

In several embodiments, the introducer probe 113 and/or the introducer probe and the catheter sheath assembly 427 can be withdrawn from the target tissue incrementally at distances of equal to or less than: about 4 mm, about 8 mm, about 12 mm, values between the aforementioned values, or ranges spanning and/or including those values. In several embodiments, the distance between irradiated regions of the target tissue can be equal to or less than: about 4 mm apart, about 8 mm apart, about 12 mm apart, values between the aforementioned values, or ranges spanning and/or including those values. In several embodiments, the distance between irradiated regions can be determined using graduations on the introducer probe.

In several embodiments, though not shown, the distance between irradiated regions can be determined using graduations on the catheter sheath. For example, in several embodiments, the catheter sheath of the catheter sheath assembly may be graduated. In several embodiments, after initial placement of the introducer probe into the catheter sheath within a target tissue, the catheter sheath can be engaged to the probe via the connectors 150, 440. In several embodiments, the introducer probe 113 and/or the introducer probe and the catheter sheath assembly 427 can be withdrawn from the target tissue together incrementally at distances of equal to or less than: about 4 mm, about 8 mm, about 12 mm, values between the aforementioned values, or ranges spanning and/or including those values. In several embodiments, the distance between irradiated regions of the target tissue can be equal to or less than: about 4 mm apart, about 8 mm apart, about 12 mm apart, values between the aforementioned values, or ranges spanning and/or including those values.

In other embodiments, the catheter sheath can be transparent optically and/or to the radiation emitted by the laser source. In several embodiments, the graduations of the introducer probe can be visible through the catheter sheath 426 to allow depth of insertion into the patient to be easily visualized during a procedure.

In several embodiments, the trocar sleeve assembly 400 can be reinserted into another region of the target tissue (for example, a laterally disposed second insertion point) and the above irradiation and withdrawal process can be repeated. In several embodiments, the laser assembly can be repositioned into different areas of a tumor after irradiation.

As for the test sample shown in Figures 7A-7K, the method of treating a tumor or tumors in a target region (e.g., a tumor in an organ or gland) can include a step for positioning the trocar sleeve assembly 400 in a patient. In some embodiments, the trocar sleeve assembly can be positioned by passing the trocar assembly through, for example, a prostate tumor such that the trocar assembly passes through a proximal face of the tumor (toward the user who is inserting the trocar sleeve assembly) and terminates at a distal side of the tumor (away from the user who is inserting the trocar sleeve assembly). In some embodiments, the insertion of the trocar sleeve assembly from the proximal to the distal side of the tumor and creates a first path within the tumor. In some embodiments, as described with the test specimen of Figures 7A-7K, the method of treating a tumor can include a step for removing the trocar from the patient and leaving the catheter sheath assembly 427 (e.g., the catheter sheath 426) in the patient within the first path. In some embodiments, a laser catheter assembly 101 (e.g., a laser illuminator assembly) can be acquired. In some embodiments, as discussed elsewhere herein, the laser catheter assembly 101 can include one or more of an introducer probe 113 and an optical fiber 202. In some embodiments, as shown in Figure 7J and 7L, the introducer probe 113 (e.g., the laser introducer probe) can include a first lumen and terminates in a sealed domed end configured to allow laser light transmission. In some embodiments, as shown in Figure 7L, the introducer probe can include an internal tube located within the first lumen 116 of the introducer probe 113. In some embodiments, the internal tube 117 can include a second lumen 118. In some embodiments, as shown in Figure 7L, the optical fiber 202 can be positioned within the second lumen 118. In some embodiments, when positioned within the second lumen, the optical fiber and its diffusing tip 204 can transmit laser radiation through the domed end 115 of the introducer probe 113. In some embodiments, the first lumen can be in fluidic communication with the second lumen when the optical fiber can be positioned within the first lumen.

Preferably, the method includes a step for inserting the laser illuminator assembly into the catheter sheath 426 and guiding the laser illuminator (e.g., the laser catheter assembly 101) to a first position within the first path in the tumor. In several embodiments, the first position can be located near or at the distal side of the tumor. In several embodiments, the catheter sheath can be partially removed to expose the introducer probe. In several embodiments, the catheter sheath can be engaged to the introducer probe using the connectors 150, 440. Preferably, the method includes a step of activating the laser illuminator at the first position within the first path to generate non-ablative infrared radiation for a first period of time thereby heating the nanoparticles to an ablative temperature.

Preferably, the method includes a step for withdrawing the laser illuminator and the catheter sheath to a second position within the first path (and connecting them via the connectors 150, 440). In several embodiments, the second position within the first path can be closer to the proximal side of the tumor than the first position in the first path. In several embodiments, the laser illuminator can be activated at the second position within the first path to generate non-ablative infrared radiation for a second period of time. In several embodiments, the illumination of the laser illuminator heats the nanoparticles to an ablative temperature.

Preferably, the method can include withdrawing the catheter and the laser illuminator from the first path and inserting the trocar assembly into the patient at a second insertion point. In several embodiments, the second insertion point can be laterally disposed on the proximal side of the tumor from the first insertion point. In several embodiments, the trocar assembly can be positioned in the patient by passing the trocar assembly through the prostate tumor such that the trocar assembly passes through the proximal face of the tumor and terminates at the distal side of the tumor thereby creating a second path through the tumor. In several embodiments, the trocar can be removed from the trocar assembly. In several embodiments, the introducer probe of the laser illuminator assembly can be then positioned into the catheter sheath. In several embodiments, the introducer probe can be guided into place at a first position within the second path in the tumor. The catheter can be moved to expose the introducer probe. In several embodiments, the first position can be located near the distal side of the tumor. In several embodiments the first position in any pathway could, alternatively, be intermediate between the proximal and distal faces of the target tissue (e.g., tumor).

In several embodiments, the laser illuminator can be activated at the first position within the second path to generate non-ablative infrared radiation for a third period. In several embodiments, the activation of the laser illuminator at the first position of the second path heats the nanoparticles to an ablative temperature. In several embodiments, similar to the procedure used in the first path, the laser illuminator (and catheter sheath) can be withdrawn proximally to a second position within the second path. In several embodiments, the laser illuminator can be activated at the second position within the second path to generate non-ablative infrared radiation for a fourth period of time thereby heating the nanoparticles to an ablative temperature.

In several embodiments, the laser illuminator can be positioned at 1, 2, 3, 4, 5, 6, or more positions for illumination within each pathway. In several embodiments, the distance between irradiated regions of the target tissue within a pathway can be equal to or less than: about 4 mm apart, about 8 mm apart, about 12 mm apart, values between the aforementioned values, or ranges spanning and/or including those values. In several embodiments, the period of time that the laser is active (e.g., the irradiation time) at each position in a pathway can be equal to or at least about 1 minute, 3 minutes, 5 minutes, 10 minutes, values between the aforementioned values, or ranges spanning and/or including those values. In several embodiments, time needed to irradiate each position in full pathway can be equal to or at least about: 15 minutes, 30 minutes, 45 minutes, 60 minutes, 90 minutes, or ranges including and/or spanning the aforementioned values.

Advantageously, the irradiate and/or treat and withdraw method helps prevent seeding agents (e.g., cancer cells, etc.) along the path of the treatment. The withdrawing method can also help cauterize tissue to prevent bleeding as the instruments are withdrawn. In several embodiments, by withdrawing the laser catheter assembly from the distal to the proximal side of the target tissue, blood can be coagulated and/or any bleeding can be substantially or entirely sealed-off. In several embodiments, by withdrawing the laser catheter assembly from the distal to the proximal side of the target tissue, the spread of tumor cells by blood flow, or push through of cancer cells out of the target tissue, etc.

The insert and withdraw methods and/or other methods disclosed elsewhere herein can be performed on tumor tissues, on glands comprising cancer tissue, on organs comprising cancer tissue, and/or on structures (e.g., the throat) comprising cancer tissue. For instance, when treating the prostate gland, the introducer probe can be inserted through the prostate to a distal side and withdrawn as described elsewhere herein.

In several embodiments, because infrared light can penetrate approximately 4 mm laterally from the introducer probe, for tumors or treatment areas having lateral widths greater than 8 mm, multiple insertions of a laser catheter assembly laser introducer probe (or probes) can be used. When multiple insertion points are used, freehanded placement of laser introducers (or placement using, for example, a ruler to try and maintain spacing between laser dosing) can result in improper placement of the laser catheter assembly. The likelihood for improper placement can be high for intra-cavity treatments because it may only be possible to set the separation of the laser catheter assembly at the skin surface. In several embodiments, for tumors or treatment areas having lateral dimensions of greater than 8 mm, a device for positioning multiple laser catheter assemblies simultaneously (or repeated individual placements of a single laser catheter assembly) can be used to maintain a fixed separation between penetration points. In several embodiments, this device can be a type of template (e.g., a grid or jig).

In several embodiments, as described elsewhere herein, a grid or template can be used to determine the insertion points for the trocar sleeve assembly into the target tissue (e.g., tumor). In several embodiments, the template grid with laterally spaced apart holes can be used to guide the trocar sleeve assembly placement. In several embodiments, each insertion point within the tissue of the patient can be determined by the spacing of the holes in the template grid. In several embodiments, the lateral distance between insertion points determined by the template grid can be equal to or less than: about 4 mm apart, about 8 mm apart, about 12 mm apart, values between the aforementioned values, or ranges spanning and/or including those values. In several embodiments, the lateral distance between insertion points can be equal to or less than: about 4 mm apart, about 8 mm apart, about 12 mm apart, values between the aforementioned values, or ranges spanning and/or including those values. In several embodiments, multiple insertions (e.g., of multiple probes from multiple laser catheters) can be performed simultaneously using a grid. In several embodiments, the insertions can be performed serially using a single laser catheter and a grid.

In several embodiments, a grid template as shown in Figure 8A can be used to set and/or position laser catheter assemblies at selected separations and/or to maintain parallel alignment of multiple penetrations of laser catheter assembly introducer probes. In several embodiments, use of a grid helps prevent or lessen the amount of untreated margins of a tumor that can occur where the laser dose has been inadequate for particle activation, for example, because of improper probe placement. In several embodiments, the grid can include a plurality of apertures 801. In several embodiments, each grid aperture 801 can be configured to receive a sheathed trocar 425, a catheter sheath 426, and/or an introducer probe 113. In several embodiments, once the trocar 425 can be placed in the target tissue, it can be removed leaving the catheter sheath 426. The introducer probe 113 of the laser catheter assembly 101 can then be inserted into the sheath 426 and the method of irradiation as disclosed elsewhere herein can be performed. In several embodiments, as described elsewhere herein, a plurality (2, 3, 4, 5, 6, 7, 8, 9, 10, or more) laser catheter assemblies 101 and introducer probes 113 can be inserted into a plurality of sheaths 426 positioned using a grid. Then, the introducer probes 113 can be withdrawn together or serially during treatment (thereby shortening the time it takes to treat a target area). In several embodiments, a guidewire can be used. In several embodiments, a guidewire is optionally not used to position the laser catheter assembly.

In several embodiments, hexagonal grids 802 can be used as shown in Figure 8A. In several embodiments, hexagonal grids 802 can be used to provide equidistant spacing to nearest neighbor probes. A square configuration grid 850 is shown in Figure 8B. As shown, the grid apertures 851 of the square configuration grid 850 align to provide a square shape 852. As noted in Figures 8A and 8B, labeled markers 803, 804, 853, 854 can be used to indicate where a laser catheter should be placed within the grid 800, 850. As shown in Figures 8A and 8B, the labeled markers can include alphabetical indicators 803, 853 (e.g., A, B, C, D, E, F, G, H, I, J, K, L, M, N, O, P, Q, R, S, T, U, V, W, X, Y, Z, AA, etc.) or numeric indicators 804, 854 (e.g., 1, 2, 3, 4, 5, etc.) on different axes of the grid. Other indicators can be used, such as colors (e.g., red, orange, yellow, green, cyan, blue, indigo, violet, purple, magenta, pink, brown, white, gray, black), shapes (squares, circles, triangles, diamonds, pentagons, hexagons), or mixtures of color-coded shapes.

While square grids, as shown in Figure 8B, permit straightforward identification of grid positions, they suffer from certain drawbacks as shown in Figures 8C and 8D. Figure 8C depicts a hexagonal embodiment with a minimum aperture spacing of 7 mm. Figure 8D shows a square configuration with an aperture spacing of 3 mm. Firstly, for square grids having 3 mm spacings (as shown in Figure 8D), for example, the grid positions can be 3 mm apart in both axes. This means that translation along a diagonal represents a translation of 4.24 mm as shown in Figure 8D. Thus, in order to completely cover the lateral extent of a tumor for which the optical penetration radius can be 4 mm, a square grid requires "oversampling". That is, for a 3 mm square grid, alternate grid positions should be used along the principal axes, but along a diagonal there will be a potential untreated zone unless each diagonal grid position is used. This can lead to time-consuming treatment planning to calculate what grid positions must be used to completely cover the target area, and which positions can be bypassed. As shown in Figure 8D, a square pattern of 9 grid positions with a 3 mm spacing showing the non-overlapping region (white) if a lateral position is not filled.

In several embodiments, the adoption of a hexagonal grid arrangement, a portion of which is shown in Figure 8C, obviates these difficulties because each nearest neighbor position can be equidistant. In several embodiments, once the boundary of the treatment zone is defined, then all of the interior grid positions can be used, completely covering the target zone with minimal overlap. This leads to the dual advantage of decreasing the number (and/or minimizing) of introducer penetrations into the patient and decreasing the number (and/or minimizing) the treatment time. In several embodiments, a 7 mm spacing between grid apertures can be used to assure a maximum of 4 mm distance from any grid position. Figure 8C shows 3 grid positions with a 7 mm spacing showing the convergence of the 4 mm radius treatment zones. As seen in Figure 8C, in several embodiments, the maximum distance, at the center of each triad of holes can be set at 4 mm. As a consequence, using adjacent holes for laser placement assures continuous coverage of a region with minimal redundancy, permitting the most efficient use of treatment time. In several embodiments, the spacing between adjacent grid apertures can be less than or equal to about: 5mm, 4 mm, 3mm, 2 mm, values between the aforementioned values, or ranges spanning those values.

In several embodiments, the grid can be prepared using a monolithic construction (e.g., a one piece design). In several embodiments, the grid can be machined by computer numerical controlled milling from a single piece of plastic. In several embodiments, lettering and numbering (e.g., indicators) can be etched into the plastic and/or printed onto the surface. In several embodiments, a monolithic design can be distinct from the 3-piece template grids shown in Figure 8E. In several embodiments, the apertures size can be less than or equal to about: 20 gauge, 14 gauge, 12 gauge, values between the aforementioned values, or ranges spanning those values.

In several embodiments, each number and letter designation in a square grid covers two holes, both vertically and horizontally, leading to a two-fold ambiguity in both axes for a given callout. In several embodiments, a 7 mm spacing of the hexagonal grid (as shown in Figure 8A) permits unambiguous identification for every position as well as printed or inscribed horizontal lines on alternate rows of holes in order to facilitate accurate identification. Drawings of embodiments of a hexagonal grid and a square grid are shown in Figures 8F and 8G, respectively.

In several embodiments, during the treatment the laser illuminator can be activated by an actuator that can be controlled by a user. In several embodiments, when the user activates the laser illuminator using the actuator, coolant automatically flows into the first inlet of the laser illuminator assembly. In several embodiments, when the laser illuminator can be not active and not irradiating a tissue, coolant does not flow into the laser illuminator assembly. In several embodiments, the actuator can be a foot pedal.

In several embodiments, as discussed elsewhere herein, laser power can be absorbed by nanoparticles within a tissue. In several embodiments, upon absorption, the nanoparticle heat to sufficiently high temperatures to induce photothermal coagulation of tissue. In several embodiments, photothermal therapy using nanoparticles can be performed using a laser that can be of sufficiently low power so that it does not in and of itself induce coagulative hyperthermia (e.g., 3.5 - 4.5 W/cm of diffuser). In several embodiments, the power of the laser can be less than or equal to about: 2 W/cm, 3 W/cm, 4 W/cm, 5 W/cm, 6 W/cm, or ranges spanning and/or including those values. In several embodiments, coagulative hyperthermia occurs when the nanoparticles absorb the radiative energy. In several embodiments, non-coagulative hyperthermia can be induced at a temperature below about 45 °C, about 35 °C, about 30 °C, or ranges spanning and/or including those values. In several embodiments, coagulative temperatures include tissue temperatures equal to or greater than about 45 °C. In several embodiments, the laser can be activated for a period of about 3 to about 5 minutes without inducing temperatures that cause photothermal coagulation.

In several embodiments, the laser illuminator emits radiation having a near infrared wavelength. In several embodiments, the nanoparticles can be designed to have light absorption maxima in radiation in the near-infrared region (e.g. ranging from about 670 nm to about 1200 nm wavelengths) that allow the penetration of this energy through normal tissue. In several embodiments, upon the application of a laser emitting within these wavelengths, the nanoparticles absorb and convert this energy into heat to elevate the temperature of the tumor to an ablative level. In several embodiments, the effect of the nanoparticle-induced hyperthermia can be to create a temperature elevation confined to the tumor and the region immediately adjacent thereto, localizing the area of tissue ablation and reducing damage on surrounding healthy tissue. In several embodiments, the laser illuminator emits radiation having a near infrared wavelength ranging from about 805 nm to about 810 nm. In several embodiments, a wavelength of about 805 to about 810 nm allows lower absorption by tissue (and hemoglobin), while increasing the absorption of the nanoparticles. In several embodiments, a wavelength of about 1000 nm can be used.

In several embodiments, the laser illuminator can emit radiation that is of insufficient power to induce photothermal coagulation of tissue. In several embodiments, the optical fiber can include a diffuser tip that distributes the non-ablative infrared radiation within the tumor. In several embodiments, the laser illuminator emits radiation between about 3.5 W/cm and about 4.5 W/cm of the diffuser tip.

In other embodiments, the lateral distance between insertion points can be not determined using a template grid.

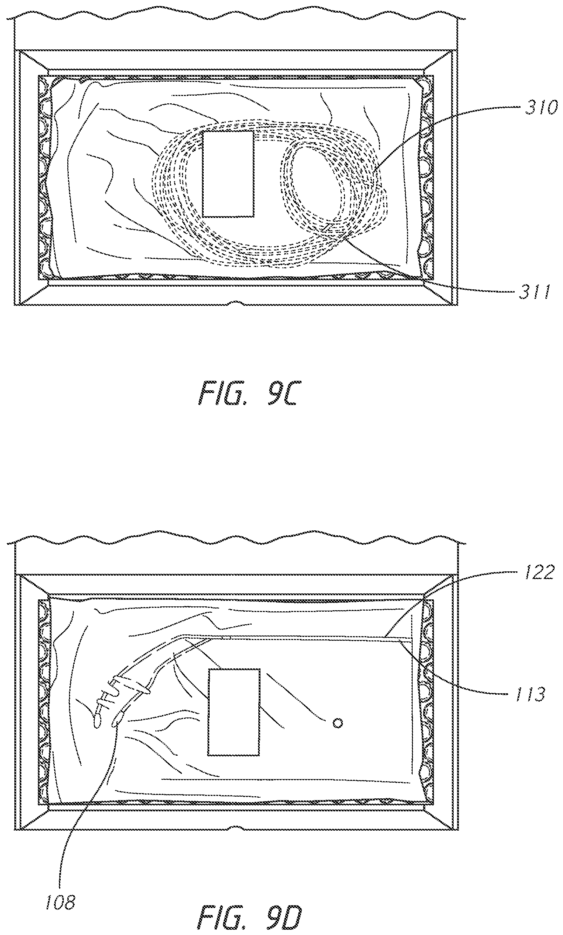

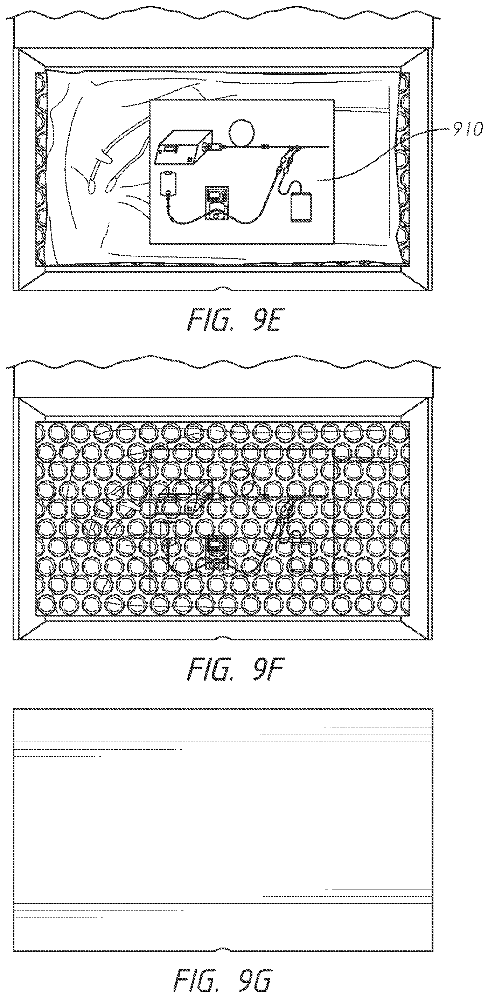

Several embodiments pertain to kits for use in laser therapy as described elsewhere herein. Figures 9A-9G show an embodiment of a kit, which can include one or more components of the laser illuminating system. In several embodiments, as shown in Figure 9A a container with packing material can be provided. In several embodiments, the kit can include an optical diffuser pack as shown in Figure 9B. In several embodiments, the optical diffuser pack can include an optical fiber 202 with an optical fiber tip and an optical fiber connector as described elsewhere herein. In several embodiments, the optical diffuser pack can include a optical fiber sheath that protects the fiber and/or the diffuser tip. In several embodiments, the kit can include a coolant supply set as shown in Figure 9C. In several embodiments, the coolant supply set can include one or more of a coolant reservoir, a coolant inlet conduit, an inlet conduit connector, a coolant recovery bag, a coolant outlet conduit, and/or a coolant outlet connector. In several embodiments, the kit can include a laser catheter assembly packet. In several embodiments, the laser catheter assembly kit can include one or more of a laser catheter assembly 101. In several embodiments, the introducer probe 113 of the laser catheter assembly can be protected using a protective sheath 122 as shown in Figure 9D. In several embodiments, the further can include instructions 910 (e.g., on the assembly or use of the laser catheter assembly) shown in Figure 9E. In several embodiments, the instructions can include drawings such as those shown in Figure 1. The method for using the laser catheter system as disclosed elsewhere herein can be provided as part of the instructions. In several embodiments, as shown in Figure 9F, an additional layer of packing material can be used to cover the kit. In several embodiments, as shown in Figure 9G, the packing container can be closed and sealed to provide a kit that can be ready to ship. While not shown, in several embodiments, the kit can further include the coolant pump, the trocar and devices related there to, the grid assembly, etc. Additionally, one or more of the components shown in Figures 9A-9G can be excluded from the kit.

Several embodiments have been described in connection with the accompanying drawings. Some of the figures are drawn to scale, but such scale should not be limiting, since dimensions and proportions other than what are shown are contemplated and are within the scope of the disclosed invention. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, it will be recognized that any methods described herein may be practiced using any device suitable for performing the recited steps.

### EXAMPLES

### Example 1: Laser Catheter Testing

It has been observed that certain laser introducer probe tips of cooled catheter laser systems melt during clinical procedures and result in the formation of char on the tips with the consequent loss of light penetration into tissue. This char also results in non-specific coagulation and thermal fixation. The heating of the embodiments having a domed laser catheter tip was compared side-by-side to a Visualase^{®} Cooled Catheter System (CCS) (having a ground conical tip). It was determined that the conical tip of the CCS created a significant focus of heat, greatly exceeding that of the domed laser catheter tip. The experiments described in this example show that the heating of the CCS is concentrated in the conical tip and that from one-third to over one-half of this heating is the result of end losses from the laser diffusing fiber (LDF). This conclusion was reached by using both the Visualase Laser Diffusing Fiber and embodiments having a domed laser catheter tip in the same conical Visualase Cooled Catheter System under the same conditions. When the embodiments having a domed laser catheter tip was used, the evolved heat was 34 - 57% less than when the Visualase LDF was used in the same Visualase conical-tipped catheter. Without being bound to a particular theory, the excess light leakage out of the end of the Visualase LDF and the conical catheter tip itself both contribute to the development of a device-destroying hot spot. The remainder of tip heating results from the shape and material of the catheter itself. In contrast to the 22 - 57° C temperature rise using a CCS, the temperature rise in a domed laser catheter tip under similar experimental conditions was less than 2°C.

### Test Organization

The Visualase CCS and a laser catheter assembly comprising a domed laser catheter tip were configured similarly to each other, with an isotropic diffuser tipped optical fiber secured within a transparent, dual lumen, liquid-cooled 16G catheter. Tests were performed in open air representing a "worst case scenario" in that there is no thermal coupling and/or heat transfer to tissue, which would tend to aid in the dissipation of heat away from the tip. The CCS was assembled as part of a standard Visualase Cooled Laser Applicator System (VCLAS). The tip of the 11 meter LDF was advanced to within 2mm of the terminus of the CCS tip. The coolant inlet and outlet were supplied with a VCLAS tubing set (not including the extension set) supplied from a 1L flask of room temperature water. Coolant was supplied by a Visualase K-Pump.

The experimental layout for the experiments performed is shown pictorially in Figures 10A and 10B. The coolant pump 307 and laser 203 were placed on a cart (at left of Figure 10A) and were connected to the CCS/LDF assembly 901 via a coolant tubing set. A thermal camera 900, the small object mounted to the adjustable arm at the center of both Figures 10A and 10B, was operated from a laptop computer. A power source 902 is also shown. The CCS/LDF assembly can be suspended on the ring stand shown in Figure 10B. The laser power meter is at the right in the Figure 10A.

### Tests

1. Heating of the CCS/LDF assembly 4.5, 6.0, and 12.0W laser power, measured in air, with nominal coolant flow.
2. Heating of the CCS assembly with an 18 mm AuroLase Optical Fiber Diffuser substituted for the Visualase LDF.

### Test conditions

Air: non-physiological condition that permits ready measurement by a thermal camera, and which represents a "worst case scenario" for thermal conductivity;
Orientation: in all cases the CCS assembly 901 was mounted vertically with the tip downward;
Laser output: 4.5±0.1W for 10 mm diffuser, 6.0±0.1W for 18mm diffuser, and 12.0±0.1W, 2X standard laser power (and near the maximum available laser output);
Nominal coolant flow: 7.9±0.2mL/min water at room temperature (20-21°C).

### CCS Experimental Setup

Laser: Diomed 15+ #10000126
Coolant pump: Visualase K-pump, property #10000125
Coolant supply: standard AuroLase Therapy CSS
Optical power optometer: Gigahertz-Optik P9710-2, NBI property #10000242/243
Thermal camera 900: ICI model 3720.
Data recording and image control software: Lenovo Ultrabook Yoga 2 pro running custom ICA IRFlash application

### Test articles

The Cooled Catheter Assembly with Laser Diffusing Fiber advanced to 2mm from catheter tip;
Cooled Catheter Assembly with 18 mm Optical Fiber Diffuser advanced to 2 mm from catheter tip.

### Heating of the CCS/LDF assembly

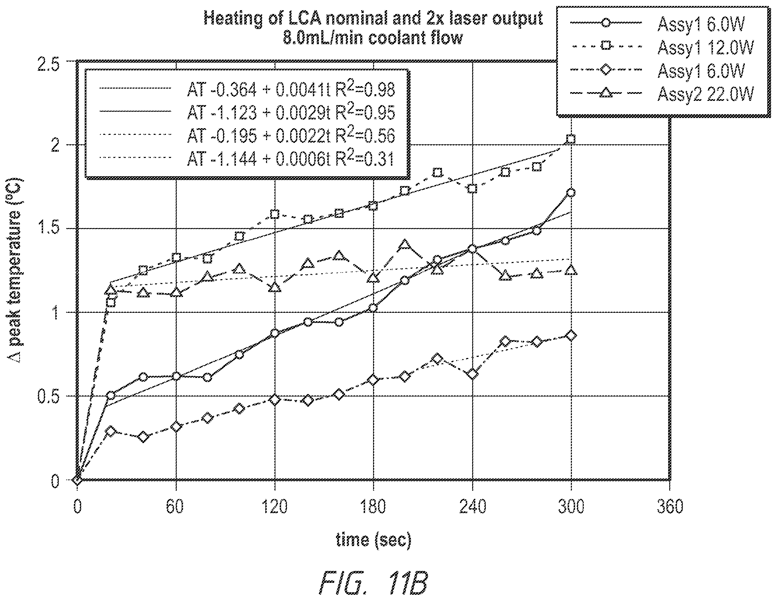

Figures 11A and 11B show the data for a Visualase^{®} Cooled Catheter System (CCS) (having a ground conical tip; Figure 11A) and for a domed laser catheter tip as disclosed elsewhere herein (Figure 11B). Figure 11A shows the increase in peak temperature of the distal end of the CCS assembly over 3 minutes of 4.5, 6.0, and 12.0W of laser output. The coolant flow was un-interrupted and set at 7.9 mL/min sourced from a room temperature (20°C) 1 liter reservoir. The temperatures followed a characteristic first order transient response. At 4.5W the temperature rose 22°, at 6.0W the rise was 28°C, and at 12.0W the rise was 57°C. Settling at the final temperature was rapid, with a time constant of 1.9 - 2.9 seconds, effectively coming to equilibrium within 15 seconds in all cases.

Figure 11B shows the heating of the laser catheter assembly comprising a domed laser catheter tip under heating conditions coinciding to those of previous irradiation of the Visualase system in Figure 11A. By comparison, for the laser catheter assembly comprising a domed laser catheter tip at 12.0W irradiation, the temperature rose 1.2 - 1.6°C above ambient temperature during the course of a standard 3-minute treatment. Figure 11B shows heating of the laser catheter assembly comprising a domed laser catheter tip under nominal (6.0W/18 mm diffuser) and 2X laser output for both Assembly #1 (circles) and Assembly #2 (squares). Assemblies #1 and #2 are two identical optical fiber/laser catheter assemblies that were tested under identical conditions. The assemblies were cooled at 8.0 mL/min with room temperature water. Figures 11A and 11B are scaled differently in order to clearly show the temperature evolution in detail. For instance, Figure 11B shows an expanded view of the temperature.

Figures 12A and 12B show false-color thermal images of the distal end 950 of the CCS/LDF assembly and the end of the 6.0W irradiation. Figures 12C and 12D show false-color thermal images of the distal end of an embodiment of a domed catheter at the end of the 6.0W irradiation. Heat can be concentrated at the very tip of the CCS, which can be 52.4°C, or 28° above ambient. Figure 12B shows a close-up of the CCS/LDF tip under 6.0W irradiation clearly showing heat buildup at the extreme end of the conical tip. From Figure 12A, it is immediately apparent and clear that the heating can be concentrated within the conical tip. This can be further demonstrated by the close-up of the CCS tip in Figure 12B where the hottest portion is seen to be the extreme tip of the CCS. From Figures 12C and 12D it is apparent that heat does not build in the tip of the domed cooled catheter.

### 2. Heating of the CCS/OFD assembly

The evolution of heat by the different laser catheters has two sources: the optical fiber diffuser itself and the material and conformation of the enclosing catheter. In order to distinguish between these two contributions an optical fiber diffuser of the laser catheter system described elsewhere herein was substituted into the CCS and the laser irradiations were repeated.

Figure 13 shows the increase in temperature of the CCS using an embodiment of a laser diffuser disclosed herein (having an 18 mm diffuser tip) instead of the Visualase Laser Diffusing Fiber. Figure 13 records the increase in peak temperature of the distal end of the CCS/OFD assembly over 3 minutes of 4.5, 6.0, and 12.0W of laser output. The coolant flow was un-interrupted and set at 7.9 mL/min sourced from a room temperature (20°C) 1 liter reservoir. The temperatures also followed a characteristic first order transient response. At 4.5W the temperature rose 9.4°, 12.3°C at 6.0W, and 38°C at 12.0W. Settling at the final temperature was less rapid than previously, with a time constant of 2.2 - 4.8 seconds, effectively coming to equilibrium within 25 seconds. Overall, the change from the LDF to the OFD reduced the evolved heat by 57% for the 4.5W output, 56% for the 6.0W output, and 34% for the 12.0W output. Since the heating of the CCS can be overwhelmingly in the conical tip it was concluded that: 1) the 34 - 57% reduction in heating afforded by switching to an optical diffuser disclosed herein can be the result of greatly reduced end loss from the diffuser tip, and 2) the appreciable heat can be the result of light being focused within the conical tip.

### Conclusion

The observed tendency for the conical tips of Cooled Catheter Systems to melt during clinical procedures results in the formation of char on the tips with the consequent loss of light penetration into tissue and in non-specific coagulation and thermal fixation. The experiments described herein demonstrate that this heating of the CCS is in fact concentrated in the conical tip, from 1/3 to over 1/2 of this heating is the result of end losses from the Laser Diffusing Fiber, and the remainder from the shape and material of the catheter itself. By contrast, with the 22 - 57° temperature rise in the CCS, the temperature rise using embodiments of laser catheter assemblies as disclosed herein (e.g., an embodiment as disclosed herein having a domed tip) under near-identical conditions was less than 2°C.

### Example 2: Treatment of a Prostate Tumor

This is a single-arm study with a single group of patients. An objective of the study is to determine the efficacy of using MRI/US fusion imaging technology to direct focal ablation of prostate tissue using nanoparticle-directed laser irradiation.

The patient population consists of men with low to intermediate risk localized prostate cancer with MRI visible and confirmed focal areas of prostate cancer using MRI/US Fusion Guided Biopsy. The patient also has no disease detected via ultrasound guided biopsy outside of areas visualized on MR imaging.

There is one arm/group to this study: Up to forty five (45) patients receive a single intravenous infusion of AuroShell particles 12 to 36 hours prior to MRI/US guided laser irradiation using an FDA cleared laser and an interstitial optical fiber.

Efficacy and acute volume of ablation can be assessed by contrast-enhanced MRI 48 - 96 hours after laser illumination to allow time for the appearance of coagulative necrosis and prior to reconfiguration of tissue by lytic action. An appearance of a 'void' on MRI is more generally expected than lesion shrinkage.

Efficacy of focal ablation of prostate tissue is assessed by MRI/Ultrasound guided biopsy at 90 days (primary endpoint) and again at 1 year after laser treatment. Per standard of care patient follow-up is on a 6 month basis beyond the one year follow up but is outside the scope of the initial study.

Nanoparticle Dose: in this study AuroShells are used. These are gold nanoshells. AuroShell Dose: Each patient receives an infusion of up to 7.5 mL/kg of AuroShell particles concentrated to 100 Optical Density (approximately 2.77x10¹¹ particles/mL or 36 mg particles/kg of patient weight). AuroShell particles are administered intravenously through a standard non-DEHP infusion set, and are infused at rates ranging from 120 mL/hour to the nominal 600 mL/hour at the investigator's discretion.

Laser Dose: Laser illumination takes place under ultrasound guidance 12 to 36 hours after particle infusion. An isotropic fiber in a water-cooled jacket with an isotropic diffusing tip can be inserted via transperineal approach. The urethra can be cooled by circulating saline (or water) through a urethral catheter. Up to 5.0 Watts per cm of optical fiber diffuser length of measured output of 810±10 nm laser power can be delivered for a period of up to 4 minutes for each treatment site. If necessary, the laser fiber can be repositioned and a separate zone illuminated.

### Inclusion Criteria

Patients have documented histological or cytological evidence of tumor(s) of the prostate. Patients are ≥ 45 years of age. Patients or their legal representative are able to read, understand and sign an informed consent. Organ confined clinical T1C or clinical T2a prostate cancer that is visualized on MR imaging. Prostate cancer is diagnosed by MR image guided biopsies. Gleason Score ≤ 7; and 2 or less positive lesions on prior MR US fusion guided prostate biopsy. If the standard biopsy cores are positive, they should be from the same location in the prostate as MR lesion was biopsied and proven to be cancerous. (Left / Right, Base, Mid Gland, Apex). Prior MRI results dated within 120 days prior to ablation. No metastatic disease as per NCCN guidelines (www.nccn.org) - Bone scan indicated to r/o metastatic disease if clinical T1 and PSA > 20 or T2 and PSA > 10 PSA < 15 ng/ml or PSA density < 0.15 ng/m12 in patients with a PSA > 15 ng/ml. The patient has given written informed consent after the nature of the study and alternative treatment options have been explained.

### Exclusion Criteria

Patients with known hypersensitivity to any of the components of a PEGylated gold nanoshell suspension (polyethylene glycol, gold). Patients who are receiving concurrent investigational therapy or who have received investigational therapy within a period of 5 half-lives of the investigational therapy in question prior to the day of dosing with the PEGylated nanoshell particles.

### Introduction

Prostate cancer is the most commonly diagnosed cancer in men. In 2014, approximately 233,000 men in the United States were diagnosed with prostate cancer and an estimated 29,000 died from the disease. The average annual incidence of prostate cancer among African American men was 60% higher than among non-Hispanic white men, and the average annual death rate was more than twice that of non-Hispanic white men.

Existing treatment modalities for primary prostate cancer include "active surveillance", surgical resection, and radiation (including brachytherapy). These modalities have a significant level of side effects, including erectile dysfunction, urinary incontinence, and rectal damage. Focal therapeutic approaches that reduce the adverse effects associated with treatment, eradicate the current disease, or potentially increase the time to progression are needed.

The optical properties of tissues inherently limit the depth of propagation of optical energy, thereby enabling a truly focal therapy. Heat evolved from an energy source is inherently diffuse and non-specific. Although thermally ablative heat can be made to propagate on the millimeter scale, it is not specific to tumor conformation. Nanoshell particles offer the opportunity for more precisely localized conformal therapy and result in tumor specific damage on the millimeter scale.

The MRI fusion imaging approach, using ultrasound guidance based on a priori MRI fusion imaging, should permit the precise placement of the optical fiber catheter within or adjacent to the prostate lesion(s) targeted for ablation.

A merging of MRI fusion imaging and the subsequent directed placement of the laser catheter represents a means of establishing the efficacy and utility of nanoshell therapy for the focal therapy treatment of prostate tissue. The MRI-Ultrasound fusion approach resolves limitations identified using an ultrasound only approach; given the ability of MR US fusion technology to allow:
1) pre-treatment target planning, and 2) guided imagery to be used as an additional level of safety after the initial use of ultrasound for planning placement of the catheters using a brachytherapy stepper which allows for the accurate placement of the laser catheter in proximity to the lesion to be ablated.

The methods can also be used in squamous cell carcinomas of the head and neck, canine melanomas and carcinomas of the oral cavity, and in canine and human prostate all serve to demonstrate the inherently focal nature of particle-directed photothermal energy. Incorporation of an imaging modality can enable precise lesion treatment. Further, the ability of particles to co-locate to neoplastic tissue can further enable lesion ablation conformal to the target tumors.

The potential benefit of nanoshell therapy can be highly selective and rapid tumor destruction with minimal damage to surrounding tissue enabling a potentially curative treatment of tumors with minimum toxicity. Preclinical studies have demonstrated that nanoshell therapy is effective and causes no detectable systemic toxicity.

### Investigational Devices and Systems

Nanoshells and the laser illuminating system disclosed herein selectively destroy solid tumors using near infrared illumination from a laser. Unlike other energy-based ablation methods, which rely upon the endogenous absorption and transduction of the deposited energy into heat, nanoshell particles, delivered systemically to the tumor, serve as an exogenous absorber of this laser illumination to generate a lethal thermal response specific to tumor tissue.

Nanoshell therapy can be comprised of three components: (i) a near infrared laser source, (ii) an interstitial fiber optic probe for delivery of the laser energy to a site near and/or inside the tumor, and (iii) nanoshell particles, a near-infrared absorbing inert material designed to absorb and convert the laser energy into heat. Nanoshell particles are delivered systemically, and allowed to accumulate selectively at the tumor site and then illuminated by a near- infrared laser. The particles absorb and convert this illumination into heat, resulting in the thermal destruction of the tumor and the blood vessels supplying it with minimal damage to surrounding healthy tissue. Since the accumulation of the particles within the peri-vascular space of tumors can be passive and can depend only upon the fenestrated neo-vasculature characteristic of solid tumors, nanoshell therapy may be used in patients previously treated with chemotherapy and radiation. During the procedure, needle thermocouples placed around the tumor may be used to monitor margins of treatment. In several embodiments, this will provide monitoring of temperatures around the ablation zone and permit the operator to minimize the risk of overheating critical structures or areas outside of the intended target tissue. Low temperature control points (about 45 °C to about 50°C threshold) are used near critical structures, such as the urethra, urinary sphincter and rectal wall, in order to avoid damage to these tissues.

Nanoshell therapy can be useful in recurrent and refractory head and neck cancer and metastatic lung tumors.

There is one arm/group to this study. Up to forty five (45) patients receive a single intravenous infusion of nanoshell particles 12 to 36 hours prior to ultrasound-guided laser irradiation using an FDA cleared laser and interstitial laser fiber. Acute efficacy and volume of ablation is assessed by contrast-enhanced MRI 48 - 96 hours following the laser illumination in order to allow time for the appearance of coagulative necrosis but, prior to reconfiguration of tissue by lytic action. An appearance of a 'void' on MRI would be more generally expected than lesion shrinkage.

Nanoshell/laser catheter therapy is a system for the photothermal ablation of solid tumors using near-infrared energy. The system utilizes an interstitial fiber optic probe to deliver near-infrared energy emitted by an FDA-cleared laser to a site proximate to and/or inside a solid tumor. Unlike other energy-based ablation methods, which rely upon the endogenous absorption and transduction of the deposited energy into heat, Nanoshell/laser catheter therapy particles, delivered systemically to the tumor, serve as an exogenous absorber of this laser illumination to generate a lethal thermal response specific to tumor tissue.

Nanoshell/laser catheter therapy can be comprised of three components: (i) a near infrared laser source, (ii) an interstitial fiber optic probe for delivery of the laser energy to a site near and/or inside the tumor, and (iii) nanoshell particles, a near-infrared absorbing inert investigational material designed to absorb and convert the laser energy into heat.

Nanoshell/laser catheter therapy may be used with an FDA-cleared clinical laser that emits near infrared energy with the desired parameters (energy, duty cycle, cycle time) and with an interstitial fiber optic probe for percutaneous energy delivery. The nanoshell particles used in this study consist of a gold metallic shell and a non-conducting, or dielectric, core that serves as the exogenous absorber of the near infrared laser energy delivered by the fiber.

Nanoshell/laser catheter therapy in this study uses an FDA-cleared laser, either the Diomed 15-PLUS (K013499) or LiteCure, LLC (K093087) as an infrared energy source and an interstitial, liquid-cooled optical fiber source.

The steps involved in Nanoshell/laser catheter therapy can include one or more of: (i) the intravenous infusion of a dose of nanoshell particles, (ii) a time delay of 12 to 36 hours to allow the accumulation of the nanoshell particles within the tumor by the enhanced permeability and retention (EPR) effect, and (iii) the illumination of the area with continuous wave (CW) or pulsed coherent light at a desired wavelength, for example, between 800 and 815 nm, for up to 4 minutes at up to 5.0 Watts average delivered output (assuming a 1-cm long isotropic diffusing delivery). The nanoshell particles in the area absorb light, and the metal shell converts the absorbed light to heat, generating heat sufficient to thermally ablate the tumor. The applicator can be positioned externally or inserted interstitially or endoscopically and can use either a collimated or dispersing fiber tip.

Nanoshell particles used in this study consist of a metallic shell and a non-conducting, or dielectric core. These particles can be designed and constructed to absorb or scatter light at desired wavelengths. This "tunability" can be achieved by altering the ratio of the thickness of the metal shell to the non-conducting core. The exterior shell of the particle can be comprised of gold, which has a long history of use *in vivo* in particulate form, as an implanted material, or as a coating on implanted devices. The non-conducting core can be silica, but can be comprised of any dielectric material. For cancer therapy, nanoshell particles are designed to absorb infrared light at wavelengths where light is not significantly absorbed by human tissue. Human tissue is minimally absorptive in the ranges from 750 nm to 1100 nm, often referred to as the "water window" or "tissue optical window". While solid gold nanoparticles and microparticles absorb light at wavelengths also absorbed by tissue, gold-coated nanoshell particles can be designed to absorb or scatter light within this "tissue optical window", enabling new *in vivo* applications.

More specifically, the nanoshell particles used in this study are comprised of a thin gold shell, 10 to 20 nm thick, deposited on a solid silica (silicon dioxide) core. To prevent aggregation of the particles in a saline environment and to provide steric hindrance *in vivo,* a 5,000 molecular weight (MW) methoxy-polyethylene glycol (PEG) chain can be attached through a thiol (sulphur) bond. The PEG coating improves the stability of the nanoshell particles in an isotonic aqueous solution, and may also improve circulating half-life on administration. For the studies reported here, the particles are concentrated to a desired level (generally less than 0.1% by volume) and suspended in an isotonic solution for injection (10% trehalose in water).

### Mechanisms of Action

Lasers and radiofrequency ablation devices are used to thermally destroy tissue by delivery of energy at a rate in excess of the tissue's ability to dissipate the energy through blood perfusion thermal diffusion. In addition, some lasers provide energy at wavelengths naturally absorbed by chromaphores within tissue or blood, using the properties of tissue or blood as a natural absorber to convert the light to thermal energy. The result is either thermal coagulation of cells or tissue thermal fixation and the disruption of the vasculature.

Nanoshells particles are infused intravenously and are known to accumulate in tumor stroma as a result of the fenestrated vasculature associated with tumors. This method of accumulation has been established by other particles and is termed the EPR effect. SEM analysis of tumor tissue following nanoshell particle intravenous injections indicates particle accumulation can be preferentially near the tumor vasculature.

Nanoshell particles are designed to absorb near-infrared energy, transducing it into heat via surface plasmon resonance. The near-infrared dose level utilized by Nanoshell/laser catheter therapy can be below that which would cause significant damage to tumor or normal tissue without the presence of nanoshell particles. In the presence of nanoshell particles in tissue, the near-infrared dose will generate a thermal response that may be sufficient to result in photothermal coagulation, leading to cell death. It is likely that this to include the disruption or occlusion of tumor vasculature in a manner similar to the FDA-cleared embolism microspheres.

### Preparation of Nanoshells

The nanoshell particles used in this study are packaged in units of 80 mL in a sterile IV bag and maintained at 2 to 8°C. For infusion, the package should be removed from refrigeration and allowed to warm to room temperature over 30 minutes. The bag should be gently kneaded or shaken to ensure the uniform dispersion of the material. The tubing for infusion should be C-Flex, non-DEHP, medical grade tubing. A 1.2 micron filter (Pall TNA1 or comparable) should be installed between the infusion tubing and the patient catheter.

### Particle Dose and Administration

The nanoshell particles are infused at a rate of 600 mL/hour (10 mL/minute). During the three days prior to and first 6 hours after administration, vasoconstrictive medications are avoided to maximize particle accumulation in tumor. Each patient receives an intravenous infusion of up to 7.5 ml/Kg of nanoshell particles concentrated to 100 Optical Density (approximately 2.77x10¹¹ particles/mL or 36 mg particles/kg of patient weight.

### Laser Application

Laser illumination will take place under MRI/US fusion guidance 12 to 36 hours after particle infusion. The urethra may be cooled by circulating saline (or water) through a urethral catheter (typ. 6-10mL/min). An optical fiber with an isotropic diffusing tip in an enclosed catheter can be inserted interstitially using a transperineal approach employing 14G needle/cannula introducers. Up to 5.0 Watts of measured output of 810±10 nm laser power can be delivered for a period of up to 4 minutes for each treatment site. If necessary, the laser fiber may be repositioned and a separate zone illuminated.

The specific procedures are described in the Instruction Manual for AuroLase Therapy (IFU), but a summary of the procedure is as follows:
Prior to use, calibrate the laser and verify laser output through the laser fiber to up to 5.0 Watts/cm average output. Prior to use, verify that the cooling system for the laser fiber is functioning properly. Using ultrasound guidance, insert needle/cannula introducers (e.g., trocar and catheter sheath) into the prostate proximal to the index lesion to be treated, avoiding the urethra and other critical structures. The MRI generated fusion images are not used for guidance of the laser placement, but serve only as a check on the placement via ultrasound guidance. Withdraw the needle from the introducer and replace it with the laser catheter. Withdraw the cannula (and connect it to the probe of the laser catheter) to expose the emitting portion of the laser catheter. If indicated by the proximity of the target lesion to a sensitive or critical structure (e.g., urethra, prostate capsule, or nerve bundle) a needle thermocouple may be inserted at clinician discretion alongside the laser catheter in order to monitor temperatures near the treatment zone. Position the fiber to illuminate a zone within the prostate for up to 4 minutes. If indicated by the size of the lesion to be treated, withdraw the fiber a distance of 2 mm less than the illumination length and illuminate the new zone. Repeat as required to illuminate the entire lesion. If additional target lesions are to be treated, repeat steps at the new site. If a single target lesion is treated, the investigator will make a single laser treatment in the contralateral prostate hemisphere to serve as a negative control of the laser treatment (i.e., no thermal damage to normal tissue in the absence of the accumulation of particles in the perivasculature of tumor tissue). In the event of the treatment of two lesions with one in each hemisphere of the prostate, this negative control procedure will not be performed. At the discretion of the investigator, if a Foley catheter was used to cool the urethra during the laser procedure it may remain in order to prevent occlusion of the urethra as a consequence of thermally-induced edema. The Foley catheter may remain in place until the follow-up MRI on Day 4/5.

Complete physical examination (general appearance, skin, neck, ears, eyes, nose, throat, lungs, heart, abdomen, back, rectal, lymph nodes, extremities, neurological status) can be performed at baseline and the 90 day follow-up.

A follow up MRI fusion biopsy can be taken at the 90 day follow-up visit to assess the efficacy of the tumor ablation with a view to ascertain the presence of viable cancer cells within the treatment zone and therefore the efficacy of focal ablation of prostate tissue. The patient has the standard of care reassessment biopsy at Year 1 and six months thereafter per physician treatment considerations. This includes MRI/US fusion biopsy and a standard 12 core biopsy.

### Results

The patients have an 70-80 percent reduction of tumor size 6 months after therapy. In 25% of the patients, the prostate cancer can be completely eradicated. In 50% of the patients, no side effects are noted and normal prostate function can be maintained.

### Example 3 Treatment of a Head and Neck Tumors

### A Pilot Study of AuroLase Therapy in Patients with Refractory and/or Recurrent Tumors of the Head and Neck.

This can be an open-label, multicenter, single-dose pilot study of AuroLase Therapy in the treatment of patients with refractory and/or recurrent tumors of the head and neck. Three (3) treatment groups of five (5) patients each are enrolled and observed for six (6) months following treatment. Each group receives a single dose of AuroShell particles. This dose may be increased by up to 67% for the second and third group if no significant unanticipated adverse device effects are observed in the first group. The laser illumination for each group consists of one or more interstitial illuminations (based on tumor size) with an escalating laser dosimetry for the second and third groups.

The first group of five patients are treated with the lowest treatment level of 4.5 ml/Kg of AuroShell particles concentrated to 100 Optical Density (approximately 1.3 x 10^12 particles/Kg or 20 mg particles/Kg). An isotropic fiber in a water-cooled jacket with a one cm diffusing tip can be inserted interstitially within and/or proximate to the tumor and up to 3.5 Watts measured average output of 800-810 nm laser power can be delivered at a 75% duty cycle, 1.25 pulses per second for a period of up to four (4) minutes. The fiber may be repositioned at distances of 1 cm from the original placement to provide illumination to other sides of the tumor, or different tumors, with appropriate precautions. For tumors greater than 1.0 cm in estimated thickness at the point of fiber placement, the fiber may be retracted within the catheter to provide illumination of the tumor mass.

Following treatment of the first three (3) patients, safety data (including data from the fourth week visit after treatment) for each patient is submitted per FDA's request. In the absence of unanticipated adverse device effects, enrollment and treatment is continued for the two additional patients being treated at the lowest concentration of 4.5 ml/Kg (totaling (5) patients in the first group of the study). The AuroShell dose is increased from 4.5 ml/Kg to 7.5 ml/Kg of AuroShell particles concentrated to 100 Optical Density (approximately 2.1 x 10^12 particles/Kg or 34 mg particles/Kg) for the second and third group if no unanticipated adverse device effects related to AuroShell dose are observed in the first group.

For the second treatment group of five (5) patients, laser illumination may be increased to 4.5 Watts measured average output of 800-810 nm laser power delivered at a 75% duty cycle, 1.25 pulses per second for a period of up to 4 minutes if no unanticipated adverse device effects are observed in the first group.

For the third treatment group of five (5) patients, laser illumination may be increased to 5.0 Watts measured average output of 800-810 nm laser power delivered at a 100% duty cycle (continuous wave) for a period of up to 4 minutes if no unanticipated adverse device effects attributable to AuroLase Therapy are observed in the second group.

In each treatment group, the AuroShell particles can be administered intravenously at a rate of 2 ml/minute for the first 15 minutes, and then increased to up to 10 ml/minute. At approximately 0.5, 2, 4, 8, 24 and 48 hours following administration of the AuroShell particles, a 2 ml blood sample can be obtained and the concentration of the particles determined by dynamic light scattering analysis (DLS) and neutron activation analysis (NAA).

Approximately 12 to 36 hours after intravenous infusion of the particles, the target tumor(s) can be illuminated by laser as described. Subsequent to the AuroLase treatment of the target lesions, biopsies can be performed and the gold accumulation measured by neutron activation analysis.

In addition to the observations through the first day after laser treatment, patient follow-up occurs on weeks 1 and 2, and monthly thereafter for the 6 months following treatment.

### Introduction

In the United States, approximately 46,000 cases of head and neck cancer are diagnosed on an annual basis, resulting in approximately 11,000 deaths annually. Unfortunately, despite multidisciplinary treatment efforts including surgery, radiation therapy, and chemotherapy, all of which are associated with substantial morbidity to patients, five year survival rates have not improved significantly over the last several years.

Nanoshell therapy can be used to selectively destroy solid tumors using near infrared illumination from a laser. Particles delivered systemically to the tumor serve as an exogenous absorber of this laser illumination to generate a thermal response.

### Nanoshells

The nanoshells (e.g., Auroshells) can comprise a silica core approximately 110 to 125 nm in diameter (total diameter of particle including the gold shell can be 140 to 170 nm); a functionalizing molecule to allow gold colloid to adhere to the silica, APTES (3-aminopropyltriethoxysilane); a thin layer of gold (10-20 nm thick) affixed to the functionalized surface of the silica core. The process of affixing the gold shell to the silica core includes the production of small colloidal gold nanoparticles 2 to 6 nm in diameter and the attachment of these nanoparticles to the amine groups present in the APTES. These colloidal nanoparticles act as nucleating sites for the reduction of gold salt (HAuC14) in the presence of formaldehyde, to complete the shell layer. The gold shell can include approximately 95% of the particle mass. The attachment of a layer of 5,000 MW PEG-thiol to the surface.

### Dose and Administration

In order to increase the EPR effect and AuroLase efficacy, at one hour prior to administration, the body temperature of the patient should be elevated by blankets or heating pads. A heating pad should be applied to the tumor area. If the tumor is in the oral cavity or proximate to the mouth or esophagus, cold beverages should be avoided.

AuroShell particles are administered intravenously at a rate of 2 ml per minute for the first 15 minutes of the infusion, increasing the rate to up to 10 ml per minute for the remainder of the infusion. Blood samples are taken at 0.5, 2, 4, 8, 24 and 48 hours for determination of blood clearance of AuroShell particles.

During the first 6 hours after administration, vaso-constrictive medications should be avoided to maximize efficacy. The first group of five (5) patients receives an intravenous infusion of 4.5 ml/Kg of AuroShell particles concentrated to 100 Optical Density (approximately 1.3 x 10^12 particles/Kg or 20 mg particles/Kg). The AuroShell dose can be increased from 4.5 ml/Kg to 7.5 ml/Kg of AuroShell particles concentrated to 100 Optical Density (or approximately 2.1 x 10^12 particles/Kg or 34 mg particles/Kg) for the second and third group if no significant unanticipated adverse device effects related to AuroShell dose are observed in the first group.

### Laser Application

Calibration and verification of proper operation of the AuroLase system can be carried out prior to initiating the laser procedure and are summarized below. Based on MRI, CT or similar imaging technique, the tumor region can be mapped to a grid by the surgeon to allow a systematic laser exposure of areas up to 1.0 cm outside the estimated tumor margin. This technique can be based on an estimated 1 cm³ tissue treatment volume per laser application. At a point 12 to 36 hours after AuroShell infusion the patient can be prepared for therapy. The patient may be sedated or anesthetized as determined by the physician. The laser and the cooling pump can be plugged in and connected together with the interlock cable. The optical fiber can be connected to the laser and threaded into the catheter. A sterile saline bag (1 liter) can be hung and connected to the Laser Fiber cooling tubing set. The Laser Fiber cooling tubing can be routed through the peristaltic cooling pump and connected to the inlet port of the catheter. The drain tube can be connected to the outlet port of the catheter and to a fluid collection bag. The cooling pump can be turned on and allowed to prime the cooling lines. The laser settings should be adjusted to deliver the desired average power. The power output of the fiber can be checked by inserting the catheter into a sterile tube mounted in the integrating sphere optometer. The LASER POWER knob can be adjusted to produce an average output power of as specified. The interstitial catheter can be placed proximal (within about 1 cm) to the tumor to be treated using the 14 gauge introducing catheter sheath to avoid tumor seeding. The laser inserted through the catheter sheath after which the catheter sheath can be engaged to the introducer probe. Laser can be applied for the specified time, then the fiber repositioned (using a fresh introducer if changing grid coordinates) to illuminate additional areas of the tumor as previously mapped and the laser re-applied. Immediately after the laser illumination of the patient's target lesion, a biopsy that will provide at least 6 mg of tumor tissue (such as an 18 gauge Tru-Cut needle biopsy 1cm in length or similar technique) can be performed to measure the gold accumulation by neutron activation analysis. If more than one tumor is being treated in a single patient, biopsies need only be obtained for the odd-numbered lesions (1st, 3rd, 5th, etc.).

### Endpoints

For each patient, up to five (5) index lesions are identified. Index lesions are those that are accessible to direct examination (examination by fiberoptic nasopharyngoscopy or laryngoscopy is permitted) and to biopsy. Each index lesion should have at least one dimension with longest diameter ≥ 15 mm using conventional techniques or ≥10 mm with spiral CT scan, and also be able to provide at least 6 mg of tumor tissue by biopsy (such as an 18 gauge Tru-Cut needle biopsy 1cm in length or similar technique) for assessment by neutron activation analysis. Tumor measurements are assessed from physical measurements and imaging techniques as appropriate for the lesion(s), e.g. CT, MRI, X-rays. Baseline measurements should be performed as close to AuroLase treatment as possible. All measurable lesions up to a maximum of five lesions are identified as target lesions and recorded and measured at baseline. Target lesions are selected on the basis of their size (lesions with the longest diameter) and their suitability for accurate repeated measurements (either by imaging techniques or clinically). A sum of the longest diameter (LD) for all target lesions will be calculated and reported as the baseline sum LD. The baseline sum LD will be used as reference by which to characterize the objective tumor.

All other lesions (or sites of disease) are identified as non-target lesions and should also be recorded at baseline. Measurements of these lesions are optionally not required, but the presence or absence of each should be noted throughout follow-up.

### Results

The patients have an 60-80 percent reduction of tumor size 6 months after therapy. In 15% of the patients, the cancer can be completely eradicated. In 40% of the patients, no side effects are noted and normal prostate function can be maintained.

### Example 4:

The following study describes results achieved using nanoparticles (Auroshells) with an embodiment of a laser catheter assembly as described elsewhere herein.

A completed Pilot Study of AuroLase Therapy (e.g., the use of nanoparticles and irradiation with a laser catheter assembly as described elsewhere herein) in 11 human subjects with head and neck cancer under a cleared FDA IDE revealed no safety-related issues in either particle delivery or the laser procedure.

Similarly, a pilot study of AuroLase Therapy in 22 human subjects with biopsy-diagnosed prostate cancer resulted in no Serious Adverse Events in either particle delivery or the laser procedure. In total, as of the current study, 33 human subjects have undergone AuroShell^{®} particle infusion and 26 have additionally undergone the related laser therapy with no reported Serious Adverse Events.

Preclinical safety has been established for AuroShell^{®} particles in vitro and in vivo in animal testing. Proof of concept studies have been carried out using particles systemically directed against inoculated tumors in the brain, directly injected into canine prostate and in human prostate tumors grown orthotopically in mice.

With the basics of animal and human safety established, the focus of the current study was to determine the efficacy of treatment with nanoshells and subablative infrared irradiation of those nanoshells using a laser catheter as disclosed herein. The treatment was performed on patients suffering from prostate disease in a 22 patient pilot study. All 22 patients were infused with nanoshells at 3 different dosing levels. Fifteen of these patients subsequently had the laser procedure as well. In a different study a further 13 patients were infused with nanoshells and subsequently underwent an ultrasound-guided focal laser ablation of tumor tissue.

While this study was conceived and organized as a safety trial, a number of whole-mount prostate sections of varying quality were available for histopathological analysis. From these analyses it was possible to draw additional conclusions about treatment efficacy.

The prostate study was carried out under the auspices of the Mexican health safety commission, COFEPRIS, as an open-label, multi-center, single-dose pilot study of AuroLase Therapy in the treatment of subjects with primary resectable prostate cancer. Only subjects that were scheduled for a radical prostatectomy were enrolled. The trial was divided into two arms: 1) Group 1 whose patients were infused with AuroShell particles one (1) day prior to a scheduled radical prostatectomy, and 2) Group 2 whose patients were given an infusion of AuroShell Particles one (1) day prior to a laser treatment, and 5±1 days prior to a scheduled radical prostatectomy. Patients were followed for 6 months following particle infusion with regular checks of vital signs, hematology, blood chemistry, and urinalysis. In total, seven (7) subjects completed the study in Group 1 and fifteen (15) subjects completed the study in Group 2.

### Prostate Results

Between January 2011 and July 2012 we enrolled 22 prostate cancer patients in México. Each of these patients was infused with clinical AuroShell particles (nanoshells as disclosed elsewhere herein), and 15 of these underwent AuroLase Therapy laser treatment. Apart from the safety results, two general conclusions were made based on the results: 1) over a narrow range of power settings, laser energy goes from producing no observable thermal damage to producing thermal lesions up to 1 cm across, and 2) Nuclear Activation Analysis (NAA) indicates there is enhanced AuroShell particle accumulation in prostatic adenocarcinoma as opposed to normal prostatic tissue.

### Adverse Events

Only two Adverse Events were attributed to the infusion element of AuroLase Therapy: a patient who suffered an apparent allergic reaction at the time of infusion, which resolved with intravenous medication, and a patient who suffered a single episode of a burning sensation of the epigastrium, for which no treatment was given, and which resolved spontaneously. No Adverse Events were attributed to the laser treatment.

### Infusion and Laser Dose

All patients were infused with the same 7.5 mL/kg (100 OD) dose of particles. This scales the particle dose by weight, and hence by blood volume. Fifteen (15) subjects in Group 2 received a laser treatment on the day following infusion. A trans-rectal ultrasound probe was used to direct a single placement of the optical fiber system within the cortex of each hemisphere of the prostate. Adjustment of the position of the optical fiber catheter within each hemisphere was based on the location of the highest Gleason score indicated by the needle biopsy. The goal of the laser treatment was to demonstrate the safety of the procedure by developing a laser dose that was ablative of tumor tissue, but not of healthy gland. Though this was a safety study, insights into efficacy by varying the laser dose beginning at 3.0W - 3.5W delivered for 3 min at each site based on the optimal laser dose was established. In total, 6 patients were treated at 3.5W or below. Over the course of the fifteen laser-treated patients this dose was increased incrementally to 5.0W (3 patients) delivered for 3min, and then reduced to 4.5W delivered for either 3 or 4min at each treatment site (6 patients).

### Limitations of the Mexico Study and Interpretation of Results

Although quite satisfactory as an evaluation of safety, the Mexico prostate study was limited in its ability to determine treatment efficacy in the following ways:
1) The standard 12-punch biopsy used to diagnose and stage patients for the trial did not provide accurate mapping of the number, extent, or specific location of patient tumor(s),
2) The small, portable ultrasound system available, while sensitive enough to insert the laser catheter into the general area of a particular prostate hemisphere, did not provide the millimeter scale resolution for reliably placing the laser catheter within or adjacent to a tumor, which was in any event invisible to the ultrasound system,
3) The logistics of fixing, cutting, embedding, transporting, assessing, and reporting on a given case rarely provided information timely enough to adjust treatment parameters prior to a subsequent case, and
4) The private clinic where the research was performed was able to provide whole mount slides for discrete, but incomplete sections of the laser-treated prostates.

Figures 14A-14I show representative whole-mount sections from the last 9 of the 15 laser-treated patients (those treated at >3.5W; scale bars = 1cm). These H&E sections (Hematoxylin and Eosin) show the spatial relationship between the tumor tissue present 1001 and the optical fiber placement 1002 in the prostate 1000, and any resulting zones of coagulo-necrosis 1003. For patients 208 and 210-215 at least one laser fiber placement was within or adjacent to regions of tumor. It is generally the case that treatments at the 4.5 W level produced coagulo-necrosis conformal to tumor boundaries, but generally not in normal gland, while treatments at the 5.0W level tended to produce thermal lesions generally. It is further noted that in no case was the prostate capsule breached by the coagulo-necrotic zones.

Figure 14A shows 4.4W irradiation for 3 min and no damage in normal gland. Figure 14B shows 4.4W irradiation for 3 min with no damage in normal gland. Figure 14C shows 5.0W irradiation for 3min with non-specific damage in normal gland. Figure 14D shows 5.0W irradiation for 3min with no damage to normal gland and non-specific damage at periphery of carcinoma. Figure 14E shows 4.5W irradiation for 4 min and a damage zone conformal to tumor boundary (1003) with no damage at a secondary site (1002). Figure 14F shows 4.5 W irradiation for 4 min with no damage in normal gland and no damage at periphery of carcinoma. Figure 14G shows 4.5W irradiation for 3 min with a thermal lesion that overlaps carcinoma and normal tissue, a thermal lesion in the normal gland enhanced by hemorrhage (upper left region, 1003). Figure 14H shows 4.5 W irradiation for 3 min with thermal damage in normal hemorrhagic gland. Figure 14I shows 5 W irradiation for 3 minutes with thermal damage in the normal hemorrhagic gland (arrow to region, 1003).

Figure 15 shows the relative accumulation of AuroShell particles in representative samples taken from patients 210 and 211 (see Figures 14D and 14E) as determined by NAA. Although the selected samples vary considerably as does the tumor grade as determined from the Gleason score, there is clearly a distinctly greater accumulation of particles in tumor tissue.

### Concept for Demonstrating Treatment Efficacy

The optical properties of tissues limit the depth of propagation of optical energy, thereby enabling a truly focal therapy. Heat evolved from an energy source is inherently diffuse and nonspecific. Although thermally ablative heat can be made to propagate on the millimeter scale, it is not specific to tumor conformation. AuroShell particles offer the opportunity for more precisely localized conformal therapy and result in tumor specific damage on the millimeter scale. The MRI fusion imaging approach, using ultrasound guidance based on a priori MRI fusion imaging, should permit the precise placement of the optical fiber catheter within or adjacent to the index prostate lesion targeted for ablation.

### Details for Demonstrating Treatment Efficacy

Particle-directed treatment can be made very focal. Over a narrow range of laser power settings, 4.0 - 5.0 W/cm, photothermal treatments can be made to spare normal glandular tissue and thermally ablate particle-containing adenocarcinoma and BPH.

As a result of the enhanced optical absorption resulting from the presence of particles, AuroLase Therapy can be able to generate photothermal lesions by elevating target tissues to 55 - 65°C over the course of a 3 - 4 minute treatment time. These temperature profiles generate coagulo-necrotic regions, which re-granulate and dissolve over the course of days, as opposed to thermally fixed lesions that tend not to resolve, while additionally appearing live on most histopathological examinations. Results of photothermal ablation are fully realized within 48 - 72 hours post treatment and are observable under MRI.

Since the accumulation of particles can be a function of neovasculature and not cell surface biomarkers of vessel and cell walls, AuroLase Therapy can be performed soon after (or prior to) chemotherapy and radiation therapy. There is, as yet, no evidence that AuroLase Therapy efficacy can be affected by previous therapies.

### Summary

Questions of safety have been answered both in terms of particle safety and laser delivery.

A merging of MRI fusion imaging and the subsequent directed placement of the laser catheter represents a means of establishing the efficacy and utility of AuroLase Therapy for the treatment of localized prostate disease, and represents the logical next step in the utilization of AuroLase Therapy. The MRI-Ultrasound fusion approach can be useful for resolving limitations identified in previous studies of AuroLase Therapy given the ability to do: 1) pre-treatment target planning, and 2) guided imagery for the accurate placement of the laser catheter in proximity to the index lesion to be ablated.

Three-dimensional localization of the optical fiber catheter within the prostate, coupled with near real-time thermal data (ideally MRTI) would permit the appropriate treatment power to be confirmed and the treatment time to be either lengthened or shortened in order to produce focal ablation that conforms to the index lesion to be treated.

Testing in squamous cell carcinomas of the head and neck, canine melanomas and carcinomas of the oral cavity, and in canine and human prostate all demonstrate the focal nature of particle-directed photothermal energy. Incorporation of an imaging modality can enable precise lesion treatment. Further, the ability of particles to co-locate to neoplastic tissue can further enable lesion ablation conformal to the target tumors.

The potential benefits of AuroLase Therapy include a highly selective and rapid tumor destruction with minimal damage to surrounding tissue enabling a potentially curative treatment of tumors with minimum toxicity. Preclinical studies have demonstrated that AuroLase therapy can be effective and can cause no detectable systemic toxicity.

In summary, various embodiments and examples of systems and devices for treating tumors have been disclosed. Although systems and devices have been disclosed in the context of those embodiments and examples, it will be understood by those skilled in the art that this disclosure extends beyond the specifically disclosed embodiments to other alternative embodiments and/or other uses of the embodiments, as well as to certain modifications thereof. This disclosure also expressly contemplates that various features and aspects of the disclosed embodiments can be combined with, or substituted for, one another. Accordingly, the scope of this disclosure should not be limited by the particular disclosed embodiments described above, but should be determined only by a fair reading of the claims that follow or that may be presented in the future.

## Claims

1. A system comprising:
a catheter sheath; and
a laser illuminator assembly (101), the laser illuminator assembly comprising:
a main body (102) comprising:
a first fluid chamber (103a);
a first hub (109) in fluidic communication with the first fluid chamber (103a), the first hub (109) comprising:
a first fluid port (125); and
a fitting (126) configured to engage a first coolant line (127);
a second fluid chamber (103b) comprising a waveguide aperture (120);
a second hub (106) in fluidic communication with the second fluid chamber (103b), the second hub (106) comprising:
a second fluid port (130); and
a fitting (131) configured to engage a second coolant line (132);
a waveguide hub (120a) providing access to the second fluid chamber (103b) of the main body, the waveguide hub (120a) being configured to receive a waveguide (202) and to direct it through the second fluid chamber (103b) and the waveguide aperture (120) of the second fluid chamber (103b);
a probe (113) extending distally from the main body (102), the probe comprising:
an elongate probe (113) member comprising a flexible external wall that defines a tubular cavity within the elongate probe (113), the external wall defining a first lumen (116) that is in fluidic communication with first fluid chamber (103a), the flexible external wall extending from a portion proximal to the main body (102) to a distal portion comprising a sealed tip (115); and
an internal tubular member (117) extending distally from the main body (102) and within the tubular cavity, the internal tubular member (117) defining a second lumen (118) and comprising an exit aperture disposed within the distal portion of the elongate probe member (113), wherein the internal tubular member (117) is configured to receive the waveguide (202) when the waveguide is directed through the waveguide aperture (120);
wherein:
a sheath connector (150) of the laser illuminator assembly (101) is configured to engage a corresponding connector of the catheter sheath (440) such that, when the sheath connector (150) of the laser illuminator assembly (101) is engaged with the connector of the catheter sheath (440), the catheter sheath (440) is configured to at least partially cover the probe (113).

2. The system of claim 1, wherein the probe (113) is made from a material that is transmissive to visible wavelengths of light, infrared light, ultraviolet light, or combinations of the foregoing.

3. The system of any one of claims 1 to 2, wherein the sealed tip (115) is domed and is transmissive to visible wavelengths of light, infrared light, ultraviolet light, or combinations of the foregoing.

4. The system of any one of claims 1 to 3, wherein the first fluid port (125) is a coolant outlet configured to distribute coolant into the first coolant line (127) and the second fluid port (130) is a coolant inlet configure to receive coolant from the second coolant line (132) and wherein the laser illuminator assembly (101) is configured to allow the passage of a coolant from the coolant inlet (130) through the second lumen (118) into the first lumen (116) and out of the coolant outlet (125).

5. The system of claim 4, wherein the coolant inlet (130) and the coolant outlet (125) are configured to interact with different connectors to prevent improper routing of coolant through the laser illuminator assembly (101).

6. The system of any one of claims 1 to 5, wherein the sheath connector (150) is threaded and configured to engage corresponding threads of the connector of the catheter sheath (440).

7. The system of any one of claims 1 to 6, wherein the sheath connector comprises a luer fitting configured to couple with a corresponding luer fitting of the connector of the catheter sheath.

8. The system of any one of claims 1-7, wherein the catheter sheath is graduated along at least a portion of its length and wherein the graduations are provided on the catheter sheath (440) at a position proximal to the connector of the catheter sheath (440).

9. The system of any one of claims 1-7, further comprising the waveguide (202), wherein the waveguide (202) comprises a fitting configured to engage a corresponding fitting of the waveguide hub (120a).

10. The system of claim 9, wherein the waveguide (202) is an optical fiber.

11. The system of claim 10, wherein the optical fiber (202) comprises a diffusive portion (223) configured to distribute radiation from the optical fiber and out of the laser illuminator assembly (101), wherein the diffusive portion (223) is not covered by the catheter sheath (427) when the catheter sheath is connected to the laser illuminator assembly (101) via the sheath connector (150) and the connector of the catheter sheath (440)..

12. The system of any one of claims 8-11, further comprising a coolant reservoir (305) configured to engage the second coolant line (132) and to be in fluidic communication with the laser illuminator assembly (101) when engaged to the second coolant line (132), wherein the coolant reservoir is configured to supply coolant to the laser illuminator assembly (101).

13. The system of any one of claims 8-12, further comprising a pump (307) configured to convey coolant from the coolant reservoir (305) to the laser illuminator assembly (101) via the second line (132) to cool the waveguide (202) when in operation.

14. The system of any one of claims 8-13, further comprising a coolant recovery bag (309) configured to engage the first coolant line (127) and to be in fluidic communication with the laser illuminator assembly (101) when engaged to the first coolant line (127), wherein the coolant recovery bag (309) is configured to receive coolant from the laser illuminator assembly (101).

15. The system of any one of claims 8-14, further comprising a laser source (203) configured to be in optical communication with the waveguide (202).

## Patentansprüche

1. System, das Folgendes umfasst:
eine Katheterhülse; und
eine Laserilluminatorvorrichtung (101), wobei die Laserilluminatorvorrichtung das Folgende umfasst:
einen Hauptkörper (102), der das Folgende umfasst:
eine erste Fluidkammer (103a);
ein erstes Anschlussstück (109), in Fluidverbindung mit der ersten Fluidkammer (103a), wobei das erste Anschlussstück (109) das Folgendes umfasst:
einen ersten Fluidanschluss (125); und
ein Verbindungselement (126), das dazu ausgebildet ist mit einer ersten Kühlmittelleitung (127) verbunden zu werden;
eine zweite Fluidkammer (103b), die eine Wellenleiteröffnung (120) umfasst;
ein zweites Anschlussstück (106) in Fluidverbindung mit der zweiten Fluidkammer (103b), wobei das zweite Anschlussstück (106) das Folgende umfasst:
ein zweiter Fluidanschluss (130); und
ein Verbindungselement (131), das zum Verbinden mit einer zweiten Kühlmittelleitung (132) ausgebildet ist;
ein Wellenleiteranschlussstück (120a), der Zugang zu der zweiten Fluidkammer (103b) des Hauptkörpers bereitstellt, wobei der Wellenleiteranschlussstück (120a) dazu ausgebildet ist, einen Wellenleiter (202) aufzunehmen und diesen durch die zweite Fluidkammer (103b) und die Wellenleiteröffnung (120) der zweiten Fluidkammer (103b) zu führen;
eine Sonde (113), die sich distal vom Hauptkörper (102) erstreckt, wobei die Sonde das Folgende umfasst:
ein längliches Sondenelement (113), das eine flexible äußere Wand umfasst, die innerhalb der länglichen Sonde (113) einen röhrenförmigen Hohlraum definiert, wobei die äußere Wand ein erstes Lumen (116) definiert, das mit der ersten Fluidkammer (103a) in Fluidverbindung steht, und wobei sich die flexible äußere Wand von einem zum Hauptkörper (102) proximalen Abschnitt bis zu einem distalen Abschnitt erstreckt, welcher eine abgedichtete Spitze (115) umfasst; und
ein inneres rohrförmiges Element (117), das sich distal vom Hauptkörper (102) und innerhalb des röhrenförmigen Hohlraums erstreckt, wobei das innere rohrförmige Element (117) ein zweites Lumen (118) definiert und eine Austrittsöffnung umfasst, die innerhalb des distalen Abschnitts des länglichen Sondenelements (113) angeordnet ist, wobei das innere rohrförmige Element (117) dazu ausgebildet ist, den Wellenleiter (202) aufzunehmen, wenn der Wellenleiter durch die Wellenleiteröffnung (120) geführt wird;
wobei:
einen Hülsenverbinder (150) der Laserilluminatorvorrichtung (101) dazu ausgebildet ist, mit einem entsprechenden Verbinder der Katheterhülse (440) in Eingriff zu gelangen, derart, dass - wenn der Hülsenverbinder (150) der Laserilluminatorvorrichtung (101) mit dem Verbinder der Katheterhülse (440) in Eingriff steht - die Katheterhülse (440) dazu ausgebildet ist, die Sonde (113) zumindest teilweise zu bedecken.

2. System nach Anspruch 1, wobei die Sonde (113) aus einem Material hergestellt ist, das für sichtbare Lichtwellenlängen, Infrarotlicht, Ultraviolettlicht oder Kombinationen der vorgenannten durchlässig ist.

3. System nach einem der Ansprüche 1 bis 2, wobei die abgedichtete Spitze (115) gewölbt ist und für sichtbare Lichtwellenlängen, Infrarotlicht, Ultraviolettlicht oder Kombinationen der vorgenannten durchlässig ist.

4. System nach einem der Ansprüche 1 bis 3, wobei der erste Fluidanschluss (125) ein Kühlmittelauslass ist, der dazu ausgebildet ist Kühlmittel in die erste Kühlmittelleitung (127) zu verteilen, und der zweite Fluidanschluss (130) ist ein Kühlmitteleinlass, der dazu ausgebildet ist Kühlmittel aus der zweiten Kühlmittelleitung (132) aufzunehmen, und wobei die Laserilluminatorvorrichtung (101) dazu ausgebildet ist den Durchgang von einem Kühlmittel von dem Kühlmitteleinlass (130), durch das zweite Lumen (118), in das erste Lumen (116) und aus dem Kühlmittelauslass (125) heraus zu ermöglichen.

5. System nach Anspruch 4, wobei der Kühlmitteleinlass (130) und der Kühlmittelauslass (125) ausgebildet sind, um mit verschiedenen Verbindern zusammenzuwirken, um eine unsachgemäße Leitung des Kühlmittels durch die Laserilluminatorvorrichtung (101) zu verhindern.

6. System nach einem der Ansprüche 1 bis 5, wobei der Hülsenverbinder (150) mit einem Gewinde versehen und dazu ausgebildet ist in das entsprechende Gewinde des Verbinders der Katheterhülse (440) einzugreifen.

7. System nach einem der Ansprüche 1 bis 6, wobei der Hülsenverbinder ein Luer-Verbindungselement umfasst, das dazu ausgebildet ist mit einem entsprechenden Luer-Verbindungselement des Verbinders der Katheterhülse in Eingriff zu gelangen.

8. System nach einem der Ansprüche 1 bis 7, wobei die Katheterhülse entlang mindestens eines Abschnittes ihrer Länge mit einer Skala versehen ist und wobei die Skala an einer Stelle proximal zum Verbinder der Katheterhülse (440) auf der Katheterhülse (440) angebracht ist.

9. System nach einem der Ansprüche 1 bis 7, das ferner den Wellenleiter (202) umfasst, wobei der Wellenleiter (202) ein Verbindungselement umfasst, das dazu ausgebildet ist mit einem entsprechenden Verbindungselement des Wellenleiteranschlussstückes (120a) in Eingriff zu gelangen.

10. System nach Anspruch 9, wobei der Wellenleiter (202) eine optische Faser ist.

11. System nach Anspruch 10, wobei die optische Faser (202) einen diffusen Abschnitt (223) umfasst, der dazu ausgebildet ist, Strahlung von der optischen Faser und aus der Laserilluminatorvorrichtung (101) heraus zu verteilen, wobei der diffuse Abschnitt (223) nicht von der Katheterhülse (427) abgedeckt ist, wenn die Katheterhülse über den Hülsenverbinder (150) und den Verbinder der Katheterhülse (440) mit der Laserilluminatorvorrichtung (101) verbunden ist.

12. System nach einem der Ansprüche 8 bis 11, das ferner ein Kühlmittelbehälter (305) umfasst, der dazu ausgebildet ist, mit der zweiten Kühlmittelleitung (132) verbunden zu werden und in Fluidverbindung mit der Laserilluminatorvorrichtung (101) zu stehen, wenn er mit der zweiten Kühlmittelleitung (132) verbunden ist, wobei das Kühlmittelreservoir dazu ausgebildet ist, die Laserilluminatorvorrichtung (101) mit Kühlmittel zu versorgen.

13. System nach einem der Ansprüche 8 bis 12, das ferner eine Pumpe (307) umfasst, die dazu ausgebildet ist Kühlmittel vom Kühlmittelbehälter (305) zur Laserilluminatorvorrichtung (101) über die zweite Leitung (132) zu befördern, um den Wellenleiter (202) während des Betriebs zu kühlen.

14. System nach einem der Ansprüche 8 bis 13, das ferner ein Kühlmittelauffangsbehälter (309) umfasst, der dazu ausgebildet ist, mit der ersten Kühlmittelleitung (127) verbunden zu werden und in Fluidverbindung mit der Laserilluminatorvorrichtung (101) zu stehen, wenn er mit der ersten Kühlmittelleitung (127) verbunden ist, wobei der Kühlmittelauffangsbehälter (309) dazu ausgebildet ist, Kühlmittel von der Laserilluminatorvorrichtung (101) aufzunehmen.

15. System nach einem der Ansprüche 8 bis 14, das ferner eine Laserquelle (203) umfasst, die dazu ausgebildet ist in optischer Kommunikation mit dem Wellenleiter (202) zu stehen.

## Revendications

1. Système comprenant :
une gaine de cathéter; et
un ensemble illuminateur laser (101), l'ensemble illuminateur laser comprenant :
un corps principal (102), comprenant :
une première chambre de fluide (103a) ;
un premier raccord (109), en communication fluidique avec la première chambre de fluide (103a), le premier raccord (109) comprenant :
un premier orifice de fluide (125) ; et
an accessoire (126), configurée pour s'engager avec une première ligne de liquide de refroidissement (127) ;
une deuxième chambre de fluide (103b), comprenant une ouverture de guide d'ondes (120) ;
un deuxième raccord (106), en communication fluidique avec la deuxième chambre de fluide (103b), le deuxième raccord (106) comprenant :
un deuxième orifice de fluide (130) ; et
un accessoire (131), configurée pour s'engager avec une deuxième ligne de liquide de refroidissement (132) ;
un raccord de guide d'ondes (120a), fournissant un accès à la deuxième chambre de fluide (103b) du corps principal, le raccord de guide d'ondes (120a) étant configuré pour recevoir un guide d'ondes (202) et pour le diriger à travers la deuxième chambre de fluide (103b) et l'ouverture de guide d'ondes (120) de la deuxième chambre de fluide (103b) ;
une sonde (113) s'étendant de manière distale depuis le corps principal (102), la sonde comprenant :
un élément de sonde allongé (113) comprenant une paroi externe flexible qui définit une cavité tubulaire à l'intérieur de la sonde allongée (113), la paroi externe définissant une première lumière (116) qui est en communication fluidique avec la première chambre de fluide (103a), la paroi externe flexible s'étendant depuis une partie proximale au corps principal (102) jusqu'à une partie distale comprenant une pointe scellée (115) ; et
un élément tubulaire interne (117) s'étendant de manière distale depuis le corps principal (102) et à l'intérieur de la cavité tubulaire, l'élément tubulaire interne (117) définissant une deuxième lumière (118) et comprenant une ouverture de sortie agencée dans la partie distale de l'élément de sonde allongé (113), dans lequel l'élément tubulaire interne (117) est configuré pour recevoir le guide d'ondes (202) lorsque le guide d'ondes est dirigé à travers l'ouverture de guide d'ondes (120) ;
dans lequel :
un connecteur de gaine (150) de l'ensemble illuminateur laser (101) est configuré pour s'engager avec un connecteur correspondant de la gaine de cathéter (440) de telle sorte que, lorsque le connecteur de gaine (150) de l'ensemble illuminateur laser (101) est engagé avec le connecteur de la gaine de cathéter (440), la gaine de cathéter (440) est configurée pour couvrir au moins partiellement la sonde (113).

2. Système, selon la revendication 1, dans lequel la sonde (113) est constituée d'un matériau qui est transmissif aux longueurs d'onde visibles de la lumière, à la lumière infrarouge, à la lumière ultraviolette, ou à des combinaisons de ce qui précède.

3. Système, selon l'une quelconque des revendications 1 à 2, dans lequel la pointe scellée (115) est bombée et est transmissive aux longueurs d'onde visibles de la lumière, à la lumière infrarouge, à la lumière ultraviolette, ou à des combinaisons de ce qui précède.

4. Système, selon l'une quelconque des revendications 1 à 3, dans lequel le premier orifice de fluide (125) est une sortie de liquide de refroidissement, configurée pour distribuer du liquide de refroidissement dans la première ligne de liquide de refroidissement (127), et le deuxième orifice de fluide (130) est une entrée de liquide de refroidissement configurée pour recevoir du liquide de refroidissement provenant de la deuxième ligne de liquide de refroidissement (132), et dans lequel l'ensemble illuminateur laser (101) est configuré pour permettre le passage d'un liquide de refroidissement depuis l'entrée de liquide de refroidissement (130) à travers la deuxième lumière (118) jusqu'à dans la première lumière (116) et hors de la sortie de liquide de refroidissement (125).

5. Système, selon la revendication 4, dans lequel l'entrée de liquide de refroidissement (130) et la sortie de liquide de refroidissement (125) sont configurées pour interagir avec différents connecteurs afin d'empêcher un acheminement incorrect du liquide de refroidissement à travers l'ensemble illuminateur laser (101).

6. Système, selon l'une quelconque des revendications 1 à 5, dans lequel le connecteur de gaine (150) est fileté et configuré pour s'engager avec des filetages correspondants du connecteur de la gaine de cathéter (440).

7. Système, selon l'une quelconque des revendications 1 à 6, dans lequel le connecteur de gaine comprend un raccord Luer configuré pour se coupler avec un raccord Luer correspondant du connecteur de la gaine de cathéter.

8. Système, selon l'une quelconque des revendications 1 à 7, dans lequel la gaine de cathéter est graduée le long d'au moins une partie de sa longueur et dans lequel les graduations sont prévues sur la gaine de cathéter (440) à une position proximale par rapport au connecteur de la gaine de cathéter (440).

9. Système, selon l'une quelconque des revendications 1 à 7, comprenant, en outre, le guide d'ondes (202), dans lequel le guide d'ondes (202) comprend an accessoire configurée pour s'engager avec un accessoire correspondant du raccord de guide d'ondes (120a).

10. Système, selon la revendication 9, dans lequel le guide d'ondes (202) est une fibre optique.

11. Système, selon la revendication 10, dans lequel la fibre optique (202) comprend une partie diffusive (223), configurée pour distribuer le rayonnement provenant de la fibre optique et hors de l'ensemble illuminateur laser (101), dans lequel la partie diffusive (223) n'est pas couverte par la gaine de cathéter (427) lorsque la gaine de cathéter est connectée à l'ensemble illuminateur laser (101) via le connecteur de gaine (150) et le connecteur de la gaine de cathéter (440).

12. Système, selon l'une quelconque des revendications 8 à 11, comprenant, en outre, un réservoir de liquide de refroidissement (305) configuré pour s'engager avec la deuxième ligne de liquide de refroidissement (132), et pour être en communication fluidique avec l'ensemble illuminateur laser (101) lorsqu'il est engagé avec la deuxième ligne de liquide de refroidissement (132), dans lequel le réservoir de liquide de refroidissement est configuré pour fournir du liquide de refroidissement à l'ensemble illuminateur laser (101).

13. Système, selon l'une quelconque des revendications 8 à 12, comprenant en outre une pompe (307), configurée pour transporter le liquide de refroidissement depuis le réservoir de liquide de refroidissement (305) vers l'ensemble illuminateur laser (101) via la deuxième ligne (132) pour refroidir le guide d'ondes (202) lorsqu'il est en fonctionnement.

14. Système, selon l'une quelconque des revendications 8 à 13, comprenant en outre un sac de récupération de liquide de refroidissement (309) configuré pour s'engager avec la première ligne de liquide de refroidissement (127) et pour être en communication fluidique avec l'ensemble illuminateur laser (101) lorsqu'il est engagé avec la première ligne de liquide de refroidissement (127), dans lequel le sac de récupération de liquide de refroidissement (309) est configuré pour recevoir le liquide de refroidissement provenant de l'ensemble illuminateur laser (101).

15. Système, selon l'une quelconque des revendications 8 à 14, comprenant, en outre, une source laser (203), configurée pour être en communication optique avec le guide d'ondes (202).
